(19) Europäisches Patentamt
European Patent Office
Office européen des brevets

(11) Publication number: **0 294 228 B1**

# (12) EUROPEAN PATENT SPECIFICATION

(45) Date of publication of patent specification: **26.08.92**

(51) Int. Cl.[5]: **C07D 339/08**, C07D 409/04, A01N 43/32, C07F 7/08

(21) Application number: **88305107.0**

(22) Date of filing: **03.06.88**

The file contains technical information submitted after the application was filed and not included in this specification

## (54) Novel heterocyclic pesticidal compounds.

(30) Priority: **05.06.87 GB 8713222**
**05.09.87 GB 8720928**

(43) Date of publication of application:
**07.12.88 Bulletin 88/49**

(45) Publication of the grant of the patent:
**26.08.92 Bulletin 92/35**

(84) Designated Contracting States:
**AT BE CH DE ES FR GB GR IT LI LU NL SE**

(56) References cited:
**EP-A- 0 073 378**

(73) Proprietor: **THE WELLCOME FOUNDATION LIMITED**
**Unicorn House 160 Euston Road**
**London NW1 2BP(GB)**

Proprietor: **THE REGENTS OF THE UNIVERSITY OF CALIFORNIA**
**300 Lakeside Drive, 22nd Floor, Office of the President**
**Oakland, California 94612-3550(US)**

(72) Inventor: **Casida, John Edward**
**1570 La Vereda Road**
**Berkeley California 94708(US)**
Inventor: **Elliott, Michael**
**9 Long Ridge Aston**
**Stevenage Hertfordshire(GB)**
Inventor: **Pulman, David Allen**
**The Wellcome Foundation Limited**
**Berkhamsted Hertfordshire(GB)**

(74) Representative: **Rollins, Anthony John et al**
**Group Patents & Agreements The Wellcome Foundation Ltd Langley Court**
**Beckenham Kent BR3 3BS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 294 228 B1

## Description

The present invention is concerned with a method of controlling pests such as arthropods, e.g. insects and acarine pests, and helminths, e.g. nematodes, by contacting the pests with novel pesticides. The invention is also concerned with the novel pesticides used for controlling the pests and processes for making such pesticides.

Certain 2,5-dialkylsubstituted dithianes have been investigated as liquid crystal materials (see for example Mol. Cryst. Liq. Cryst., 131. 101) but no pesticidal activity has been reported for such compounds.

It has been discovered that a class of novel 2,5-disubstituted dithianes has pesticidal activity.

Accordingly, the present invention provides a method for controlling pests which comprises contacting the pest with a pesticidally effective amount of a compound of the formula (I):

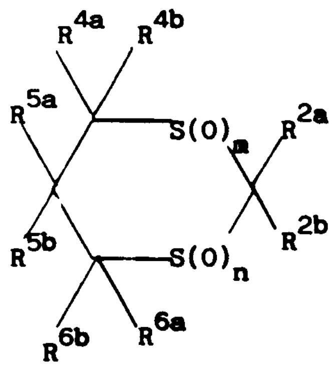

(I)

which contains between 9 and 30 carbon atoms, and wherein m and n are independently selected from 0, 1 and 2; $R^{2a}$ is hydrogen, cyano, methoxy, ethoxy, a group $S(O)_qR^7$ where q is 0, 1 or 2 and $R^7$ is methyl or ethyl, or $R^{2a}$ is a group $C{\equiv}CR^8$ where $R^8$ is hydrogen, $C_{1-4}$ alkyl or a silyl group containing three $C_{1-4}$ alkyl groups or two $C_{1-4}$ alkyl groups and a phenyl group or $R^{2a}$ is vinyl, halo, $C_{1-3}$ alkyl optionally substituted by halo, methoxy, ethoxy or cyano, or $R^{2a}$ is a group $COR^9$ where $R^9$ is hydrogen, $C_{1-4}$ alkoxy, $C_{1-4}$ alkyl or a group $NR^{10}R^{11}$ wherein $R^{10}$ and $R^{11}$ are independently selected from hydrogen, methyl or ethyl; $R^{2b}$ which contains between 2 and 18 carbon atoms is a 5 or 6-membered ring containing an oxygen, sulphur and/or nitrogen atom or a group $R^{12}$ wherein $R^{12}$ is a $C_{1-16}$ hydrocarbyl group, each optionally substituted by an azido, cyano, nitro ot two hydroxy groups or by one to five halo atoms which are the same or different or by one to four groups $R^{13}$ which are the same or different and each contains one to four hetero atoms, which are the same or different and are chosen from oxygen, sulphur, nitrogen and silicon, 1 to 16 carbon atoms and optionally 1 to 9 fluoro or chloro atoms or in the case of the 5 or 6 membered ring optionally substituted by a group $R^{12}$; $R^{4a}$ and $R^{4b}$, $R^{6a}$ and $R^{6b}$ are independently selected from hydrogen, $C_{1-3}$ alkyl, $C_{2-3}$ alkenyl or alkynyl each being optionally substituted by halo, cyano or $C_{1-4}$ alkoxy; cyano, halo or a group $COR^{9'}$ wherein $R^{9'}$ is hydrogen, $C_{1-4}$ alkoxy, $C_{1-4}$ alkyl or a group $NR^{10'}R^{11'}$ wherein $R^{10'}$ and $R^{11'}$ are independently selected from hydrogen, methyl or ethyl; $R^{5a}$ is a non-aromatic hydrocarbyl group containing up to seven carbon atoms, or phenyl each optionally substituted by cyano, halo, $C_{1-4}$ alkyl, $C_{1-4}$ haloalkyl, $C_{3-4}$ cycloalkyl, $C_{1-4}$ alkoxy or a group $S(O)_q$, $R^{7'}$ wherein $q'$ is 0,1 or 2 or $R^{7'}$ is methyl or ethyl and $R^{5b}$ is hydrogen or $C_{1-4}$ alkyl optionally substituted by alkoxy provided that when $R^{2a}$ is hydrogen, methyl, or ethyl; $R^{2b}$ is acetylene or contains between 3 and 18 carbon atoms and is a group $R^{14}$, wherein $R^{14}$ is a $C_{1-13}$ non-aromatic hydrocarbyl group, optionally substituted by a $C_{1-4}$ carbalkoxy or cyano group and/or by one or two hydroxy groups and/or by one to five halo atoms which are the same or different and/or by one to three groups $R^{15}$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{15}$ and/or by a group $-C{\equiv}CH$, $-C{\equiv}C-R^{14}$ or $C{\equiv}C$-halo and/or by one to five halo atoms and/or by one to three $C_{1-4}$ haloalkyl groups wherein $R^{14}$ and $R^{15}$ are as hereinbefore defined; $R^{4a}$ and $R^{6a}$ are the same or different and are chosen from hydrogen, methyl, trifluoromethyl or cyano; $R^{4b}$ and $R^{6b}$ are hydrogen, and $R^{5b}$ is hydrogen or methyl then $R^{5a}$ is not tertiary butyl.

By the term "halo" is meant fluoro, chloro, bromo or iodo.

By the term "hydrocarbyl" group is meant an alkyl, cyclic alkyl, alkenyl cyclic alkenyl, alkynyl phenyl or naphthyl group or combinations of such groups, for example a cyclic alkyl or phenyl group substituted by

an alkynyl group.

By the term "non-aromatic hydrocarbyl group" is meant an alkyl, alkenyl or alkynyl group (including a cyclic alkyl or alkenyl group optionally substituted by alkyl, alkenyl or alkynyl; and alkyl or alkenyl substituted by cyclic alkyl and alkenyl).

$R^{2b}$ suitably contains between 5 and 12 carbon atoms. $R^{2b}$ is suitably a $C_{5-9}$ alkyl, alkenyl or alkynyl group or a substituted phenyl or cyclohexyl group.

Suitable substituents for the group $R^{2b}$ when this is phenyl, $C_{4-8}$ alkyl or cycloalkyl include halo, $C_{1-4}$ haloalkyl, cyano, azido or a group $(C{\equiv}C)_p$ $R^{16}$ wherein p is 1 or 2 and $R^{16}$ is hydrogen, bromine, chlorine, iodine or a group $S(O)_wR^{17}$ wherein $R^{17}$ is trifluoromethyl, methyl or ethyl and w is 0, 1 or 2; or $R^{16}$ is an aliphatic group containing up to 5 carbon atoms optionally substituted by $C_{1-4}$ alkoxy, $C_{1-6}$ alkoxyalkoxy, $C_{1-8}$ acyloxy, halo or hydroxy, a group - $COR^9$ as hereinbefore defined, or $R^{16}$ is Si $R^{18}$ $R^{19}$ $R^{20}$ wherein $R^{18}$ to $R^{20}$ are the same or different and each is a $C_{1-4}$ aliphatic group or $R^{18}$ and $R^{19}$ are $C_{1-4}$ aliphatic groups and $R^{20}$ is a phenyl group.

The group $R^{13}$ is linked to the hydrocarbyl group via a hetero atom of $R^{13}$. Suitable substituents $R^{13}$ for the group $R^{12}$ include alkoxy, alkenyloxy, alkynyloxy, alkoxyalkoxy, acyloxy, alkylthio, trialkylsilyl, haloalkoxy, haloalkenyloxy, haloalkynyloxy, carbalkoxy and mono or di-substituted alkylamino groups. When a silyl group is present this is normally adjacent to an ethynyl group. Preferred substituents $R^{13}$ include alkoxy, alkoxyalkoxy, alkenyloxy, alkynyloxy, haloalkoxy, haloalkenyloxy and haloalkynyloxy.

Preferably there is only one silyl group present. Preferably there is a maximum of two sulphur atoms present in $R^{2b}$. Suitably there is a maximum of four and preferably a maximum of three oxygen atoms in $R^{2b}$. Preferably there is only one nitrogen atom present in $R^{2b}$.

In one suitable embodiment, $R^{2b}$ is a phenyl group substituted at the 3-,4-or 5-positions by one to three substituents each selected from halo, $C_{1-4}$ haloalkyl, cyano, azido, a group $(C{\equiv}C)_pR^{16}$ or a group $S(O)_wR^{17}$ wherein p w, $R^{16}$ and $R^{17}$ are as hereinbefore defined. $R^{2b}$ is additionally optionally substituted at the 2- and/or 6-positions by fluoro or chloro. Suitably when the substituent is a group $(C{\equiv}C)_pR^{16}$, there is only one such substituent on the phenyl ring.

In one preferred embodiment $R^{2b}$ is phenyl substituted at the 3-, 4- or 5-positions by one to three substituents each selected from halo, cyano, $C_{1-4}$ haloalkyl or a group $C{\equiv}C\text{-}R^{21}$ where $R^{21}$ is hydrogen, methyl, or ethyl each optionally substituted by hydroxy, methoxy, ethoxy, acetoxy; or $R^{21}$ is $C_{1-4}$ carbalkoxy, or a silyl group substituted by three $C_{1-4}$ alkyl groups. $R^{2b}$ is additionally optionally substituted at the 2- and/or 6- positions by fluoro or chloro.

In a second preferred embodiment $R^{2b}$ is a group $-A(C{\equiv}C)Z$, wherein A is a polymethylene chain optionally containing a double bond and/or an oxygen atom and/or $S(O)_wR^{17}$ wherein w and $R^{17}$ are as hereinbefore defined, and Z is hydrogen, $C_{1-5}$ alkyl, $C_{1-3}$ alkoxymethyl or a group $SiR^{18}$ $R^{19}$ $R^{20}$ wherein $R^{18}$ to $^{20}$ are as hereinbefore defined.

In a third preferred embodiment $R^{2b}$ is a group $-BZ^1$, wherein B is a group $-CH_2O-$ or $CH_2S(O)_w$ wherein w is 0,1 or 2 or a $C_{2-6}$ aliphatic group optionally substituted by one to three halo atoms and $Z^1$ is silyl substituted by three $C_{1-4}$ alkyl groups or a group

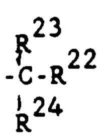

wherein $R^{22}$, $R^{23}$ and $R^{24}$ are the same or different and are each independently selected from halo, $C_{1-4}$ alkyl optionally substituted by halo, $C_{1-4}$ alkoxy or a group $S(O)_q$ $R^{25}$ wherein q is 0, 1 or 2 and $R^{25}$ is $C_{1-4}$ alkyl, or $R^{22}$, $R^{23}$ and $R^{24}$ are selected from $C_{1-4}$ alkoxy or a group $S(O)_wR^{26}$ wherein w is 0, 1 or 2 and $R^{26}$ is $C_{1-4}$ alkyl optionally substituted by fluoro or $R^{22}$ and $R^{23}$ are linked to form a $C_{3-6}$ cycloalkyl ring, or one or $R^{22}$, $R^{23}$ and $R^{24}$ may be hydrogen.

By the term "aliphatic group" is meant an alkyl, alkenyl or alkynyl group.

Most suitably B is a group $-C{\equiv}C-$, $-CH{=}CH-$ or $-CH_2CH_2-$ and preferably B is $-C{\equiv}C-$.

Preferably $Z^1$ is tertiary butyl, trichloromethyl or 2-methoxyprop-2-yl.

In a fourth preferred embodiment $R^{2b}$ is a group

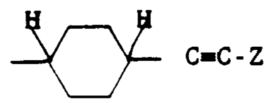

wherein Z is as hereinbefore defined

Suitably $R^{2a}$ is hydrogen, methyl or ethyl.

Suitably $R^{4a}$, $R^{4b}$, $R^{6a}$ and $R^{6b}$ are each selected from hydrogen, methyl, cyano or trifluoromethyl and preferably they are all hydrogen.

Suitably $R^{5a}$ is a primary, secondary or tertiary $C_{2-5}$ alkyl group.

Suitably $R^{5b}$ is hydrogen, methyl or ethyl, preferably $R^{5b}$ is hydrogen.

In accordance with another embodiment of the present invention there is provided a compound of the formula (I) wherein $R^{2a}$, $R^{4a}$, $R^{4b}$, $R^{5b}$, $R^{6a}$, $R^{6b}$, m and n are as hereinbefore defined and $R^{2b}$ is phenyl, substituted phenyl, a heterocycle containing 4 to 6 carbon atoms except that (i) when $R^{5a}$ is straight chain alkyl or tolyl and $R^{2a}$, $R^{4a}$, $R^{4b}$, $R^{6a}$, $R^{6b}$ and $R^{5b}$ are all hydrogen, $R^{2b}$ is not para-cyanophenyl; (ii) when $R^{5a}$ and $R^{5b}$ are both ethyl and $R^{4a}$, $R^{4b}$, $R^{6a}$, $R^{6b}$ and $R^{2a}$ are all hydrogen, $R^{2b}$ is not para-tolyl; (iii) when $R^{5a}$ is n-pentyl and $R^{2a}$, $R^{4a}$, $R^{4b}$, $R^{6a}$, $R^{6b}$ and $R^{5b}$ are all hydrogen, $R^{2b}$ is not para-bromophenyl, (iv) when $R^{5b}$ and $R^{5a}$ are both methyl and $R^{4a}$, $R^{4b}$, $R^{6b}$ and $R^{2a}$ are all hydrogen, $R^{2b}$ is not unsubstituted phenyl and (v) when $R^{2b}$ is n-propyl, $R^{2a}$ is methyl and $R^{4a}$, $R^{4b}$, $R^{6a}$, $R^{6b}$ and $R^{5b}$ are all hydrogen $R^{5a}$ is not ethyl or i-propyl.

In accordance with yet another embodiment of the present invention there is provided a pesticidal composition containing a compound of the formula (I) in admixture with a diluent or carrier thereof.

In accordance with another embodiment of the present invention, there is provided preferred compounds of formula (I) including those wherein $R^{2a}$, $R^{4a}$, $R^{4b}$, $R^{5b}$, $R^{6a}$, $R^{6b}$, m and n are as hereinbefore defined and $R^{2b}$ is substituted phenyl or optionally substituted $C_{4-8}$ alkyl or cycloalkyl or a six membered N-containing heterocycle, the substituents being selected from halo, $C_{1-4}$ haloalkyl , cyano, azido or a group $(C{\equiv}C)_pR^{16a}$ wherein p is 1 or 2 and $R^{16a}$ is hydrogen, bromo, chloro, iodo or a group $S(O)_qR^7$ as hereinbefore defined, or $R^{16a}$ is an aliphatic group containing up to five carbon atoms optionally substituted by $C_{1-4}$ alkoxy, halo or hydroxy or $R^{16a}$ is a group $COR^9$ or $SiR^{18}$, $R^{19}$, $R^{20}$ wherein $R^9$, $R^{18}$, $R^{19}$, $R^{20}$ are as hereinbefore defined.

In accordance with a further embodiment of the present invention, there is provided a compound of formula (Ia):

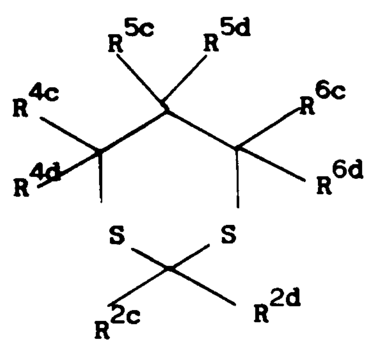

(Ia)

wherein $R^{2c}$ is selected from hydrogen, cyano, methoxy, a group $S(O)_qR^7$ wherein q is 0, 1 or 2 and $R^7$ is methyl or ethyl or $R^{2c}$ is vinyl, halo, $C_{1-3}$ alkyl optionally substituted by halo, methoxy, ethoxy or cyano; $R^{2d}$ is a group $(C{\equiv}C)_r$ $Y(C{\equiv}C)_t$ $Z^2$ wherein r is 0 or 1 and t is 1 or 2 and the sum of r and t is not greater than 2, Y is a single bond, a group

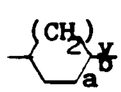

wherein v is 1, 2 or 3 and the $(C{\equiv}C)_tZ^2$ fragment is attached to the a or b position of the ring, or Y is a polymethylene chain containing between 1 and 8 carbon atoms in which one or two heteroatoms and/or

double or triple bonds may be interspersed, the chain being optionally substituted by one to four substituents which may be the same or different and are each independently selected from hydroxy, oxo, halo, $C_{1-4}$ alkyl, $C_{1-4}$ alkoxy, $C_{1-4}$ acyloxy, epoxy, a $C_{1-4}$ alkylidene group, a $C_{1-6}$ carbalkoxy group, $C_{1-4}$ haloalkyl or cyano, $Z^2$ is selected from hydrogen, $C_{1-10}$ hydrocarbyl optionally substituted by halo, $C_{1-4}$ alkoxy, hydroxy, oxo, a group $S(O)qR^7$ as hereinbefore defined, cyano, $C_{1-4}$ acyloxy or carbalkoxy, halo or a group $SiR^{27}$ $R^{28}$ $R^{29}$ wherein $R^{27}$, $R^{28}$ and $R^{29}$ are the same or different and are each a hydrocarbyl group containing up to 4 carbon atoms or $Z^2$ is a group $R^{30}OCO$ wherein $R^{30}$ is $C_{1-4}$ alkyl; $R^{4c}$, $R^{4d}$, $R^{6c}$ and $R^{6d}$ are each selected from hydrogen, methyl, cyano and trifluoromethyl; $R^{5c}$ is alkyl, cycloalkyl, alkenyl, cycloalkenyl or alkynyl each containing up to seven carbon atoms or phenyl each optionally substituted by halo, cyano, $C_{1-4}$ alkyl, $C_{1-4}$ haloalkyl, $C_{3-4}$ cycloalkyl, $C_{1-4}$ alkoxy or a group $S(O)_q$ $R^7$ as hereinbefore defined; and $R^{5d}$ is hydrogen or $C_{1-4}$ alkyl provided that $R^{5c}$ is not tertiary butyl.

Suitably r is 0, t is 1 and Y is a single bond or a $C_{3-5}$ polymethylenechain optionally containing a double bond and $Z^2$ is hydrogen, $C_{1-5}$ alkyl, $C_{1-3}$ alkoxymethyl or a group $SiR^{31}$ $R^{32}$ $R^{33}$ wherein $R^{31}$, $R^{32}$ and $R^{33}$ are the same or different and each is selected from $C_{1-4}$ alkyl.

Suitably $R^{2c}$ is hydrogen, methyl or ethyl.

Suitably $R^{2d}$ is a group $(C{\equiv}C)_r$ $Y^1$ $(C{\equiv}C)_t$ $Z^1$ as hereinbefore defined. Preferably r is 0, t is 1 and $Y^1$ is a single bond and $Z^1$ is $C_{1-4}$ alkyl and preferably $R^{2d}$ is a group $-C{\equiv}C\text{-}Bu^t$.

Preferably $R^{4c}$, $R^{4d}$, $R^{6c}$ and $R^{6d}$ are all hydrogen.

Preferably $R^{5c}$ is a secondary or tertiary $C_{3-5}$ alkyl group.

Preferably $R^{5d}$ is hydrogen.

The compounds of the formula (I) may exist in a number of stereoisomeric forms. The present invention encompasses both individual conformational and stereoisomers and mixtures thereof. The present invention also encompasses compounds of the formula (I), containing radiosotopes particularly those in which or one to three hydrogen atoms are replaced by tritium or one or more carbon atoms are replaced by $^{14}C$.

Preferred compounds of the invention include:

cis-2(a)-(4-Bromophenyl)-5(e)-butyl-1,3-dithiane
5(e)-t-Butyl-2(e)-(4-methylphenyl)-1,3-dithiane
5(e)-t-Butyl-2(e)-(4-nitrophenyl)-1,3-dithiane
2(e)-(4-Bromophenyl)-5(e)-neopentyl-1,3-dithiane
cis-2(e)-(4-Bromophenyl)-5(e)-t-butyl-2(a)-methylthio-1,3-dithiane
2(e)-(4-Bromophenyl)-5(e)-t-butyl-2(a)-methoxy-1,3-dithiane
trans-2(e)-(4-Bromophenyl)-5(e)-ethyl-1,3-dithiane
2(e)-(4-Bromophenyl)-2(a)-methyl-5(e)-(1,1-dimethylpropyl)-1,3-dithiane
2(e)-(4-Bromophenyl)-5(e)-t-butyl-1,3-dithiane-2(a)-carbonitrile
2(a)-(4-Bromophenyl)-5(e)-ethyl-2(e)-methyl-1,3-dithiane
trans-5(e)-t-Butyl-2(e)-(4-t-butylphenyl)-1,3-dithiane
2-(5(e)-t-Butyl-1,3-dithian-2(e)-yl)propan-2-one
trans-5(e)-t-Butyl-(2e)-(2,2-dibromoethenyl)-1,3-dithiane
trans-2(e)-Bromoethynyl-5(e)-t-butyl-1,3-dithiane
4-(trans-5(e)-t-Butyl-1,3-dithian-2(e)-yl)-2,2-dimethylbutan-3-one
2(e)-(3,3-Dimethylbutyl)-5(e)-ethyl-1,3-dithiane
2(a)-(4-Bromophenyl)-2(e)-methyl-5(e)-(1,1-dimethylpropyl)-1,3-dithiane

The present invention also provides for the preparation of the compounds of the formula (I) by methods derived from those known in the art for the preparation of analogous compounds. Thus, the compounds may be prepared by the reaction of a compound of the formula (II):

(i)

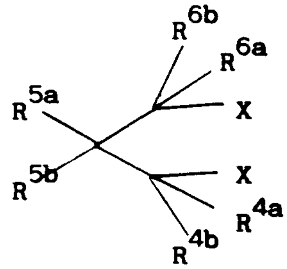

(II)

wherein X is SH with a suitable aldehyde or ketone of the formula

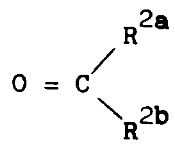

or a reactive derivative thereof, wherein $R^{2a}$, $R^{2b}$, $R^4$, $R^5$ and $R^6$ are as hereinbefore defined and, if required, thereafter oxidizing one or both of the ring sulphur atoms.

The reaction is suitably carried out in the presence of a catalyst or of a dehydrating agent in a nonpolar solvent at a non-extreme temperature. Suitable catalysts include a dimethyl formamide/dimethyl sulphate catalyst and catalysts such as sulphonic acids or perfluorinated resins thereof or Lewis acids such as boron trifluoride etherate, or stannic chloride or concentrated formic acid which also serves as the reaction medium. Suitable solvents include hydrocarbons such as benzene, toluene or xylene or chlorinated hydrocarbons such as dichloromethane. The reaction is normally performed between 0° and 200° and conveniently between 20° and 120°.

Suitable reactive derivatives of aldehydes and ketones include acetals and ketals.

The compounds of the formula (II) may be prepared from the corresponding diols wherein X is hydroxy via the sulphonate derivatives (i.e., compounds of the formula (II) wherein X is a group $OSO_2R^{34}$ wherein $R^{34}$ is $C_{1-4}$ alkyl or para-tolyl) as outlined in Appendix 1. The preparation of the diols and their conversion to the corresponding dithiols can be carried out by methods known in the art for example as outlined in Appendices 1 and 2.

The aldehydes and ketones reacted with the dithiols of the formula (II) are either known in the literature or are prepared by literature methods, for example, ethynylcyclohexylcarboxaldehydes may be prepared as outlined in Appendix 3.

(ii) When $R^{2a}$ is hydrogen, the reaction of a dithiaborinane-dimethylsulphide complex of a compound of the formula (II) with a carboxylic acid

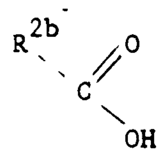

This reaction is carried out in the presence of a reducing agent such as stannous chloride in an inert solvent such as an ether, conveniently tetrahydrofuran, at a non-extreme temperature, for example between -20° and 100° and conveniently between 10° and 30°.

The dithiaborinane-dimethylsulphide complex is prepared from the corresponding dithiol by methods well known to those skilled in the art.

(iii) when it is required to prepare a compound of the formula (I) and $R^{2a}$ is other than hydrogen,

a) when it is required that $R^{2a}$ is alkoxy, alkenyl or alkynyl, the reaction of the corresponding compound of the formula (III):

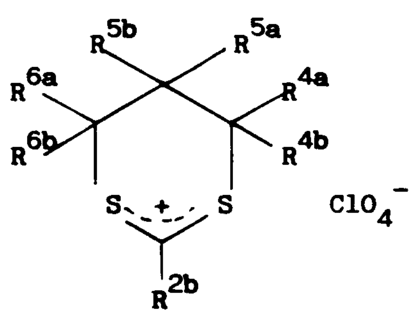

(III)

with the appropriate alcohol $R^{2a}OH$ or Grignard reagent, for example vinyl magnesium bromide or ethynyl magnesium bromide. This reaction is conveniently carried out at a non-extreme temperature,

such as between -50° and 50°C for example 0 to 20°C, and, in the case of reaction with a Grignard reagent, in the presence of an inert solvent such as an ether, for example diethyl ether. The carbonium ion of the dithiane is conveniently formed by reaction of the corresponding dithiol of the formula (II) with a compound hal.OCR$^{2b}$ wherein hal is haloger and $R^{2b}$ is as hereinbefore defined in the presence of perchloric acid. The reaction is carried out at a non-extreme temperature, for example between -50° and 50°C and no solvent is required.

b) when it is required that $R^{2a}$ is an alkylthio group by the reaction of the corresponding compound wherein $R^{2a}$ is hydrogen with a dialkyldisulphide in the presence of an alkyllithium compound, for example butyllithium. The alkyllithium is added to the compound of the formula (I) before the addition of dialkyldisulphide. The reaction is carried out in the presence of an ether, such as tetrahydrofuran, at a low temperature, for example between -50°C and 20°C, such as -20°C.

c) when it is required that $R^{2a}$ is cyano by the reaction of the corresponding compound wherein $R^{2a}$ is methylthio with a compound $(Alk)_3SiCN$ wherein Alk is a $C_{1-4}$ alkyl group. This reaction is conveniently carried out in the presence of a suitable Lewis acid catalyst such as boron trifluoride etherate at a non-extreme temperature for example between 0° and 70°C and conveniently at room temperature, and in a suitable solvent such as a halogenated hydrocarbon such as chloroform. The reaction is conveniently carried out in an inert atmosphere for example in a nitrogen atmosphere.

(iv) when it is required to prepare a compound of the formula (I) wherein $R^{2b}$ is pyridyl, the reaction of the corresponding compound wherein $R^{2b}$ is hydrogen with pyridine. This reaction is conveniently carried out in the presence of a strong base, such as butyllithium, at a non-extreme temperature, for example between -70° and 50°C and conveniently between -30° and 30°C, in an inert solvent, for example, an ether such as tetrahydrofuran. The compound wherein $R^{2b}$ is hydrogen may be prepared by the reaction of a compound of the formula (II) with formaldehyde under the conditions described for the preparation of compounds of the formula (I) from a compound of the formula (II) and an aldehyde or ketone.

It is often convenient to prepare compounds of the formula (I) by interconversion from other compounds of the formula (I), for example:

(a) when it is required to prepare a compound of the formula (I) which contains an optionally substituted ethynyl aryl group.

(i) by the reaction of the corresponding compound in which $R^{2b}$ is a 6-membered aromatic ring which contains iodo in place of $-C{\equiv}C-R^{35}$ with a compound $HC{\equiv}CR^{35}$ wherein $R^{35}$ is a group $R^{14}$ or $R^{16}$ as hereinbefore defined. This reaction is carried out in the presence of a suitable palladium catalyst well know to those skilled in the art for this type of reaction, for example bistriphenylphosphine palladium dichloride, and a catalytic amount of a cuprous halide, such as cuprous iodide. The reaction will normally be carried out in the presence of basic solvent such as diethylamine or triethylamine at a non-extreme temperature, for example between -50° and 100° and conveniently at room temperature. The starting material, i.e. the iodophenyl dithiane may be prepared as described above.

By the conversion of a group, for example a group $CH=C(hal)_2$ or $(hal)CH=CH_2$ wherein hal is chloro or bromo, into an ethynyl group.

The reaction is conveniently carried out by methods well known to those skilled in the art, for example when the group $-CH=C(hal)_2$ at about or below room temperature, for example between -70°C and 25°C, in an inert solvent, conveniently an ether such as tetrahydrofuran, in the presence of a strong base, such as an alkllithium conveniently butyllithium.

(b) when it is desired to prepare a compound of the formula (I) which contains a substituted ethynyl group from a compound of formula (I) which contains a group $-C{\equiv}C-H$, by reaction of the anion from such a compound with an alkylating or acylating agent hal $R^{14}$, hal $R^{16}$, or halZ respectively, wherein hal is halogen and $R^{14}$, $R^{16}$ and Z are as hereinbefore defined except that $R^{16}$ or Z is other than hydrogen. This reaction is particularly suitable for the preparation of those compounds wherein $R^{14}$, $R^{16}$ or Z is a $C_{1-4}$ alkyl group or a group $COR^9$ wherein $R^9$ is a $C_{1-4}$ alkoxy group. The reaction is normally carried out in the presence of a strong base, such as an alkyllithium conveniently butyllithium in an inert solvent, such as an ether, for example tetrahydrofuran, at a non-extreme temperature, for example between -50° and 50°C and conveniently between -10° and 30°. The starting material, i.e. the unsubstituted alkynylphenyl dithiane may be prepared as described above.

(c) when it is desired to prepare a compound of the formula (I) wherein $R^8$, $R^{16}$ or Z is hydrogen by the desilylation of a compound of the formula (I) wherein $R^8$, $R^{16}$ or Z is a tri-$C_{1-4}$ alkylsilyl group. This reaction may be carried out by methods well known to those skilled in the art, for example by reaction with tetrabutylammonium fluoride in an ether, such as tetrahydrofuran, at a non-extreme temperature, for example between 0° and 70°C and conveniently at room temperature.

(d) when it is requred to prepare a compound of the formula (I) wherein $R^{2b}$ is an alkylthiophenyl group

by the reaction of the corresponding compound wherein $R^{2b}$ is a halophenyl group with a dialkyldisulphide in the presence of an alkyllithium compound, for example butyllithium. The alkyllithium is added to the compound of the formula (I) before the addition of the dialkyldisulphide. The reaction is carried out in the presence of an ether, such as tetrahydrofuran, at a low temperature, for example between -50° and 20°C, such as -20°C.

(e) when it is required to convert a compound of the formula (I) wherein $R^{2a}$ is an axial hydrogen atom to the corresponding compounds wherein $R^{2a}$ is an equatorial atom by the addition of a strong base to the compound of the formula (I). The reaction is conveniently carried out in an inert solvent, conveniently an ether such as tetrahydrofuran, at a non-extreme temperature, conveniently -50° to 50°C and conveniently at 0°C followed by quenching with water. If the reaction is carried out in the presence of an alkylating agent, such as methyl iodide, the corresponding equatorial alkylated compound is formed.

(f) when it is required to prepare a compound of the formula (I) wherein $R^{2b}$ contains a hydroxyalkyl group by the reduction of the corresponding compound containing an ester group. This reduction is conveniently carried out by a complex metal hydride such as lithium alumminium hydride in an inert solvent such as an ether, for example diethyl ether, at a non-extreme temperature, for example between 0°C and 70°C and conveniently at room temperature.

(g) The compounds of the formula (I) may contain two or more sulphur atoms which may be oxidised if required. Oxidations can be carried out by methods well known to those skilled in the art, for example using peracids such as peracetic acid from hydrogen peroxide and acetic acid, or 3-chloroperbenzoic acid in chloroform or dichloromethane, or using periodate such as tetrabutylammonium periodate in a halogenated hydrocarbon, for example chloroform at a non-extreme temperature, for example between 0° and 100°C and conveniently between 10° and 30°C.

These processes may also be used to prepare compounds of the formula (I) wherein $R^{5a}$ is tertiary butyl and $R^{2a}$ is other than hydrogen, methyl or ethyl.

These processes may also be used to prepare compounds of formula (I) from the corresponding compound wherein $R^{5a}$ is tertiary butyl and $R^{2a}$ is hydrogen, methyl or ethyl.

The corresponding compound wherein $R^{5a}$ is tertiary butyl and $R^{2a}$ is hydrogen may be prepared by the methods described for the preparation of structurally related compounds, i.e. by methods described herein for the preparation of compounds of the formula (I).

The compounds of formula (I) may be used to control pests such as arthropods, e.g. insect and acarine pests, and helminths, e.g. nematodes. Thus, the present invention provides a method for the control of arthropods and/or helminths which comprises administering to the arthropod and/or helminth or to their environment an effective amount of a compound of the formula (I). The present invention also provides a method for the control of arthropod and/or helminth infestations of animals (including humans) and/or of plants (including trees) and/or stored products which comprises administering an effective amount of a compound of the formula (I). The present invention further provides for the compounds of the formula (I) for use in human and veterinary medicine, in public health control and in agriculture for the control of arthropod and/or helminth pests.

By the term "control" is meant the amelioration of present or future deleterious effects of pests and includes killing adults, larvae and eggs, the inhibition of reproduction, the repellency and/or knockdown of pests, and any other influence on behaviour.

Compounds of formula (I) are of particular value in the protection of field, forage, plantation, glasshouse, orchard and vineyard crops, of ornamentals and of plantation and forest trees, for example, cereals (such as maize, wheat, rice, millet, oats, barley, sorghum), cotton, tobacco, vegetables and salads (such as beans, cole crops, cucurbits, lettuce, onions, tomatoes and peppers), field crops (such as potato, sugar beet, ground nuts, soyabean, oil seed rape), sugar cane, grassland and forage crops (such as lucerne), plantations (such as of tea, coffee, cocoa, banana, oil palm, coconut, rubber, spices), orchards and groves (such as of stone and pip fruit, citrus fruits, kiwifruit, avocado, mango, olives and walnuts), vineyards, ornamental plants, flowers and shrubs under glass and in gardens and parks, forest trees (both deciduous and evergreen) in forests, plantations and nurseries and plants grown for industrial or pharmaceutical purposes (such as the evening primrose).

They are also valuable in the protection of timber (standing, felled, converted, stored or structural) from attack by sawflies (e.g. Urocerus) or beetles (e.g. scolytids, platypodids, lyctids, bostrychids, cerambycids, anobiids).

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly

attack.

Compounds of formula (I) are of value in the control of public health pests, for example cockroaches and ants.

Compounds of formula I are also of value in the control of arthropods or helminths which are injurious to, or spread or act as vectors of diseases in man and domestic animals, for example those hereinbefore mentioned, and more especially in the control of ticks, mites, lice, fleas, midges biting, nuisance and myiasis flies, mosquitos and hemiptrean bugs.

The compounds of Formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspension, oil solution, pressure-pack, impregnated article, microcapsule, pour on formulation or other standard formulations well known to those skilled in the art. Sprays may be applied by hand or by means of a spray race or arch or by vehicle or aircraft mounted apparatus. The animal, soil, plant or other surface being treated may be saturated with the spray by means of high volume application or superficially coated with the spray by means of light or ultra low volume application. Dip concentrates are not applied per se, but diluted with water and the animals immersed in a dipping bath containing the dip wash. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

Compounds of Formula (I) may be prepared either as formulations ready for use on the animals, plants or surface or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powders and granules and other solid formulations comprise the compound of formula (I) in intimate admixture with a powdered solid inert carrier for example suitable clays, kaolin, bentonite, attapulgite, adsorbent carbon black, talc, mica, silica, chalk, gypsum, tricalcium phosphate, powdered cork, magnesium silicate, vegetable carriers, starch and diatomaceous earths. Such solid formulations are generally prepared by impregnating the solid diluents with solutions of the compound of formula (I) in volatile solvents, evaporating the solvents and, if desired, grinding the products so as to obtain powders and, if desired, granulating, compacting or encapsulating the products.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 99.5% w/v of the composition and may be selected from kerosene, ketones, alcohols, xylene, aromatic naphtha, water, mineral oil, aromatic and aliphatic esters, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates, soaps, lecithins, hydrolysed glues, etc.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 0.5 to 99.5% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (I) in intimate admixture

with a dispersing agent and one or more surface active agents.

Aqueous suspensions of a compound of Formula (I) may comprise a suspension in water together with suspending, stabilizing or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of Formula (I) is preferably distributed uniformly through the mixture in solution or suspension. Grease may also be made from emulsifiable concentrates by diluting them with an ointment base.

Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of Formula (I) required for treatment.

Aerosol sprays may be prepared as a simple solution of the active ingredient in the aerosol propellant and co-solvent such as halogenated alkanes, propane, butane, dimethyl ether and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body. Suitably the plastics material is a polyvinyl chloride (PVC).

The concentration of the compound of formula (I) to be applied to an animal, premises, other substrates or outdoor areas will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited will vary according to the compound chosen, the method of application, area of application, concentration of the compound in the applied formulation, factor by which the formulation is diluted and the nature of the formulation.

Undiluted formulations such as pour-on formulations in general will be applied at a concentration in the range from 0.1 to 20.0% w/w and preferably 0.1 to 10%. The amount of compound to be applied to stored products in general will lie in the range of from 0.1 to 20ppm. Space sprays may be applied to give an average initial concentration of 0.001 to 1 mg of compound of formula (I) per cubic metre of treated space.

Compounds of formula (I) are of use in the protection and treatment of plant species, in which case an effective insecticidal, acaricidal or nematocidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (I) and conveniently between 0.1 and 15% by weight of a compound of the formula (I).

Dusts, greases, pastes and aerosol formulations are usually applied in a random fashion as described above and concentrations of 0.001 to 20% w/v of a compound of Formula (I) in the applied formulation may be used.

The compounds of formula (I) have been found to have activity against the common housefly (Musca domestica). In addition certain compounds of formula (I) have activity against other arthropod pests including Myzus persicae, Tetranychus urticae. Plutella xylostella, Culex spp. Tribolium castaneum, Sitophilus granarius, Periplaneta americana and Blattella germanica. The compounds of formula (I) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium,Ceutorhynchus,Rhynchophorus. Cosmopolites, Lissorhoptrus. Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma. Agrotis, Amathes, Wiseana, Tryporyza, Diatrea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops. Phytomyza, Ceratitis, Liriomyza and Melophagus spp.), Phthiraptera (Malophaga e.g. Damalina spp. and Anoplura e.g. Linognathus and Haematopinus spp.) Hemiptera (e.g. Aphis, Bemisia,Phorodon, Aeneolamia, Empoasca, Parkinsiella, Pyrilla, Aonidiella, Coccus, Pseudococcus. Helopeltis, Lygus, Dysdercus, Oxycarenus, Nezara, Aleurodes, Triatoma,

Rhodnius, Psylla, Myzus, Megoura, Phylloxera, Adelyes, Niloparvata, Nephrotettix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta Lasius, Solenopsis or Monomorium spp.), Isoptera (e.g. Odontotermes and Reticulitermes spp.), Siphonaptera (e.g. Ctenocephalides or Pulex spp.), Thysanura (e.g. Lepisma spp.), Dermaptera (e.g. Forficula spp.), Psocoptera (e.g. Peripsocus spp.) and Thysanoptera (e.g. Thrips tabaci),.

Acrarine pests include ticks, e.g. members of the genera Boophilus, Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes, Eutrombicula, Demodex, PanonychusBryobiaand Eriophyes spp.

Nematodes which attack plants and trees of importance to agriculture, forestry, horticulture, either directly or by spreading bacterial, viral, mycoplasma or fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g.R. reniformis); Rotylenchus spp. (e.g. R. robustus); Helicotylenchus spp. (e.g. H. multicinctus); Hemicycliophora spp. (e.g. HU. gracilis); Criconemoides spp. (e.g. C. similis); Trichodorus spp. (e.g. T primitivus); dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi); stem and bulb eelworms such as Diylenchus spp. (e.g. D. dipsaci).

Compounds of the invention may be combined with one or more other pesticidally active ingredients (for example pyrethroids, carbamates lipid amides and organophosphates) and/or with attractants, repellents, bacteriocides, fungicides, anthelmintics and the like. Furthermore, the activity of compounds of the invention may be enhanced by the addition of a synergist or potentiator, for example: one of the oxidase inhibitor class of synergists, such as piperonyl butoxide, propyl 2-propynylphenylphosphonate; a second compound of the invention; or a pyrethroid pesticidal compound. When an oxidase inhibitor synergist is present in a formulation of the invention, the ratio of synergist to compound of Formula (I) will be in the range 500:1-1:25 eg about 100:1 to 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilisers and as scavengers.

The following Examples illustrate, in a non-limiting manner, preferred aspects of the invention.

## EXPERIMENTAL

### General Synthetic Methods and Procedures:

Various compounds were synthesised and characterised in accordance with the following experimental procedures.

$^1$H N.m.r. spectra were obtained on a Bruker AM-2650 or WM-300 spectrometer in deuterochloroform solutions with tetramethylsilane as internal standard and are expressed as ppm from TMS, number of protons, number of peaks, coupling constant J Hz.

Mass spectra were obtained on Finnigan 4500 or Hewlett Packard 5985B instruments. Gas-liquid chromatography (g.l.c.) was performed using a Pye Unicam GCD chromatograph fitted with a 3% OV210 column on Gas-Chrom Q and a flame-ionisation detector. Progress of reactions could also be conveniently monitored on plastic sheets (40 x 80 mm) precoated with 0.25 mm layers of silica gel with fluorescent indicator and developed in benzene. Temperatures are in degrees Celsius throughout.

## SECTION I

### Dithianes from 1,3-Dithiols

### Preparation of Intermediates in Dithiane Syntheses

1. Dithiols

(a) General method from 1,3-diol di-methanesulphonates

1,3-Diol di-methanesulphonate (1 mole equivalent) (see (b) for conversion of 1,3-diols to di-methanesulphonates) was added to sodium sulphide nonahydrate (1 mole equivalent) and sulphur (1 mole equivalent) in DMF (2 litres per mole) and heated with stirring for 24 hours. The reaction mixture was cooled and poured into water. The aqueous mixture was extracted with ether. The aqueous layer was acidified with hydrochloric acid and re-extracted with ether and dried over magnesium sulphate. The solvent was removed in vacuo to yield crude 1,2-dithiolane. This crude material was then added dropwise to a stirred suspension of lithium aluminium hydride (0.75 mol/1 mol of initial di-methanesulphonate) in ether under nitrogen. The mixture was refluxed with stirring for 1 hour and, after cooling, water was added carefully. The reaction mixture was then acidified with sulphuric acid (2M) and extracted with diethyl ether. The ether extracts were dried over magnesium sulphate and the solvent removed to give 1,3-dithiol which was obtained as a yellow oil and used without further purification (ref. E.L. Eliel et al J.Org.Chem. 1975, 40(4), 524).

(b) 2-Neopentylpropane-1,3-dithiol

(i) A solution of diethyl neopentylmalonate (20.6g; Brit 795173, Chem. Abs. 53, 229d) in dry ether (100 ml) was added dropwise to a stirred suspension of lithium aluminium hydride (10g) in dry ether (200 ml), under nitrogen. After stirring for 1 hour, water (14ml) was carefully added with cooling. Aqueous sodium hydroxide (2M, 14ml) was then added, followed by water (42ml). The reaction mixture was filtered. The filtrate was dried over magnesium sulphate and evaporated to give 2-neopentylpropane-1,3-diol as a colourless oil.

Mass Spectrum (MS), (electron impact), M + I, 147.

(ii) 2-neopentylpropane-1,3-diol (12g) and dry pyridine (15g) was stirred in dry ether (50 ml). Methanesulphonyl chloride (21g) in dry ether (50ml) was then added dropwise at 0°C. After 2 hours, water (50ml) was added and the organic layer separated, washed with dilute hydrochloric acid, saturated sodium bicarbonate solution, brine and then dried over magnesium sulphate. Evaporation gave 2-neopentylpropane-1,3-diol di-methanesulphonate as a colourless solid.

(iii) 2-neopentylpropane-1,3-diol di-methanesulphonate (16g) was refluxed with potassium thioacetate (15g) in ethanol (200ml) for 3 hours. After cooling the solvent was removed in vacuo. To the residue, water (200ml) and ether (500ml) were added and the mixture shaken to allow the solid to dissolve. The organic layer was separated, washed twice with water, once with brine and dried over magnesium sulphate. Evaporation gave 2-neopentylpropane-1,3-dithiol di-acetate.

Mass Spectrum (MS), (Electron impact), M+I, 263.

(iv) A mixture of 2-neopentylpropane-1,3-dithiol di-acetate (2.89g), 1,2-diaminoethane (0.99g) and ethanol (5ml) were stirred overnight at room temperature under nitrogen. The reaction mixture was then evaporated to give crude product. Water (20ml) and chloroform (20ml) were added. The organic layer was then washed with brine, dried and evaporated to yield 2-neopentylpropane-1,3-dithiol as a yellow oil which was used without further purification.

(c) 2-n-Propyl-1,3-butanedithiol

(i) Sodium hydride (1.07g., 60% dispersion in oil) was added to a stirred solution of benzyl mercaptan (3.2ml in dry dimethylformamide (60ml)) at 0°, under a current of nitrogen. Stirring was maintained for 30 minutes. 2-n-Propyl-1,3-butanediol di-methanesulfonate (4.0g) [prepared from 2-n-propyl-1,3-butanediol (ref: Chem. Abs. 76, 13663n), as described in (b)(ii)above] was added and the stirred mixture was maintained at 70° for 2 hours. The mixture was cooled and poured into water. The aqueous mixture was extracted with hexane. The hexane extracts were washed with water and dried over anhydrous magnesium sulfate. The solvent was removed in vacuo. The residue was purified by chromatography on silica, eluting with hexane. 1g of 2-benzylthio-3-benzylthiomethylhexane was obtained. Mass spectrum (MS) chemical ionization: M + I 345.

(ii) 2-Benzylthio-3-benzylthiomethylhexane (1g) in dry diethyl ether (20ml) was added to liquid ammonia (60ml) at -70°. Sodium (0.7g) was added to the stirred solution and stirring was continued for 1 hour at -70°. The mixture was allowed to warm to room temperature. Solid ammonium chloride (10g), methanol (20ml) and chloroform (400 ml) were added in succession, and the mixture was filtered. The filtrates were concentrated in vacuo. 2-n-Propyl-1,3-butanedithiol was obtained as an oil (0.21g) and was used without further purification.

(d) 2-t-Butyl-2-methoxymethylpropane-1,3-dithiol

A mixture of sodium hydride (60% dispersion in oil, 2g) and diethyl t-butylmalonate (10g, Aldrich) in dry toluene (50ml) was heated at reflux under nitrogen for 2 hours. After cooling, water was added and the solutions separated. The organic solution was dried (magnesium sulphate), evaporated and distilled to give diethyl t-butyl(methoxymethyl)malonate (7g) as a colourless liquid, b.p. 62-5°C at 0.5 mmHg.

2-t-Butyl-2-methoxymethylpropane-1,3-dithiol was prepared from diethyl t-butyl(methoxymethyl)-malonate by an analogous route to that described in (b).

(e) 3-n-Propylpentane-2,4-dithiol

3-n-Propylpentane-2,4-dione (prepared by method of T.M. Shepherd, Chem. and Ind., 1970, 17, 567) was converted into 3-n-propylpentane-2,4-diol by the method described in (b)(i).

The di-methanesulphonate from this diol (method (b)(ii)) was converted into 3-n-propylpentane-2,4-dithiol as described in (a).

The following dithiols were prepared using one of the above routes:-

2-t-Butylpropane-1,3-dithiol (E.L. Eliel et al, J.Amer.Chem.Soc. 1969, 91, 2703) was prepared from 2-t-butylpropane-1,3-diol (E.L.Eliel et al, J.Amer.Chem.Soc,. 1968, 90, 3444.

2-Ethylpropane-1,3-dithiol was prepared from diethyl ethylmalonate (Aldrich).

2-n-Butylpropane-1,3-dithiol was prepared from diethyl-n-butyl malonate (Aldrich).

2-n-Propylpropane-1,3-dithiol was prepared from diethyl n-propylmalonate (Aldrich).

2-Methyl-2-n-propyl-1,3-dithiol was prepared from diethyl 2-methyl-2-n-propylmalonate (Aldrich).

2-(1,1-Dimethylpropyl)propane-1,3-dithiol was prepared from diethyl 2-(1,1-dimethylpropyl)malonate (ref: Chem. Abs, 53, 9054d).

2-s-Butylpropane-1,3-dithiol was prepared from diethyl 2-s-butylmalonate (Brit. 798,563; Chem. Abs., 53, 2096h).

2-Cyclohexylpropane-1,3-dithiol was prepared from 2-cyclohexylpropane-1,3-diol ref: J.A.Marshall et al, J.Org.Chem., 1967, 32(1), 113-8).

2-Phenylpropane-1,3-dithiol was prepared from 2-phenylpropane-1,3-diol (ref: E.L. Eliel et al J.Amer.Chem.Soc. 1968, 90(13), 3444).

## 2.Aldehydes and related compounds used in Dithiane Syntheses

### A. 3-(4-Bromophenyl)prop-2-yn-1-al

(i) 1-Bromo-4-iodobenzene (10g, Aldrich) and propargyl alcohol (2g) were reacted at 25°C in dry triethylamine (50ml) containing copper (I) iodide (10mg) and bis-triphenylphosphine palladium chloride (20mg) for 3 hours. The mixture was diluted with ether (100ml) and the precipitate removed by filtration. The solid was washed with ether and the combined filtrates washed with dilute hydrochloric acid (2M). The organic layer was dried over magnesium sulphate and evaporated to give crude product. 3-(4-Bromophenyl)prop-2-yn-1-ol was purified by column chromatography on silica, eluting with ether.

$^1$H NMR was as follows: 1.95,1H,t; 4.40,2H,d; 7.30,4H,q.

(ii) A solution of dimethyl sulphoxide (3.4ml) in dry dichloromethane (50ml) was added to a stirred solution of oxalyl chloride (2.1ml) in dry dichloromethane (50ml) at -65°C. After stirring under nitrogen for 30 minutes, 3-(4-bromophenyl)prop-2-yn-1-ol (5g) in dry dichloromethane (20ml) was added. After a further 30 minutes triethylamine was added (13ml). The mixture was allowed to warm to room temperature over 2 hours before water (50ml) was added, the organic layer separated, washed with dilute hydrochloric acid, saturated sodium bicarbonate solution and brine and dried over magnesium sulphate. Evaporation gave 3-(4-bromophenyl)prop-2-yn-1-al.

$^1$H NMR was as follows: 7.4,4H,m; 9.2,1H,s.

### B. 1,1-Diethoxy-3-phenylprop-2-yne

A solution of phenylacetylene (10g, Aldrich) in dry ether (50ml) was added to a solution of ethyl magnesium bromide (33ml of a 3M solution in ether) with stirring under nitrogen. The mixture was stirred for one hour.

Triethyl orthoformate (16.3ml) in ether (50ml) was added dropwise and the mixture refluxed for 4 hours. After cooling, saturated ammonium chloride solution was added carefully, the ether layer separated, washed with brine and dried over magnesium sulphate. Evaporation gave 1,1-diethoxy-3-phenylprop-2-yne which was purified by distillation (B.pt 142°C at 20 mmHg).

C 4,4-Dimethylpent-1-yn-3-one

(i) Trimethylacetaldehyde (2g, Aldrich) was added over 15 minutes to a stirred suspension of lithium acetylide-ethylenediamine complex (2.4g) in dry THF (20ml) at 35°C under nitrogen. After stirring at room temperature for 3 days, water (5ml) was added and the mixture heated under reflux for 1 hour. The mixture was cooled, separated and the aqueous phase extracted with ether. The combined organic solution was washed with brine and dried (Magnesium sulphate). Distillation gave 4,4-dimethylpent-1-yn-3-ol (0.5g), b.p. 75°C at 30 mmHg, $n_D$ 1.4512.

(ii) A solution of dimethyl sulphoxide (1g) in dichloromethane (5ml) was added over ten minutes to a stirred solution of oxalylchloride (0.8g) in dichloromethane (10ml) at -65°C under nitrogen. A solution of 4,4-dimethylpent-1-yn-3-ol (0.45g) in dichloromethane (5ml) was added over 30 minutes, maintaining the temperature below -60°C. After stirring for 1 hour triethylamine (15ml) was added and the mixture allowed to warm to room temperature over 2 hours. Water was added and the solutions separated. The organic solution was washed with dilute hydrochloric acid, saturated sodium bicarbonate solution, water and then dried over magnesium sulphate. Evaporation gave 4,4-dimethylpent-1-yn-3-one, 200mg, $n_D$ = 1.4525.

D. 4′-Bromo-2,2,2-trifluoroacetophenone

A solution of 1,4-dibromobenzene (22.1g, Aldrich) in dry ether (150ml) was added dropwise to a stirred suspension of magnesium turnings (2.25g) in dry diethyl ether (10ml) containing a crystal of iodine. The mixture was heated at reflux for 30 minutes when a solution of trifluoroacetic acid (3.56g) in ether (20ml) was added dropwise. After stirring at room temperature overnight water and dilute hydrochloric acid were added. The solutions were separated and the organic phase washed with sodium bicarbonate solution, brine, dried over magnesium sulphate and evaporated. Chromatography on silica with ether was eluent yielded 4′-bromo-2,2,2-trifluoroacetophenone,(4g) as a yellow oil (Mass spectrum, M+I, 253).

Preparation of Dithianes from 1,3-Dithiols

Method I

General procedure for condensing aldehydes and ketones with substituted propane-1,3-dithiol

To the dithiol (about 1.0ml) weighed accurately into a 10ml stoppered flask, was added an equivalent proportion plus ca 10% of aldehyde (usually measured by volume), followed by formic acid (5ml, 95-97%). The reaction mixture was then stoppered firmly and stirred from 2-18 hours. If sold separated, water was added and the product was collected by filtration, dried and recrystallised from hexane. For non-solid products, the formic acid layer was separated from the oil, diluted with 10ml ice/water, and extracted twice with dichloromethane. The organic layers were combined, washed with saturated sodium hydrogen carbonate solution until no more carbon dioxide was evolved, and with saturated sodium chloride, and dried over magnesium sulphate. After evaporating the solvent, the residue was distilled at 0.2-0.5 mm to give 1.0 - 1.3 g of product.

Method II

5-t-Butyl-2-(2-phenylethynyl)-1,3-dithiane

Boron trifluoride etherate (0.86ml) was added to a solution of acetic acid (2ml) in chloroform (4ml) stirred under nitrogen and heated to reflux. A solution of 2-t-butylpropane-1,3-dithiol (1.0g) and 1,1-diethoxy-3-phenyl-prop-2-yne (1.36g) in chloroform (10ml) was added dropwise over 30 minutes and heating continued for a further 1 hour. After cooling water was added and the mixture separated. The organic phase was washed with 10% sodium hydroxide solution, brine, dried over magnesium sulphate and evaporated. Chromatography on silica eluting with hexane yielded both isomers of 5-t-butyl-2-(2-phenylethynyl)-1,3-dithiane as pale yellow solids.

Method III

2-[2-(4-Bromophenyl)ethynyl]-5-t-butyl-1,3-dithiane

A mixture of 3-(4-bromophenyl)-prop-2-yn-1-al (4.0g), 2-t-butylpropane-1,3-dithiol (3.1g) and p-toluenesulphonic acid (100mg) in benzene (100ml) was refluxed in a Dean and Stark apparatus for 6 hours. After cooling the mixture was poured into water and the aqueous mixture was extracted with ether. The organic extracts were washed with water, dried over anhydrous magnesium sulphate and evaporated in vacuo. Chromatography on silica gel, eluting with hexane, gave both isomers of 2-[2-(4-bromophenyl)-ethynyl]-5-t-butyl-1,3-dithiane as colourless crystalline solids.

Method IV)

5(e)-t-Butyl-2(e)-(3-chlorobenzyl)-1,3-dithiane

5-t-Butyl-1,3,2-dithiaborinane-dimethyl sulphide complex was prepared by analogy to the method described by S.Kim et al, J. Org. Chem., 1987, 52(10), 2114-2116.

To anhydrous stannous chloride (2.3g) in dry tetrahydrofuran (10ml), was added 3-chlorophenylacetic acid (1g). The mixture was stirred at room temperature for 20 minutes. 5-t-Butyl-1,3,2-dithiaborinane-dimethyl sulphide complex (0.2 M; 50 ml) was added via syringe over 15 minutes. The resulting mixture was stirred overnight.

Potassium hydroxide solution (10% aqueous, 90ml) was carefully added. Ether (100ml) was then added and the two layers shaken and separated.

The organic layer was washed with brine (1 x 50ml) and dried over magnesium sulphate. The crude material was purified by "flash" column chromatography and eluted with hexane and up to 2% ether in hexane.

5(e)-t-Butyl-2(e)-(3-chlorobenzyl)-1,3-dithiane was obtained as a colourless solid.

Method V

5(e)-t-Butyl-2-(4-bromophenyl)-2-trifluoromethyl-1,3-dithiane

4-Bromotrifluoroacetophenone (1.5g), 2-t-butylpropane-1,3-dithiol (1g) and a catalytic amount of aluminium trichloride in xylene (100ml) were heated at reflux under Dean and Stark conditions for 4 hours. After cooling the mixture was filtered and the filtrate concentrated and chromatographed on silica eluting with hexane:ether; 5:1 to give 5(e)-t-butyl-2-(4-bromophenyl)-2-trifluoromethyl-1,3-dithiane as a yellow solid.

SECTION II

Dithianes Prepared from Dithiane Precursors

Example 1

5(e)-t-Butyl-2(e)-(4-pyridyl)-1,3-dithiane

A solution of 5-t-butyl-1,3-dithiane (1.1g, ref: E.L.Eliel et al, J.Amer.Chem.Soc., 1976, 98(12) 3583) in dry tetrahydrofuran (10ml) was cooled to -30° and stirred under nitrogen while 1.6M n-butyllithium in hexane (5.7ml) was added. After 1 hour pyridine (0.5g) was added and the solution stirred overnight at room temperature. Water and ether were added and the mixture separated. The organic phase was washed with brine, dried over magnesium sulphate, evaporated and the residue chromatographed on silica eluting with diethyl ether:hexane; 1:9. 5(e)-t-Butyl-2(e)-(4-pyridyl)-1,3-dithiane (0.25g) was obtained as a colourless solid.

Example 2

5(e)-t-Butyl-2(a)-(4-bromophenyl)-2(e)-methylthio-1,3-dithiane

5(e)-t-Butyl-2(e)-(4-bromophenyl)-1,3-dithiane (0.5g, prepared from 2-t-butylpropane-1,3-dithiol and 4-bromobenzaldehyde as in Section I, Method III) was added to a solution of diisopropylamine (0.21ml) and 1.6M n-butyllithium in hexane (1.13ml) in dry tetrahydrofuran (50ml) stirred at -70° under nitrogen. After 0.5 hours dimethyl disulphide (0.26ml) in tetrahydrofuran (5ml) was added and the mixture warmed to room temperature. Chloroform (100ml) was added and the mixture washed with brine, dried over magnesium sulphate and evaporated to dryness. Chromatography on silica eluting with hexane:diethyl ether; 10:1

yielded 5(e)-t-butyl-2(a)-(4-bromophenyl)-2(e)-methylthio-1,3-dithiane (0.38g) as a white solid.

Example 3

5(e)-t-Butyl-2(e)-(4-bromophenyl)-2(a)-methylthio-1,3-dithiane

5(e)-t-Butyl-2(a)-(4-bromophenyl)-2(e)-methylthio-1,3-dithiane (0.35g) in dry chloroform (50ml) was stirred under nitrogen while boron trifluoride etherate (0.1ml) was added. After stirring at room temperature for 2 hours water was added and the solution separated, washed with saturated sodium hydrogen carbonate solution, dried over magnesium sulphate and evaporated to dryness to give 5(e)-t-butyl-2(e)-(4-bromophenyl)-2(a)methylthio-1,3-dithiane (0.13g) as a white solid.

Example 4

5(e)-t-Butyl-2(e)-(4-bromophenyl)-2(a)-cyano-1,3-dithiane

A solution of trimethylsilyl cyanide (0.13g), 5(e)-t-butyl-2(a)-(4-bromophenyl)-2(e)-methylthio-1,3-dithiane (0.35g) in dry chloroform was stirred under nitrogen whilst boron trifluoride etherate (0.1ml) was added. After heating at reflux for 1 hour the solution was cooled and water added. The organic solution was separated, washed with saturated sodium carbonate solution, brine, dried over magnesium sulphate and evaporated to dryness. Crystallisation from hexane gave 5(e)-t-butyl- 2(e)-(4-bromophenyl-2(a)-cyano-1,3-dithiane (0.12g) as a white solid.

Example 5

5(e)-t-Butyl-2(e)-(4-bromophenyl)-2(a)-methoxy-1,3-dithiane

i) A solution of 4-bromobenzoyl chloride (5.5g) and 2-t-butylpropan-1,3-dithiol (4.1g) was stirred under nitrogen for 30 minutes and 70% perchloric acid (3ml) was added dropwise with cooling. After a further 30 minutes acetic anhydride (20ml) was added with cooling and the mixture stirred for 1 hour. Ether (100ml) was added and the mixture filtered and the brown residue of 2-(4-bromophenyl)-5-t-butyl-1,3-dithian-2-ylium perchlorate (0.9g.) was dried in vacuo.

ii) Triethylamine (5ml) was added to a solution of 2-(4-bromophenyl)-5-t-butyl-1,3-dithian-2-ylium perchlorate (0.45g) in dry methanol (20ml). After stirring for 2 hours the precipitate was filtered and dried to give 2(e)-(4-bromophenyl)-5(e)-t-butyl-2(a)-methoxy-1,3-dithiane (0.2g).

2(e)-Bromophenyl)-5(e)-t-butyl-2(a)-ethoxy-1,3-dithiane was prepared in a directly analogous manner by using dry ethanol in stage ii).

Example 6

2-(4-Bromophenyl)-5(e)-t-butyl-2-vinyl-1,3-dithiane

A solution of vinyl magnesium bromide in tetrahydrofuran (Aldrich, 1.5ml of a 1M solution) was added to a solution of 2-(4-bromophenyl)-5-t-butyl-1,3-dithian-2-ylium perchlorate (from Example 5, stage i)), (0.62g), in dry dichloromethane (20ml) under nitrogen at -70 °C. After allowing to warm to room temperature dichloromethane (100ml) was added and the mixture washed with water then brine. Drying (magnesium sulphate) and evaporation of the solvent gave a yellow oil (0.44g). This was chromatographed (silica, ether:hexane, 1:20) to give 5(e)-t-butyl-2-(4-bromophenyl)-2-vinyl-1,3-dithiane as a yellow oil.

5(e)-t-Butyl-2-(4-bromophenyl)-2-ethynyl-1,3-dithiane was prepared in a directly analogous manner from ethynyl magnesium bromide.

BIOLOGICAL ACTIVITIES OF DITHIANES

The following examples illustrate in a non-limiting manner, the pesticidal activity of compounds of formula (I).

Bioassay Procedure and Results

The compounds of the invention were tested by dissolving in acetone (5%) and then diluting in water: 'Synperonic' (94.5% : 0.5%) to give a water emulsion. The solution was then used to treat the following insects.

Musca domestica:

20 female Musca were contained in a cardboard cylinder with gauze over both ends. Solution containing the compound was sprayed onto the insects so enclosed and mortality assessed after 48 hours at 25°.

Sitophilus granarius:

20 adult Sitophilus were added to 10g wheat which had been previously treated with 2ml of the solution containing the compound. Mortality is assessed after 6 days at 25°.

Plutella xylostella:

7 Plutella larvae (3rd instar) were sprayed with the solution containing the compound and added to a Chinese cabbage leaf which had been similarly sprayed and left to dry. Mortality was assessed after 2 days at 25°.

Myzus persicae:

10 adult Myzus were placed on a leaf disc of chinese cabbage. 24 Hours later the disc was sprayed with the solution containing the compound. Mortality is assessed after 2 days at 25°.

Tetranychus urticae:

Leaf discs of infested french bean were sprayed with the solution containing the compound. Mortality was assessed after 2 days at 25°.

The following compounds were active against M.domestica at less than 1000 ppm
1, 28, 27, 32, 25, 11

The following compounds were active against S.granarius at less than 1000 ppm
1, 9, 46

The following compound was active against S.granarius at less than 200 ppm
34

The following compounds were active against P.xylostella at less than 1000 ppm
52, 3, 53, 54

The following compounds were active against M.persicae at less than 1000 ppm
24, 46, 5

The following compounds were active against T.urticae at less than 1000 ppm
3, 7, 43, 54, 10

Additional spray tests

The activity of the compounds of the invention was further tested by dissolving the compounds in acetone (75%) and then adding water (25%). The solution was then used to spray the following insects.

Tetranychus urticae

A mixed population of T.urticae was tested on bean leaves.

The following compound was active at less than 1000 ppm
52

Aphis fabae

A mixed population of Aphis fabae was tested on nasturtium leaf.

The following compounds were active at less than 1000 ppm
25, 2, 3, 32

Macrosteles fascifrons

Adult M.fascifrons were tested on wheat seedlings.

The following compound was active at less than 1000 ppm

34

Diabrotica undecimpunctata

3rd Instar D. undecimpunctata were tested on filter paper.

The following compounds were active at less than 1000 ppm

1,32

## Formulations

### 1. Emulsifiable Concentrate

| | |
|---|---|
| Compound of formula (I) | 10.00 |
| Ethylan KEO | 20.00 |
| Xylene | 67.50 |
| Butylated Hydroxyanisole | 2.50 |
| | 100.00 |

### 2. Wettable Powder

| | |
|---|---|
| Compound of formula (I) | 25.00 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

### 3. Dust

| | |
|---|---|
| Compound of formula (I) | 0.50 |
| Butylated Hydroxyanisole | 0.10 |
| Talc | 99.40 |
| | 100.00 |

### 4. Bait

| | |
|---|---|
| Compound of formula (I) | 40.25 |
| Icing Sugar | 59.65 |
| Butylated hydroxy toluene | 0.10 |
| | 100.00 |

5. Aerosol

| | |
|---|---|
| Compound of formula (I) | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

6. Spray

| | |
|---|---|
| Compound of formula (I) | 0.1 |
| Butylated Hydroxyanisole | 0.1 |
| Xylene | 10.0 |
| Odourless Kerosene | 89.8 |
| | 100.00 |

7. Potentiated Spray

| | |
|---|---|
| Compound of formula (I) | 0.1 |
| Piperonyl Butoxide | 0.5 |
| Butylated Hydroxyanisole | 0.1 |
| Xylene | 10.1 |
| Odourless Kerosene | 89.2 |
| | 100.00 |

Appendix 1

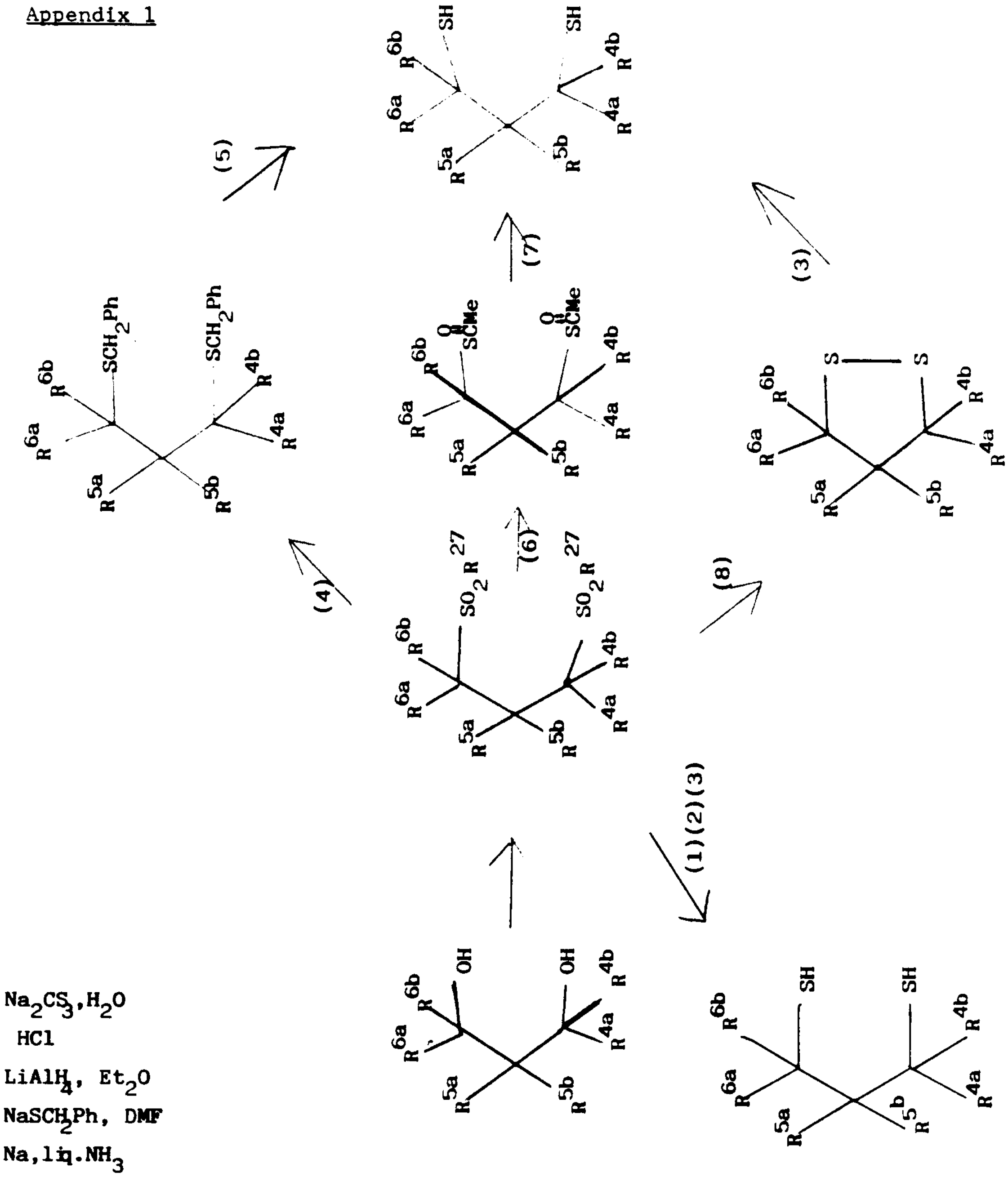


(1) $Na_2CS_3, H_2O$

(2) HCl

(3) $LiAlH_4$, $Et_2O$

(4) $NaSCH_2Ph$, DMF

(5) Na, liq. $NH_3$

(6) KSC(O).Me, EtOH

(7) $H_2NCH_2CH_2NH_2$, EtOH

(8) $Na_2S/S$, DMF

Appendix 2

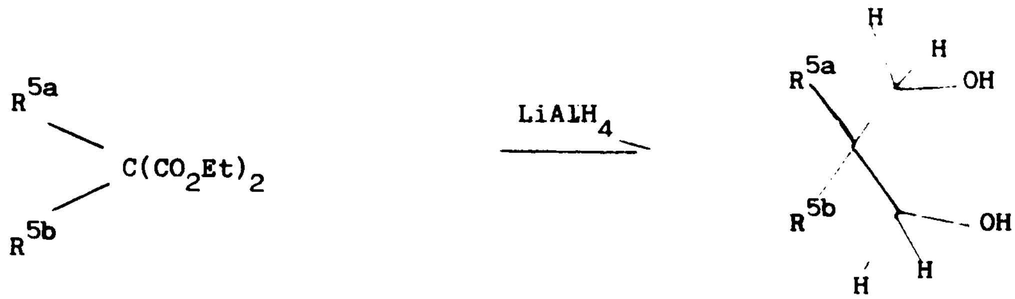


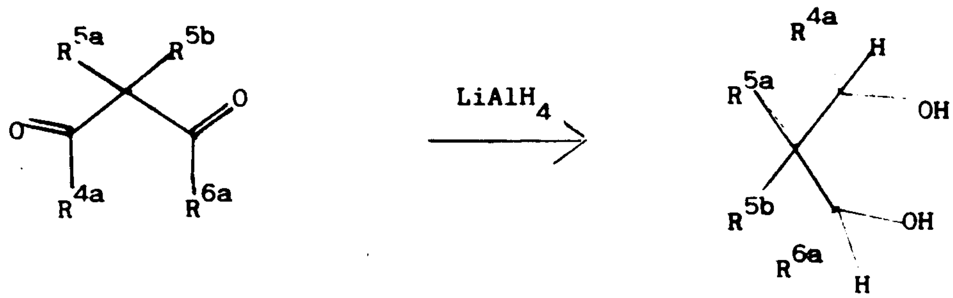


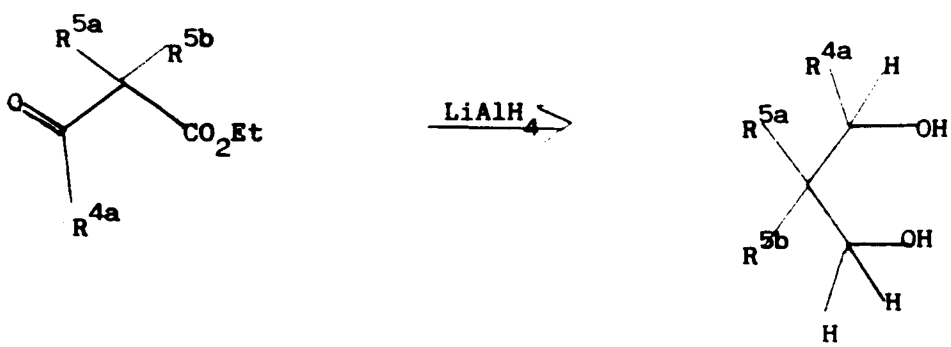

Appendix 3

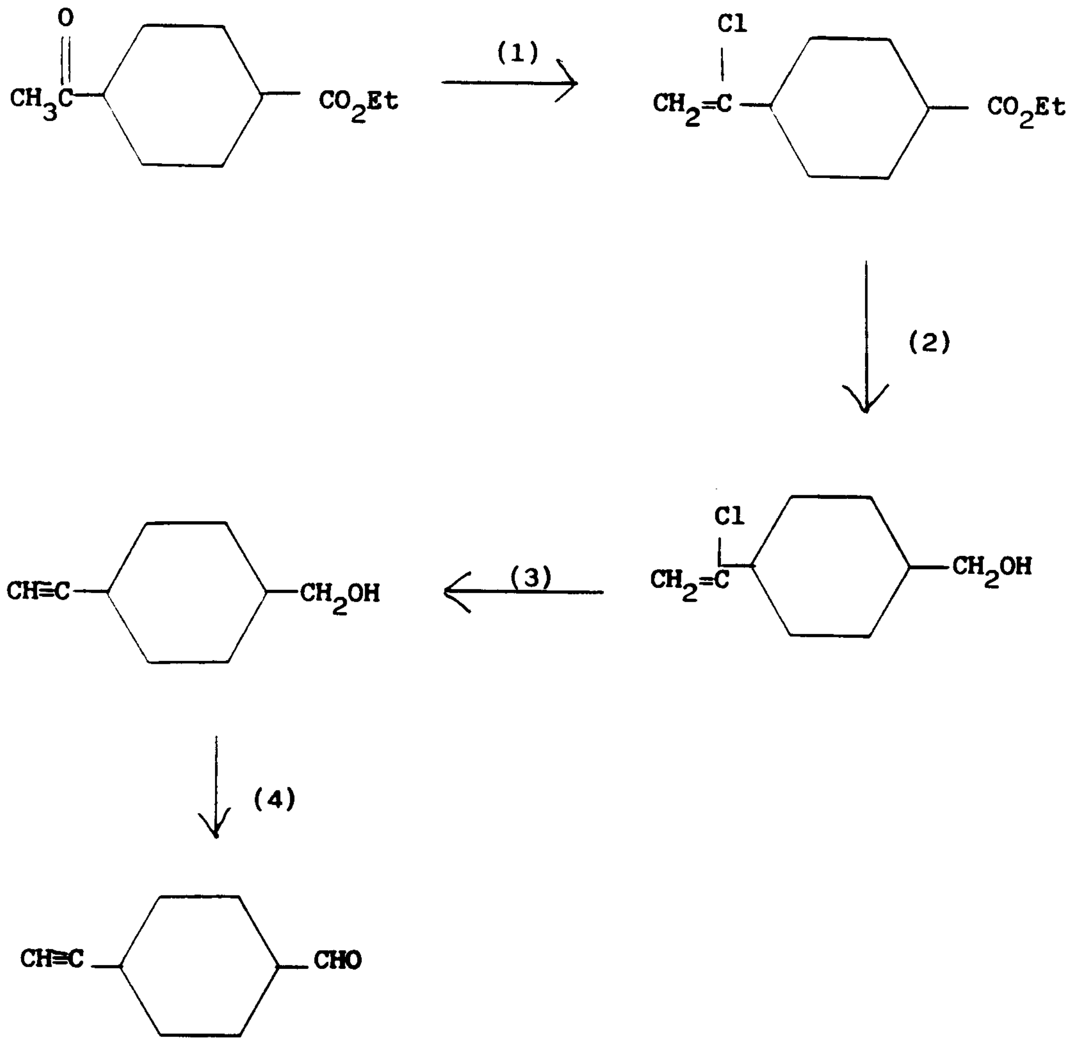


(1) $PCl_5$,pyridne (2) $LiAlH_4$,$Et_2O$ (3) n-BuLi, Thf

(4) oxalyl chbride, $CH_2Cl_2$,DMSO,$NEt_3$

**Table 1A** **Dithianes Prepared from 1,3-dithiols**

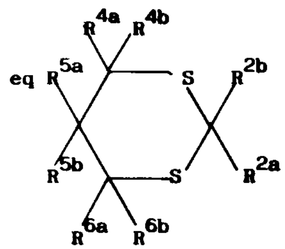

where $R^{5b} = R^{4a} = R^{4b} = R^{6a} = R^{6b} = H$

| Compound No. | $R^{2a,b}$ | eq $R^{5a}$ | Isomer Ratio | Starting Material | Method of Dithiane Synthesis |
|---|---|---|---|---|---|
| 1 | eq.4-Bromophenyl; ax.H | Et | | 4-Bromobenzaldehyde[1] | III |
| 2 | ax.4-Bromophenyl;eq.Me | $EtMe_2C$ | | 4-Bromoacetophenone[1] | III |
| 3 | eq.4-Bromophenyl;ax.Me | $EtMe_2C$ | | 4-Bromoacetophenone[1] | III |
| 4 | eq.4-Bromophenyl; ax.H | $EtMe_2C$ | | 4-Bromobenzaldehyde[1] | III |
| 5 | i) eq.4-Bromophenyl;ax.H<br>ii) ax.4-Bromophenyl;eq.H | n-Bu<br>n-Bu } | 1:3 | 4-Bromobenzaldehyde[1] | III |

| | | | | | |
|---|---|---|---|---|---|
| 6 | i) eq.4-Bromophenyl;ax.Me | n-Bu | 1:1 | 4-Bromoacetophenone[1] | III |
| | ii) ax.4-Bromophenyl;eq.Me | n-Bu | | | |
| 7 | eq.4-Bromophenyl; ax.H | t-BuCH$_2$ | | 4-Bromobenzaldehyde[1] | III |
| 8 | i) eq.4-Bromophenyl;ax.Me | t-BuCH$_2$ | 1:1 | 4-Bromoacetophenone[1] | III |
| | ii) ax.4-Bromophenyl;eq.Me | t-BuCH$_2$ | | | |
| 9 | eq.2,2-Dibromoethenyl; ax.H | t-Bu | | 3,3-Dibromopropenal[2] diethylacetal | II |
| 10 | i) eq.4-Bromophenyl;ax.H | s-Bu | 1:2 | 4-Bromobenzaldehyde[1] | III |
| | ii) ax.4-Bromophenyl;eq.H | s-Bu | | | |
| 11 | i) eq.4-Bromophenyl;ax.H | n-Pr | 2:1 | 4-Bromobenzaldehyde[1] | III |
| | ii) ax.4-Bromophenyl;eq.H | n-Pr | | | |
| 12 | i) eq.4-Bromophenyl;ax.H | cyclohexyl- | 2:3 | 4-Bromobenzaldehyde[1] | III |
| | ii) ax.4-Bromophenyl;eq.H | cyclohexyl- | | | |
| 13 | i) ax.4-Bromophenyl;eq.Me | s-Bu | 2:3 | 4-Bromoacetophenone[1] | III |
| | ii) eq.4-Bromophenyl;ax.Me | s-Bu | | | |
| 14 | ax.4-Bromophenyl;eq.Me | cyclohexyl | | 4-Bromoacetophenone[1] | III |
| 15 | i) eq.4-Bromophenyl;ax.Me | cyclohexyl | 1:3 | 4-Bromoacetophenone[1] | III |
| | ii) ax.4-Bromophenyl;eq.Me | cyclohexyl | | | |
| 16 | i) eq.4-Bromophenyl;ax.Me | n-Pr | 1:1 | 4-Bromoacetophenone[1] | III |
| | ii) ax.4-Bromophenyl;eq.Me | n-Pr | | | |
| 18 | eq.4-Bromophenyl;ax.Me | Ph | | 4-Bromoacetophenone[1] | III |
| 19 | eq.4-Bromophenyl; ax.H | Ph | | 4-Bromobenzaldehyde[1] | III |

| | | | | | |
|---|---|---|---|---|---|
| 20 | i) eq.4-Bromophenyl;ax.H | Ph | 1:2 | 4-Bromobenzaldehyde[1] | III |
| | ii) ax.4-Bromophenyl;eq.H | Ph | | | |
| 23 | eq. 2-Naphthyl; ax.H | t-Bu | | 2-Naphthaldehyde[1] | III |
| 24 | eq. 4-Methylphenyl; ax.H | t-Bu | | p-Tolualdehyde[1] | III |
| 25 | eq. 4-Nitrophenyl; ax.H | t-Bu | | 4-Nitrobenzaldehyde[1] | III |
| 30 | eq. 3-Methylphenyl; ax.H | t-Bu | | m-Tolualdehyde[1] | III |
| 31 | i) eq. 4-Bromophenyl; ax. trifluoromethyl | t-Bu | 1:1 | 4'-Bromo-2,2,2-trifluoromethylacetophenone[9] | V |
| | ii) ax. 4-Bromophenyl; eq. trifluoromethyl | t-Bu | | | |
| 33 | i) eq.4-Bromophenyl;ax.Me | Et | 4:1 | 4-Bromoacetophenone[1] | III |
| | ii) ax.4-Bromophenyl;eq.Me | Et | | | |
| 34 | i) eq.4-Bromophenyl;ax.Me | Et | 1:1 | 4-Bromoacetophenone[1] | III |
| | ii) ax.4-Bromophenyl;eq.Me | Et | | | |
| 35 | i) eq.4-Bromophenyl;ax.Me | Et | 1:4 | 4-Bromoacetophenone[1] | III |
| | ii) ax.4-Bromophenyl;eq.Me | Et | | | |
| 37 | eq. Phenylethynyl; ax.H | t-Bu | | 1,1-Diethoxy-3-phenylprop-2-yne[3] | II |
| 38 | ax. Phenylethynyl; eq.H | t-Bu | | 1,1-Diethoxy-3-phenylprop-2-yne[3] | II |

| | | | | | | |
|---|---|---|---|---|---|---|
| 40 | eq. 4-Bromophenylethynyl | t-Bu | | | 3-(4-Bromophenyl)prop-2-yn--1-al[4] | III |
| 41 | ax. 4-Bromophenylethynyl | t-Bu | | | 3-(4-Bromophenyl)prop-2-yn--1-al[4] | III |
| 42 | eq. 3-Chlorobenzyl; ax.H | t-Bu | | | 3-Chlorophenylacetic acid[1] | IV |
| 43 | eq. 2-Bromoethynyl; ax.H | t-Bu | | | 3-Bromopropynal diethyl acetal[5] | II |
| 44 | ax. 2-Bromoethynyl; eq.H | t-Bu | | | 3-Bromopropynal diethyl acetal[5] | II |
| 45 | eq. 2,2-Diiodoethenyl; ax.H | t-Bu | | | 3,3-Diiodopropenal diethyl acetal[5] | II |
| 46 | eq. 3,3-Dimethylbutyl; ax.H | Et | | | 4,4-Dimethylpentanal[6] | III |
| 47 | eq. (E)Styryl; ax.H | t-Bu | | | Cinnamaldehyde[1] | I |
| 50 | i) eq. 2-Phenylethyl; ax.H<br>ii) ax. 2-Phenylethyl; eq.H | t-Bu<br>t-Bu | } | 1:1 | Hydrocinnamaldehyde[1] | III |
| 52 | eq. 4-t-Butylbenzaldehyde; ax.H | t-Bu | | | 4-t-Butylbenzaldehyde[7] | III |
| 53 | i) eq. 2-oxopropyl; ax.H<br>ii) ax. 2-oxopropyl; eq.H | t-Bu<br>t-Bu | } | 4:1 | 3-Butyn-2-one[1] | III |
| 54 | eq. 3,3-Dimethyl-2-oxobutyl; ax.H | t-Bu | | | 4,4-Dimethylpent-1-yn-3-one[8] | III |

<u>REFERENCES</u> (TABLE 1A)


1. Commercially available from Aldrich.

2 M.Levas and E.Levas <u>Bull.Soc.Chim.Fr.</u>, 1959, 1800-6.

3. Process B.

4. Process A.

5. M.A.Gorgaes <u>C.R.Acad.Sc.Paris C</u>, 1967, <u>265</u>, 1130.

6. R.Quelet <u>et al</u> <u>C.R.Acad.Sc.Paris C</u>, 1965, <u>260</u>, 1191.

7. Commercially available from Lancaster.

8. Process C.

9. Process D.

**Table 1B** **Dithianes prepared from 1,3-Dithiols**

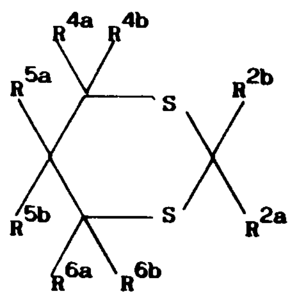

where $R^2$ = ax H
$R^{4b} = R^{6b}$ = H
$R^{2b}$ = eq 4-bromophenyl
starting materials 4-bromobenzaldehyde and appropriate dithiol from Section I

| Compound Number | $R^{5a,5b}$ | $R^{4a}$ | $R^{6a}$ | Isomer Ratio | Method of Synthesis |
|---|---|---|---|---|---|
| 17 | eq. n-Pr; ax.H | Me | H | 4 isomers | III |
| 21 | eq. n-Pr; ax.H | eq.Me | eq.Me | | III |
| 22 | eq. n-Pr; ax.H | eq.Me | ax.Me | | III |
| 48 | i) eq. n-Pr; ax.Me | H | H | 1:1 | III |
| | ii) ax. n-Pr; eq.Me | H | H | | |
| 51 | eq. t-Bu; ax.$MeOCH_2$- | H | H | | III |

**Table 1C** **Structures and Methods for Compounds Prepared from Dithiane Precursors**

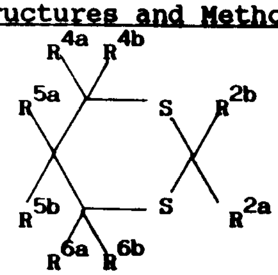

where $R^{5a}$ = t-Bu, $R^{5b}$ = H
$R^{4a} = R^{4b} = R^{6a} = R^{6b}$ = H

| Compound Number | $R^{2a,2b}$ | Isomer Ratio | Synthetic Method Example |
|---|---|---|---|
| 26 | ax. 4-Bromophenyl; eq. methylthio | | Example 2 |
| 27 | eq. 4-Bromophenyl; ax. methylthio | | Example 3 |
| 28 | eq. 4-Bromophenyl; ax. methoxy | | Example 5 |
| 29 | i) eq. 4-Bromophenyl; ax. vinyl<br>ii) ax. 4-Bromophenyl; eq. vinyl | 1:1 | Example 6 |
| 32 | eq. 4-Bromophenyl; ax. cyano | | Example 4 |
| 36 | eq. 4-Bromophenyl; ax. ethoxy | | Example 5 |
| 39 | i) eq. 4-Bromophenyl; ax. ethynyl<br>ii) ax. 4-Bromophenyl; eq. ethynyl | 3:1 | Example 6 |
| 49 | eq. 4-Pyridyl; ax. H | | Example 1 |

TABLE 2 Nuclear Magnetic Resonance Spectra:- $^{1}H$ carried out in $CDCl_3$ and expressed as p.p.m. downfield from TMS, (number of protons, multiplicity, JHz, assignment).

1. 1.0,3H,t; 1.4,2H,q; 1.8,1H,tt; 2.7-3.0,4H,m; 5.1,1H,s; 7.3-7.6,4H,m.

2. 0.8,6H,s; 0.9,3H,t; 1.2,2H,q; 1.6,3H,s; 2.4,2H,dd; 2.7,2H,dd; 7.5,2H,d; 7.8,2H,d.

3. 0.8,3H,t; 0.9,6H,s; 1.3,2H,q; 1.8,1H,tt; 2.2,3H,s; 2.8,2H,dd; 3.1,2H,dd; 7.4,2H,d; 7.7,2H,d.

4. 0.8,3H,t; 0.9,6H,s; 1.3,2H,q; 1.8,1H,tt; 2.8-3.0,4H,m; 5.1,1H,s; 7.3-8.6,4H,dd.

5. 0.9,3H,m; 1.3,6H,m; 1.9,1H,m; 2.6-3.2,4H,m; 5.05 and 5.08,1H,s; 7.4,4H,m.

6. 0.9,3H,m; 1.2,6H,m; 1.7,1H,m; 1.7 and 1.85, 1H,s; 2.25-2.85,4H,m; 7.6, 4H,m.

7. 0.95,9H,s; 1.25,2H,m; 1.9,1H,m; 2.8,4H,m; 7.52,4H,m.

8. 0.93,9H,s; 1.55 and 1.67,1H,s; 1.9,1H,m; 2.3-2.8,4H,m; 7.6,4H,m.

9. 0.95,9H,s; 1.65,1H,tt; 2.8,2H,dd; 2.9,2H,dd; 4.85,1H,d; 6.35,1H,d.

| | |
|---|---|
| 10. | 0.90(6H,m,MeCH$_2$CHMe), 1.10-2.10(4H,m,5-H$_{ax}$ and MeCH$_2$CHMe) 2.60-3.20(4H,m,-SCH$_2$), 5.08 and 5.10 (1H, two singlets, 2-H), 7.30-7.70 (4H,m,ArH). |
| 11. | 0.95(3H,m,CH$_2$Me) 1.20-2.10(5H,m,5-H$_{ax}$ and CH$_2$CH$_2$), 2.60-3.30(4H,m,SCH$_2$), 5.00 and 5.05(1H, two singlets in the ratio 1:2, 2-H), 7.40(4H,m,ArH). |
| 12. | 0.8-1.9(12H,m,5-H$_{ax}$+ C$_6$H$_{11}$), 2.70-3.20(4H,m,SCH$_2$), 5.08 and 5.12(1H,two singlets, 2-H), 7.40(4H,m,ArH). |
| 13. | 0.70-1.40 (9H,m,MeCH$_2$CHMe), 1.60-2.00(4H,m,2-Me and 5-H$_{ax}$), 2.35-3.00(4H,m,-SCH$_2$), 7.50(2H,m,ArH), 7.82(2H,m,ArH). |
| 14. | 0.8-1.8(12H,m,5-H$_{ax}$ and C$_6$H$_{11}$), 1.70(3H,s,2-Me$_{eq}$), 2.45(2H,dd,J 14.3 and 11.2 Hz, 4 and 6-H$_{ax}$), 2.65(2H,dd,J 14.3 and 2.9 Hz,4 and 6-H$_{eq}$), 7.50(2H,d,J 7Hz,ArH), 7.85(2H,d,J 7Hz,ArH). |
| 15. | 0.8-1.9(15H,m,2-Me,5-H$_{ax}$ and C$_6$H$_{11}$), 2.30-3.00(4H,m,SCH$_2$), 7.50(2H,m,ArH), 7.85(2H,m,ArH). |
| 16. | 0.90(3H,m,MeCH$_2$), 1.00-2.00(8H,m,2-Me,5-H$_{ax,}$ and CH$_2$CH$_2$), 2.20-2.90(4H,m,SCH$_2$), 7.50(2H,m,ArH), 7.82(2H,m,ArH). |

| | |
|---|---|
| 17 | 0.85-2.40(11H,m,5-H,4-Me and $\underline{CH_2CH_2Me}$), 2.5-3.45(3H,m,$SCH_2$ and -S$\underline{CH}$Me), 5.1,5.17,5.25 and 5.3(1H, four singlets, 2-H), 7.3-7.5(4H,m,ArH). |
| 18. | 2.3,3H,s; 2.8,2H,dd; 3.2,1H,m; 3.4,2H,dd; 7.5,9H,m. |
| 19. | 3.0,2H,dd; 3.2,3H,m; 5.2,1H,s; 7.4,9H,m. |
| 20. | 3.2,5H,m; 4.9 and 5.2,1H,s; 7.5,9H,m. |
| 21. | 0.95,3H,t; 1.31,6H,d; 1.3,1H,m; 1.45,2H,m; 1.7,2H,m; 3-3.15,2H,m; 5.22,1H,s; 7.4,4H,m. |
| 22. | 1.0,3H,t; 1.20,3H,d; 1.4-1.5,4H,m; 1.65,3H,d; 2.0-2.1,1H,m; 3.15,1H,dq; 3.58,1H,dq; 5.33,1H,s; 7.40,2H,d; 7.48,2H,d. |
| 23. | 0.99,9H,s; 1.80,1H,tt; 2.88,2H,dd; 3.02,2H,dd; 5.32,1H,s; 7.7,7H,m. |
| 24. | 1.0,9H,s; 1.75,1H,tt; 2.36,3H,s; 2.84,2H,dd; 2.97,2H,dd; 5.1,1H,s; 7.15,2H,d; 7.36,2H,d. |
| 25. | 1.0,9H,s; 1.79,1H,tt; 2.88,2H,dd; 3.02,2H,dd; 5.2,1H,s; 7.67,2H,d; 8.11,2H,d. |
| 26 | 0.85,9H,s; 1.75,1H,tt; 2.3,3H,s; 2.5,2H,dd; 2.7,2H,dd; 7.5,2H,d; 7.95,2H,d. |

27. 0.95,9H,s; 1.70,1H,tt; 1.88,3H,s; 2.75,2H,dd; 3.25,2H,dd; 7.45,2H,d; 7.75,2H,d.

28. 1.0,9H,s; 1.85,1H,tt; 2.8,2H,dd; 3.05,2H,dd; 3.3,3H,s; 7.5,4H,s.

29. 0.82,9H,s; 1.4,1H,m; 2.4-3.1,4H,m; 5.15,2H,dd; 6.4,1H,dd; 7.15,4H,m.

30. 0.95,9H,m; 1.8,1H,m; 2.4,3H,s; 2.9,4H,m; 5.1,1H,s; 7.2,4H,m.

31. i) 0.82,9H,s; 1.8,1H,m; 2.80 and 3.10,4H,m; 7.5-8.0,4H,m.
ii) 0.95,9H,s; 1.8,1H,m; 2.45 and 2.80,4H,m; 7.5-8.0,4H,m.

32. 1.0,9H,s; 1.75,1H,tt; 3.02,2H,dd; 3.22,2H,dd; 7.55,2H,d; 7.70,2H,d.

33. 0.9,3H,m; 1.3,3H,m; 1.6 and 1.7,3H,s; 2.5,4H,m; 7.6,4H,m.

34. 0.8,3H,m; 1.2,2H,m; 1.8,1H,tt; 1.8 and 1.9,3H,s; 2.4-2.8,4H,m; 7.4-7.8,4H,m.

35. 0.9,3H,m; 1.2,1H,m; 1.7,2H,m; 1.8,3H,s; 2.6,4H,m; 7.6,4H,m.

36. 1.0,9H,s; 1.30,3H,t; 1.82,1H,tt; 2.78,2H,dd; 3.10,2H,dd; 3.40,2H,q; 7.52,4H,s.

37. 0.95,9H,s; 1.75,1H,tt; 2.7-3.0,4H,m; 5.05,1H,s; 7.30 and 7.45,5H,m.

38. 1.0,9H,s; 1.8,1H,tt; 2.85,2H,dd; 3.30,2H,dd; 4.60,1H,s; 7.35 and 7.50,5H,m.

39. 0.95 and 1.0,9H,s; 1.4,1H,s; 1.8,1H,t; 2.6-3.4,4H,m; 7.6,4H,m.

40. 0.95,9H,s; 1.75,1H,tt; 2.75-2.95,4H,m; 5.0,1H,s; 7.3,2H,d; 7.45,2H,d.

41. 1.0,9H,s; 1.78,1H,tt; 2.82,2H,dd; 3.25,2H,dd; 4.45,1H,s; 7.30,2H,d; 2.48,2H,d.

42. 0.9,9H,s; 1.6,1H,m; 2.6,2H,t; 2.8,2H,d; 2.9,2H,d; 4.4,1H,t; 7.1-7.3,4H,m.

43. 0.95,9H,s; 1.7,1H,tt; 2.75,2H,dd; 2.9,2H,dd; 4.85,1H,s.

44. 0.95,9H,s; 1.7,1H,tt; 2.8,2H,dd; 3.15,2H,dd; 4.4,1H,s.

45. 0.95,9H,s; 1.65,1H,tt; 2.8,2H,dd; 2.9,2H,dd; 4.55,1H,d; 6.85,1H,d.

46. 0.9,9H,s; 0.9,3H,m; 1.3-1.7,7H,m; 2.5,2H,dd; 2.7,2H,dd; 3.95,1H,t.

| | |
|---|---|
| 48. | 1.03 and 1.30,3Hs; 0.97 and 1.02,3H,m; 0.90-1.90,4H,m; 2.46-2.95,4H,m; 4.99 and 5.01,1H,s; 7.32-7.50,4H,m. |
| 49. | 0.99,9H,s; 1.79,1H,tt; 2.88,2H,dd; 3.0,2H,dd; 5.12,1H,s; 7.42,2H,d; 8.62,2H,d. |
| 51. | 1.0,9H,s; 3.0,4H,m; 3.3,3H,s; 4.1,2H,s; 5.0,1H,s; 7.5,4H,m. |
| 52. | 0.9,9H,s; 1.3,9H,s; 1.8,1H,tt; 2.7-3.0,4H,m; 5.2,1H,s; 7.3-7.5,4H,dd. |
| 53. | 0.9,9H,s; 1.6,1H,m; 2.18 and 2.2,3H,s; 2.7,6H,m; 4.1 and 4.45,1H,m. |
| 54. | 0.9,9H,s; 1.1,9H,s; 1.6,1H,m; 2.8,6H,m; 4.5,1H,m. |

**Table 3** **Dithianes:- Further Characterising Data**

| Compound Number | Mass Spectrum:- Chemical Ionisation M+1 * Electron impact M | m.p. °C or refractive index $n_D$ | Description |
|---|---|---|---|
| 1 | - | 135 | Colourless Solid |
| 2 | 359 | 96 | " |
| 3 | 359 | 126 | " |
| 4 | - | 114 | " |
| 5 | 331 | - | " |
| 6 | 345 | - | Colourless Oil |
| 7 | 345 | - | Colourless Solid |
| 8 | 359 | - | " |
| 9 | 359 | 86-7 | " |
| 10 | 331 | 74 | Colourless Crystals |
| 11 | 317 | 103 | Pale yellow crystalline solid |
| 12 | 357 | 126 | " " " |
| 13 | 345 | - | Colourless oil |

| | | | |
|---|---|---|---|
| 14 | 371 | - | Colourless oil |
| 15 | 371 | - | " |
| 16 | 331 | - | " |
| 17 | 331 | - | Oily solid |
| 18 | 365 | 144 | Colourless solid |
| 19 | 351 | 199 | " |
| 20 | 351 | - | " |
| 21 | 345 | - | " |
| 22 | 345 | - | yellow solid |
| 23 | - | 209°C | off-white solid |
| 24 | - | 187-90 | cream crystals |
| 25 | - | - | light brown solid |
| 26 | - | 156 | white solid |
| 27 | 377 | 94 | " |
| 28 | - | 141 | colourless solid |
| 29 | 357 | - | yellow oil |
| 30 | - | 121 | colourless solid |
| 31 | 399 | 128 | yellow solid |
| 32 | 356 | 192 | white solid |
| 33 | - | - | low melting solid |
| 34 | - | - | " |

| | | | |
|---|---|---|---|
| 35 | - | - | yellow oil |
| 36 | 375 | 111 | colourless solid |
| 37 | 277 | 133 | pale yellow solid |
| 38 | 277 | 101-6 | " |
| 39 | 355 | 47.2 | yellow solid |
| 40 | 355 | 251 | colourless crystals |
| 41 | 355 | 153 | " |
| 42 | - | - | colourless solid |
| 43 | 279 | 93 | yellow solid |
| 44 | 279 | 91 | colourless solid |
| 45 | 455 | 125 | " |
| 46 | 233 | - | colourless oil |
| 47 | 279 | 144-6 | colourless solid |
| 48 | 331 | 95-103 | colourless solid |
| 49 | - | 122-4 | " |
| 50 | 281 | 48-50 | " |
| 51 | 375 | 102 | colourless solid |
| 52 | 309 | 158 | colourless solid |
| 53 | 233 | ($n_D$ 1.5274) | yellow oil |
| 54 | 275 | 130-3 | colourless solid |

## Claims

**Claims for the following Contracting States : AT, BE, CH, DE, FR, GB, IT, LI, LU, NL, SE**

**1.** A compound of the formula (I):

(I)

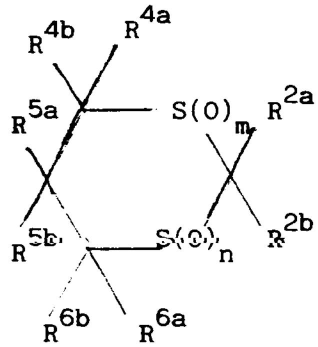

which contains between 9 and 30 carbon atoms, and wherein m and n are independently selected from 0, 1 and 2; $R^{2a}$ is hydrogen, cyano, methoxy, ethoxy, a group $S(O)_qR^7$ where q is 0, 1 or 2 and $R^7$ is methyl or ethyl, or $R^{2a}$ is a group $C{\equiv}CR^8$ where $R^8$ is hydrogen, $C_{1-4}$ alkyl or a silyl group containing three $C_{1-4}$ alkyl groups or two $C_{1-4}$ alkyl groups and a phenyl group, or $R^{2a}$ is vinyl, halo, $C_{1-3}$ alkyl optionally substituted by halo, methoxy, ethoxy or cyano, or $R^{2a}$ is a group $COR^9$ where $R^9$ is hydrogen, $C_{1-4}$ alkoxy, $C_{1-4}$ alkyl or a group $NR^{10}R^{11}$ wherein $R^{10}$ and $R^{11}$ are independently selected from hydrogen, methyl or ethyl; $R^{2b}$ which contains between 2 and 18 carbon atoms is a 5 or 6-membered ring containing an oxygen, sulphur and/or nitrogen atom or a group $R^{12}$ wherein $R^{12}$ is a $C_{1-16}$ hydrocarbyl group, each optionally substituted by an azido, cyano, nitro or two hydroxy groups or by one to five halo atoms which are the same or different or by one to four groups $R^{13}$ which are the same or different and each contains one to four hetero atoms, which are the same or different and are chosen from oxygen, sulphur, nitrogen and silicon, 1 to 16 carbon atoms and optionally 1 to 9 fluoro or chloro atoms or in the case of the 5 or 6 membered ring optionally substituted by a group $R^{12}$; $R^{4a}$ and $R^{4b}$, $R^{6a}$ and $R^{6b}$ are independently selected from hydrogen, $C_{1-3}$ alkyl, $C_{2-3}$ alkenyl or alkynyl each being optionally substituted by halo, cyano or $C_{1-4}$ alkoxy; cyano, halo or a group $COR^{9'}$ wherein $R^{9'}$ is hydrogen, $C_{1-4}$ alkyl, or a group $NR^{10'}$, $R^{11'}$ wherein $R^{10'}$ and $R^{11'}$ are independently selected from hydrogen, methyl or ethyl; $R^{5a}$ is a non-aromatic hydrocarbyl group containing up to seven carbon atoms, or phenyl each optionally substituted by cyano, halo $C_{1-4}$ alkyl, $C_{1-4}$ haloalkyl, $C_{3-4}$ cycloalkyl, $C_{1-4}$ alkoxy or a group $S(O)_{q'}$, $R^{7'}$ wherein $q'$ is 0, 1 or 2 and $R^{7'}$ is methyl or ethyl and $R^{5b}$ is hydrogen or $C_{1-4}$ alkyl optionally substituted by alkoxy; provided that when $R^{2a}$ is hydrogen, methyl, or ethyl; $R^{2b}$ is acetylene or contains between 3 and 18 carbon atoms and is a group $R^{14}$, wherein $R^{14}$ is a $C_{1-13}$ non-aromatic hydrocarbyl group, optionally substituted by a $C_{1-4}$ carbalkoxy or cyano group and/or by one or two hydroxy groups and/or by one to five halo atoms which are the same or different and/or by one to three groups $R^{15}$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{15}$ and/or by a group $-C{\equiv}CH$, $-C{\equiv}C-R^{14}$ or $C{\equiv}C$-halo and/or by one to five halo atoms and/or by one to three $C_{1-4}$ haloalkyl groups wherein $R^{14}$ and $R^{15}$ are as hereinbefore defined; $R^{4a}$ and $R^{6a}$ are the same or different and are chosen from hydrogen, methyl, trifluoromethyl or cyano; $R^{4b}$ and $R^{6b}$ are hydrogen, and $R^{5b}$ is hydrogen or methyl then $R^{5a}$ is not tertiary butyl.

**2.** A compound of the formula (I) according to claim 1 wherein $R^{2b}$ is a phenyl group substituted at the 3-,4- or 5-positions by one to three substituents each selected from halo, $C_{1-4}$ haloalkyl, cyano, azido, a group $(C{\equiv}C)_pR^{16}$ or a group $S(O)_wR^{17}$ wherein p is 1 or 2, w is 0, 1 or 2, $R^{16}$ is hydrogen, bromine, chlorine or iodine and $R^{17}$ is trifluoromethyl, methyl or ethyl and the phenyl group is additionally optionally substituted at the 2- and/or 6-positions by fluoro or chloro.

**3.** A compound of the formula (I) according to either claim 1 or 2 wherein $R^{2b}$ is phenyl substituted at the 3-, 4- or 5- positions by one to three substituents each selected from halo, cyano, $C_{1-4}$ haloalkyl or a group $C{\equiv}C-R^{21}$ where $R^{21}$ is hydrogen, methyl, or ethyl each optionally substituted by hydroxy, methoxy, ethoxy, acetoxy; or $R^{21}$ is $C_{1-4}$ carbalkoxy, or a silyl group substituted by three $C_{1-4}$ alkyl groups and the phenyl group is additionally optionally substituted at the 2-and/or 6- positions by fluoro or chloro.

**4.** A compound of the formula (I) according to either claim 1 or 2 wherein $R^{2b}$ is a group -A(C≡C)Z, wherein A is a methylene or polymethylene chain optionally containing a double bond and/or an oxygen or sulphur atom and Z is hydrogen, $C_{1-5}$ alkyl, $C_{1-3}$ alkoxymethyl or a group $SiR^{18}$ $R^{19}$ $R^{20}$ wherein $R^{18}$ to $R^{20}$ are the same or different and each is a $C_{1-4}$ aliphatic group or $R^{18}$ and $R^{19}$ are $C_{1-4}$ aliphatic groups and $R^{20}$ is a phenyl group.

**5.** A compound of the formula (I) according to either claim 1 or 2 wherein $R^{2b}$ is a group $-BZ^1$, wherein B is a group $-CH_2O-$ or $-CH_2S(O)w-$ wherein w is 0, 1 or 2 or a $C_{2-6}$ aliphatic group optionally substituted by one to three halo atoms and $Z^1$ is silyl substituted by three $C_{1-4}$ alkyl groups or a group

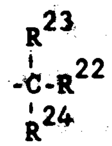

wherein $R^{22}$, $R^{23}$ and $R^{24}$ are the same or different and are each independently selected from halo, $C_{1-4}$ alkyl optionally substituted by halo, $C_{1-4}$ alkoxy or a group $S(O)_q$ $R^{25}$ wherein q is 0, 1 or 2 and $R^{25}$ is $C_{1-4}$ alkyl, or $R^{22}$, $R^{23}$ and $R^{24}$ are selected from $C_{1-4}$ alkoxy or a group $S(O)_wR^{26}$ wherein w is 0, 1 or 2 and $R^{26}$ is $C_{1-4}$ alkyl optionally substituted by fluoro or $R^{22}$ and $R^{23}$ are linked to form a $C_{3-6}$ cycloalkyl ring, or one of $R^{22}$, $R^{23}$ and $R^{24}$ may be hydrogen.

**6.** A compound of the formula (I) according to claim 1 wherein $R^{2b}$ is a group

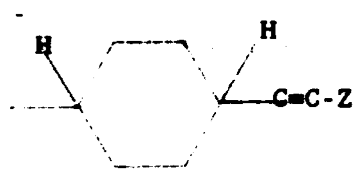

wherein Z is as defined in claim 4.

**7.** A compound of the formula (I) according to any one of claims 1 to 6 wherein $R^{2a}$ is hydrogen, methyl or ethyl.

**8.** A compound of the formula (I) according to claim 1 wherein $R^{2b}$ is phenyl, substituted phenyl or a heterocycle containing 4-6 carbon atoms except that (i) when $R^{5a}$ is straight chain alkyl or tolyl and $R^{2a}$, $R^{4a}$, $R^{4b}$, $R^{6a}$, $R^{6b}$ and $R^{5b}$ are all hydrogen, $R^{2b}$ is not para-cyanophenyl; (ii) when $r^{5a}$ and $R^{5b}$ are both ethyl and $R^{4a}$, $R^{4b}$, $R^{6a}$, $R^{6b}$ and $R^{2a}$ are all hydrogen, $R^{2b}$ is not para-tolyl; (iii) when $R^{5a}$ is n-pentyl and $R^{2a}$, $R^{4a}$, $R^{4b}$, $R^{6a}$, $R^{6b}$ and $R^{5b}$ are all hydrogen, $R^{2b}$ is not para-bromophenyl, (iv) when $R^{5b}$ and $R^{5a}$ are both methyl and $R^{4a}$,$R^{4b}$, $R^{6b}$ and $R^{2a}$ are all hydrogen, $R^{2b}$ is not unsubstituted phenyl and (v) when $R^{2b}$ is n-propyl, $R^{2a}$ is methyl and $R^{4a}$, $R^{4b}$, $R^{6a}$, $R^{6b}$ and $R^{5b}$ are all hydrogen $R^{5a}$ is not ethyl or i-propyl.

**9.** A compound of the formula (I) according to any one of claims 1 to 8 in which $R^{4a}$, $R^{4b}$, $R^{6a}$ and $R^{6b}$ are hydrogen.

**10.** A compound according to claim 1 of formula (Ia):

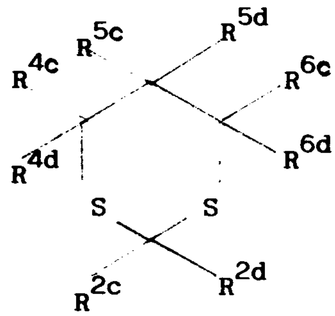

(Ia)

wherein $R^{2c}$ is selected from hydrogen, cyano, methoxy, a group $S(O)_qR^7$ wherein q is 0, 1 or 2 and $R^7$ is methyl or ethyl or $R^{2c}$ is vinyl, halo, $C_{1-3}$ alkyl optionally substituted by halo, methoxy, ethoxy or cyano; $R^{2d}$ is a group $(C{\equiv}C)_r$ $Y(C{\equiv}C)_t$ $Z^2$ wherein r is 0 or 1 and t is 1 or 2 and the sum of r and t is not greater than 2, Y is a single bond, a group

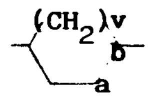

wherein v is 1, 2 or 3 and the $(C{\equiv}C)_tZ^2$ fragment is attached to the a or b position of the ring, or Y is a methylene or polymethylene chain containing between 1 and 8 carbon atoms in which one or two heteroatoms and/or double or triple bonds may be interspersed, the chain being optionally substituted by one to four substituents which may be the same or different and are each independently selected from hydroxy, oxo, halo, $C_{1-4}$ alkyl, $C_{1-4}$ alkoxy, $C_{1-4}$ acyloxy, epoxy, a $C_{1-4}$ alkylidene group, a $C_{1-6}$ carbalkoxy group, $C_{1-4}$ haloalkyl or cyano, $Z^2$ is selected from hydrogen, $C_{1-10}$ hydrocarbyl optionally substituted by halo, $C_{1-4}$ alkoxy, hydroxy, oxo, a group $S(O)qR^7$ wherein q is 0, 1 or 2 and $R^7$ is methyl or ethyl; or $Z^2$ is a group cyano, $C_{1-4}$ acyloxy or carbalkoxy, halo or a group $SiR^{27}$ $R^{28}$ $R^{29}$ wherein $R^{27}$, $R^{28}$ and $R^{29}$ are the same or different and are each a hydrocarbyl group containing up to 4 carbon atoms or $Z^2$ is a group $R^{30}OCO$ wherein $R^{30}$ is $C_{1-4}$ alkyl; $R^{4c}$, $R^{4d}$, $R^{6c}$ and $R^{6d}$ are each selected from hydrogen, methyl, cyano and trifluoromethyl; $R^{5c}$ is alkyl, cycloalkyl, alkenyl, cycloalkenyl or alkynyl each containing up to seven carbon atoms or phenyl each optionally substituted by halo, cyano, $C_{1-4}$ alkyl, $C_{1-4}$ haloalkyl, $C_{3-4}$ cycloalkyl, $C_{1-4}$ alkoxy or a group $S(O)_q$ $R^7$ as hereinbefore defined; and $R^{5d}$ is hydrogen or $C_{1-4}$ alkyl provided that $R^{5c}$ is not tertiary butyl.

**11.** A compound of the formula (Ia) according to claim 10 wherein $R^{2d}$ is a group $-(C{\equiv}C)-Bu^t$.

**12.** A compound selected from:
cis-2(a)-(4-Bromophenyl)-5(e)-butyl-1,3-dithiane
5(e)-t-Butyl-2(e)-(4-methylphenyl)-1,3-dithiane
5(e)-t-Butyl-2(e)-(4-nitrophenyl)-1,3-dithiane
2(e)-(4-Bromophenyl)-5(e)-neopentyl-1,3-dithiane
cis-2(e)-(4-Bromophenyl)-5(e)-t-butyl-2(a)-methylthio-1,3-dithiane
2(e)-(4-Bromophenyl)-5(e)-t-butyl-2(a)-methoxy-1,3-dithiane
trans-2(e)-(4-Bromophenyl)-5(e)-ethyl-1,3-dithiane
2(e)-(4-Bromophenyl)-2(a)-methyl-5(e)-(1,1-dimethylpropyl)-1,3-dithiane
2(e)-(4-Bromophenyl)-5(e)-t-butyl-1,3-dithiane-2(a)-carbonitrile
2(a)-(4-Bromophenyl)-5(e)-ethyl-2(e)-methyl-1,3-dithiane
trans-5(e)-t-Butyl-2(e)-(4-t-butylphenyl)-1,3-dithiane
2-(5(e)-t-Butyl-1,3-dithian-2(e)-yl)propan-2-one
trans-5(e)-t-Butyl-(2e)-(2,2-dibromoethenyl)-1,3-dithiane
trans-2(e)-Bromoethynyl-5(e)-tbutyl-1,3-dithiane
4-(trans-5(e)-t-Butyl-1,3-dithiane-2(e)-yl)-2,2-dimethylbutan-3-one
2(e)-(3,3-Dimethylbutyl)-5(e)-ethyl-1,3-dithiane
2(a)-(4-Bromophenyl)-2(e)-methyl-5(e)-(1,1-dimethylpropyl)-1,3-dithiane

**13.** A pesticidal formulation comprising a compound of the formula (I) as defined according to claim 1 in admixture with one or more carriers or diluents.

**14.** A pesticidal formulation according to claim 13 which additionally contains a synergist or potentiator.

**15.** A pesticidal formulation according to either claim 13 or claim 14 which additionally contains one or more pesticidally active ingredients, attractants, repellents, bacteriocides, fungicides and/or anthelmintics.

**16.** A compound of the formula (I) for use in human or veterinary medicine.

**17.** A method for the control of pesticidal infestations on plants and/or stored products which comprises administering an effective amount of a compound of the formula (I) as defined according to claim 1 to the plant and/or stored product.

**18.** A process for the manufacture of a compound of the formula (I) as defined according to any one of claims 1 to 11 which comprises
(i) the reaction of a compound of the formula (II):

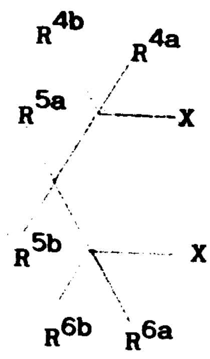

wherein X is SH with a suitable aldehyde or ketone of the formula

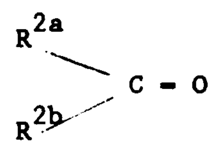

or a reactive derivative thereof or
(ii) for the production of compounds where $R^{2a}$ is hydrogen, the reaction of a dithiaborinane dimethyl sulphide complex of a compound of the formula (II) with a carboxylic acid of the formula $R^{2b}CO_2H$
(iii) for the production of compounds where $R^{2a}$ is alkyl, alkenyl or alkynyl, the reaction of a compound of the formula (III)

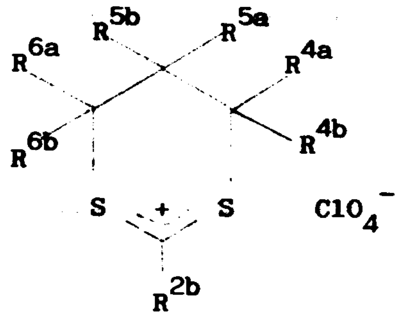

with an alcohol $R^{2a}OH$ or Grignard reagent $R^{2a}MgBr$ wherein $R^{2b}$, $R^{4a}$, $R^{4b}$, $R^{5a}$, $R^{5b}$, $R^{6a}$ and $R^{6b}$ are as defined in any one of claims 1 to 12 or

(iv) for the production or compounds where $R^{2a}$ is cyano, the reaction of the corresponding compound wherein $R^{2a}$ is methylthio with a compound $(Alk)_3SiCN$ wherein Alk is a $C_{1-4}$ alkyl group or

(v) for the production of compounds where $R^{2a}$ is alkylthio, the reaction of the corresponding compound wherein $R^{2a}$ is hydrogen with a dialkyldisulphide in the prescence of an alkyllithium compound or

(vi) for the production of compounds wherein $R^{2b}$ is pyridyl, the reaction of the corresponding compound wherein $R^{2b}$ is hydrogen with pyridine and after any one of step (i) to step (vi) optionally oxidising one or both of the ring sulphur atoms or

(v) the conversion of a compound of the formula (I) into another compound of the formula (I).

**Claims for the following Contracting States : ES, GR**

**1.** A process for the preparation of a compound of the formula (I):

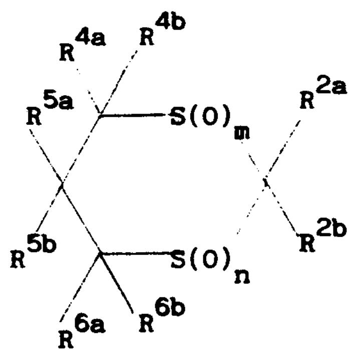

(I)

which contains between 9 and 30 carbon atoms, and wherein m and n are independently selected from 0, 1 and 2; $R^{2a}$ is hydrogen, cyano, methoxy, ethoxy, a group $S(O)_qR^7$ where q is 0, 1 or 2 and $R^7$ is methyl or ethyl, or $R^{2a}$ is a group $C{\equiv}CR^8$ where $R^8$ is hydrogen, $C_{1-4}$ alkyl or a silyl group containing three $C_{1-4}$ alkyl groups or two $C_{1-4}$ alkyl groups and a phenyl group, or $R^{2a}$ is vinyl, halo, $C_{1-3}$ alkyl optionally substituted by halo, methoxy, ethoxy or cyano, or $R^{2a}$ is a group $COR^9$ where $R^9$ is hydrogen, $C_{1-4}$ alkoxy, $C_{1-4}$ alkyl or a group $NR^{10}R^{11}$ wherein $R^{10}$ and $R^{11}$ are independently selected from hydrogen, methyl or ethyl; $R^{2b}$ which contains between 2 and 18 carbon atoms is a 5 or 6-membered ring containing an oxygen, sulphur and/or nitrogen atom or a group $R^{12}$ wherein $R^{12}$ is a $C_{1-16}$ hydrocarbyl group, each optionally substituted by an azido, cyano, nitro or two hydroxy groups or by one to five halo atoms which are the same or different or by one to four groups $R^{13}$ which are the same or different and each contains one to four hetero atoms, which are the same or different and are chosen from oxygen, sulphur, nitrogen and silicon, 1 to 16 carbon atoms and optionally 1 to 9 fluoro or chloro atoms or in the case of the 5 or 6 membered ring optionally substituted by a group $R^{12}$; $R^{4a}$ and $R^{4b}$, $R^{6a}$ and $R^{6b}$ are independently selected from hydrogen, $C_{1-3}$ alkyl, $C_{2-3}$ alkenyl or alkynyl each being optionally substituted by halo, cyano or $C_{1-4}$ alkoxy; cyano, halo or a group $COR^{9'}$ wherein $R^{9'}$

is hydrogen, $C_{1-4}$ alkoxy, $C_{1-4}$ alkyl, or a group $NR^{10'}$, $R^{11'}$ wherein $R^{10'}$ and $R^{11'}$ are independently selected from hydrogen, methyl or ethyl; $R^{5a}$ is a non-aromatic hydrocarbyl group containing up to seven carbon atoms, or phenyl each optionally substituted by cyano, halo $C_{1-4}$ alkyl, $C_{1-4}$ haloalkyl, $C_{3-4}$ cycloalkyl, $C_{1-4}$ alkoxy or a group $S(O)_{q}$, $R^{7'}$ wherein $q'$ is 0, 1 or 2 and $R^{7'}$ is methyl or ethyl and $R^{5b}$ is hydrogen or $C_{1-4}$ alkyl optionally substituted by alkoxy provided that when $R^{2a}$ is hydrogen, methyl, or ethyl; $R^{2b}$ is acetylene or contains between 3 and 18 carbon atoms and is a group $R^{14}$, wherein $R^{14}$ is a $C_{1-13}$ non-aromatic hydrocarbyl group, optionally substituted by a $C_{1-4}$ carbalkoxy or cyano group and/or by one or two hydroxy groups and/or by one to five halo atoms which are the same or different and/or by one to three groups $R^{15}$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{15}$ and/or by a group -C≡CH, -C≡C-$R^{14}$ or C≡C-halo and/or by one to five halo atoms and/or by one to three $C_{1-4}$ haloalkyl groups wherein $R^{14}$ and $R^{15}$ are as hereinbefore defined; $R^{4a}$ and $R^{6a}$ are the same or different and are chosen from hydrogen, methyl, trifluoromethyl or cyano; $R^{4b}$ and $R^{6b}$ are hydrogen, and $R^{5b}$ is hydrogen or methyl then $R^{5a}$ is not tertiary butyl which process comprises:

(i) the reaction of a compound of the formula (II):

(II)

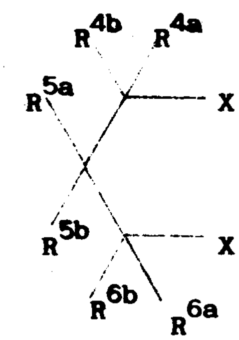

wherein X is SH with a suitable aldehyde or ketone of the formula

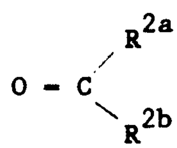

or a reactive derivative thereof or

(ii) when $R^{2a}$ is hydrogen the reaction of a dithiaborinanedimethylsulphide complex of a compound of the formula (II) with a carboxylic acid of the formula:

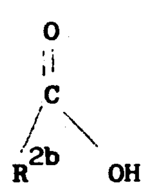

iii) for the production of compounds where $R^{2a}$ is alkyl, alkenyl or alkynyl,the reaction of a compound of the formula (III)

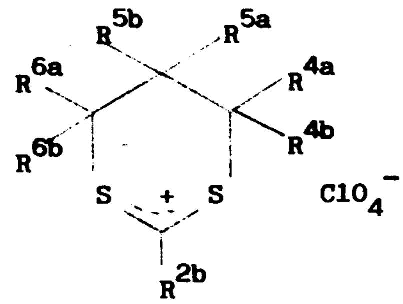

with an alcohol $R^{2a}OH$ or Grignard reagent $R^{2a}MgBr$ wherein $R^{2b}$,$R^{4a}$, $R^{4b}$, $R^{5a}$, $R^{5b}$, $R^{6a}$ and $R^{6b}$ are as hereinbefore defined.

(iv) for the production of compounds where $R^{2a}$ is cyano, the reaction of the corresponding compound wherein $R^{2a}$ is methylthio with a compound $(Alk)_3SiCN$ wherein Alk is a $C_{1-4}$ alkyl group or

(v) for the production of compounds where $R^{2a}$ is alkylthio, the reaction of the corresponding compound wherein $R^{2a}$ is hydrogen with a dialkyldisulphide in the presence of an alkyllithium compound or

(vi) for the production of compounds wherein $R^{2b}$ is pyridyl, by the reaction of the corresponding compound wherein $R^{2b}$ is hydrogen with pyridine and after any one of steps (i) to (vi) optionally oxidising one or both of the ring sulphur atoms

(vii) the conversion of a compound of the formula (I) into another compound of the formula (I).

**2.** A process according to claim 1 for the preparation of a compound of the formula (I) wherein $R^{2b}$ is a phenyl group substituted at the 3-,4-or 5-positions by one to three substitutents each selected from halo, $C_{1-4}$ haloalkyl, cyano, azido, a group $(C{\equiv}C)_pR^{16}$ or a group $S(O)_wR^{17}$ wherein p is 1 or 2, w is 0, 1 or 2, $R^{16}$ is hydrogen, bromine, chlorine or iodine and $R^{17}$ is trifluoromethyl, methyl or ethyl and the phenyl group is additionally optionally substituted at the 2- and/or 6-positions by fluoro or chloro.

**3.** A process according to either claim 1 or 2 for the preparation of a compound of the formula (I) wherein $R^{2b}$ is phenyl substituted at the 3-, 4- or 5- positions by one to three substituents each selected from halo, cyano, $C_{1-4}$ haloalkyl or a group $C{\equiv}C\text{-}R^{21}$ where $R^{21}$ is hydrogen, methyl, or ethyl each optionally substituted by hydroxy, methoxy, ethoxy, acetoxy; or $R^{21}$ is $C_{1-4}$ carbalkoxy, or a silyl group substituted by three $C_{1-4}$ alkyl groups and the phenyl group is additionally optionally substituted at the 2- and/or 6- positions by fluoro or chloro.

**4.** A process according to either claim 1 or 2 for the preparation of a compound of the formula (I) wherein $R^{2b}$ is a group $-A(C{\equiv}C)Z$, wherein A is a polymethylene chain optionally containing a double bond and/or an oxygen or sulphur atom and Z is hydrogen, $C_{1-5}$ alkyl, $C_{1-3}$ alkoxymethyl or a group $SiR^{18}R^{19}R^{20}$ wherein $R^{18}$ to $R^{20}$ are the same or different and each is a $C_{1-4}$ aliphatic group or $R^{18}$ and $R^{19}$ are $C_{1-4}$ aliphatic groups and $R^{20}$ is a phenyl group.

**5.** A process according to either claim 1 or 2 for the preparation of a compound of the formula (I) wherein $R^{2b}$ is a group $-BZ^1$, wherein B is a group $-CH_2O-$ or $-CH_2S(O)w-$ wherein w is 0, 1 or 2 or a $C_{2-6}$ aliphatic group optionally substituted by one to three halo atoms and $Z^1$ is silyl substituted by three $C_{1-4}$ alkyl groups or a group

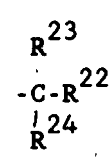

wherein $R^{22}$, $R^{23}$ and $R^{24}$ are the same or different and are each independently selected from halo, $C_{1-4}$ alkyl optionally substituted by halo, $C_{1-4}$ alkoxy or a group $S(O)_q R^{25}$ wherein q is 0, 1 or 2 and $R^{25}$ is $C_{1-4}$ alkyl, or $R^{22}$, $R^{23}$ and $R^{24}$ are selected from $C_{1-4}$ alkoxy or a group $S(O)_wR^{26}$ wherein w is

0, 1 or 2 and $R^{26}$ is $C_{1-4}$ alkyl optionally substituted by fluoro or $R^{22}$ and $R^{23}$ are linked to form a $C_{3-6}$ cycloalkyl ring, or one of $R^{22}$, $R^{23}$ and $R^{24}$ may be hydrogen.

6. A process according to claim 1 for the preparation of a compound of the formula (I) wherein $R^{2b}$ is a group

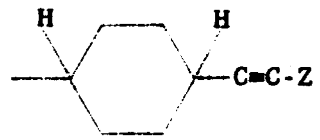

wherein Z is as defined in claim 4.

7. A process according to claim 1 for the preparation of a compound of the formula (I) wherein $R^{2b}$ is hydrogen, methyl or ethyl.

8. A process according to claim 1 for the preparation of a compound of the formula (I) wherein $R^{2b}$ is phenyl, substituted phenyl or a heterocycle containing 4 to 6 carbon atoms except that (i) when $R^{5a}$ is straight chain alkyl or tolyl and $R^{2a}$, $R^{4a}$, $R^{4b}$, $R^{6a}$, $R^{6b}$ and $R^{5b}$ are all hydrogen, $R^{2b}$ is not para-cyanophenyl; (ii) when $R^{5a}$ and $R^{5b}$ are both ethyl and $R^{4a}$, $R^{4b}$, $R^{6a}$, $R^{6b}$ and $R^{2a}$ are all hydrogen, $R^{2b}$ is not para-tolyl; (iii) when $R^{5a}$ is n-pentyl and $R^{2a}$, $R^{4a}$, $R^{4b}$, $R^{6a}$, and $R^{6b}$ and $R^{5b}$ are all hydrogen, $R^{2b}$ is not para-bromophenyl, (iv) when $R^{5b}$ and $R^{5a}$ are both methyl and $R^{4a}$, $R^{4b}$, $R^{6b}$ and $R^{2a}$ are all hydrogen, $R^{2b}$ is not substituted phenyl and (v) when $R^{2b}$ is n-propyl, $R^{2a}$ is methyl and $R^{4a}$, $R^{4b}$, $R^{6a}$, $R^{6b}$ and $R^{5b}$ are all hydrogen $R^{5a}$ is not ethyl or i-propyl.

9. A process according to claim 1 for the preparation of a compound of the formula (I) wherein $R^{4a}$, $R^{4b}$, $R^{6a}$ and $R^{6b}$ are hydrogen.

10. A process according to claim 1 for the preparation of a compound of formula (Ia):

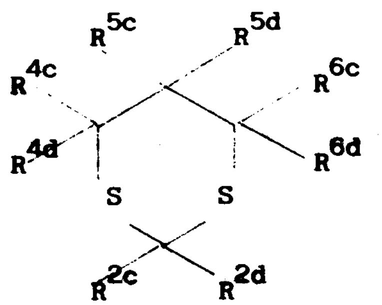

(Ia)

wherein $R^{2c}$ is selected from hydrogen, cyano, methoxy, a group $S(O)_qR^7$ wherein q is 0, 1 or 2 and $R^7$ is methyl or ethyl or $R^{2c}$ is vinyl, halo, $C_{1-3}$ alkyl optionally substituted by halo, methoxy, ethoxy or cyano; $R^{2d}$ is a group $(C{\equiv}C)_r$ $Y(C{\equiv}C)_t$ $Z^2$ wherein r is 0 or 1 and t is 1 or 2 and the sum of r and t is not greater than 2, Y is a single bond, a group

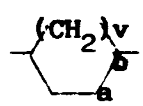

wherein v is 1, 2 or 3 and the $(C{\equiv}C)_tZ^2$ fragment is attached to the a or b position of the ring, or Y is a methylene or polymethylene chain containing between 1 and 8 carbon atoms in which one or two hetero-atoms and/or double or triple bonds may be interspersed, and the chain being optionally

substituted by one to four substitutents which may be the same or different and are each independently selected from hydroxy, oxo, halo, $C_{1-4}$ alkyl, $C_{1-4}$ alkoxy, $C_{1-4}$ acyloxy, epoxy, a $C_{1-4}$ alkylidene group, a $C_{1-6}$ carbalkoxy group, $C_{1-4}$ haloalkyl or cyano, $Z^2$ is selected from hydrogen, $C_{1-10}$ hydrocarbyl optionally substituted by halo, $C_{1-4}$ alkoxy, hydroxy, oxo, a group $S(O)_qR^7$ where q is 0, 1 or 2 and $R^7$ is methyl or ethyl or $Z^2$ is a group cyano, $C_{1-4}$ acyloxy or carbalkoxy, halo or a group $SiR^{27}$ $R^{28}$ $R^{29}$ wherein $R^{27}$, $R^{28}$ and $R^{29}$ are the same or different and are each a hydrocarbyl group containing up to 4 carbon atoms or $Z^2$ is a group $R^{30}OCO$ wherein $R^{30}$ is $C_{1-4}$ alkyl; $R^{4c}$, $R^{4d}$, $R^{6c}$ and $R^{6d}$ are each selected from hydrogen, methyl, cyano and trifluoromethyl; $R^{5c}$ is alkyl, cycloalkyl, alkenyl, cycloalkenyl or alkynyl each containing up to seven carbon atoms or phenyl each optionally substituted by halo, cyano, $C_{1-4}$ alkyl, $C_{1-4}$haloalkyl, $C_{3-4}$ cycloalkyl, $C_{1-4}$ alkoxy or a group $S(O)_q$ $R^7$ as hereinbefore defined; and $R^{5d}$ is hydrogen or $C_{1-4}$ alkyl provided that $R^{5c}$ is not tertiary butyl.

**11.** A process according to claim 1 for the preparation of a compound of the formula (Ia) according to claim 10 wherein $R^{2d}$ is a group -(C≡C)-Bu$^t$.

**12.** A process according to claim 1 for the preparation of:
cis-2(a)-(4-Bromophenyl)-5(e)-butyl-1,3-dithiane
5(e)-t-Butyl-2(e)-(4-methylphenyl)-1,3-dithiane
5(e)-t-Butyl-2(e)-(4-nitrophenyl)-1,3-dithiane
2(e)-(4-Bromophenyl)-5(e)-neopentyl-1,3-dithiane
cis-2(e)-(4-Bromophenyl)-5(e)-t-butyl-2(a)-methylthio-1,3-dithiane
2(e)-(4-Bromophenyl)-5(e)-t-butyl-2(a)-methoxy-1,3-dithiane
trans-2(e)-(4-Bromophenyl)-5(e)-ethyl-1,3-dithiane
2(e)-(4-Bromophenyl)-2(a)-methyl-5(e)-(1,1-dimethylpropyl)-1,3-dithiane
2(e)-(4-Bromophenyl)-5(e)-t-butyl-1,3-dithiane-2(a)-carbonitrile
2(a)-(4-Bromophenyl)-5(e)-ethyl-2(e)-methyl-1,3-dithiane
trans-5(e)-t-Butyl-2(e)-(4-t-butylphenyl)-1,3-dithiane
2-(5(e)-t-Butyl-1,3-dithian-2(e)-yl)propan-2-one
trans-5(e)-t-Butyl-(2e)-(2,2-dibromoethenyl)-1,3-dithiane
trans-2(e)-Bromoethynyl-5(e)-tbutyl-1,3-dithiane
4-(trans-5(e)-t-Butyl-1,3-dithiane-2(e)-yl)-2,2-dimethylbutan-3-one
2(e)-(3,3-Dimethylbutyl)-5(e)-ethyl-1,3-dithiane
2(a)-(4-Bromophenyl)-2(e)-methyl-5(e)-(1,1-dimethylpropyl)-1,3-dithiane

**13.** A pesticidal formulation comprising a compound of the formula (I) as defined according to any one of the preceding claims in admixture with one or more carriers or diluents.

**14.** A pesticidal formulation according to claim 13 which additionally contains a synergist or potentiator.

**15.** A pesticidal formulation according to either claim 13 or claim 14 which additionally contains one or more pesticidally active ingredients, attractants, repellents, bacteriocides, fungicides and/or anthelmintics.

**16.** A compound of the formula (I) for use in human or veterinary medicine.

**17.** A method for the control of arthropod or helminth pests which comprises administering to the arthropod or helminth or their environment an effective amount of a compound of the formula (I) as prepared according to any one of claims 1 to 12.

**18.** A method for the control of pesticidal infestations on animals and/or stored products and/or an environment which comprises administering an effective amount of a compound of the formula (I) as defined according to any one of the preceding claims to the animal and/or stored product and/or an environment susceptible to pest infestation.

**19.** A method for the control of pesticidal infestations on plants and/or stored products and/or an environment which comprises administering an effective amount of a compound of the formula (I) as defined according to any one of the preceding claims to the plant and/or stored product and/or an environment susceptible to pest infestation.

**Patentansprüche**

**Patentansprüche für folgende Vertragsstaaten : AT, BE, CH, DE, FR, GB, IT, LI, LU, NL, SE**

1. Verbindung der Formel (I):

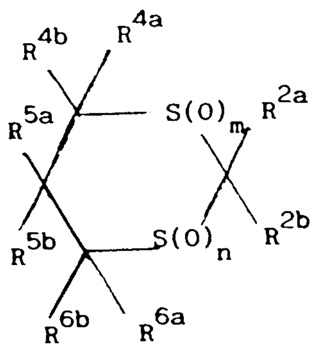

(I),

die 9 bis 30 Kohlenstoffatome enthält und worin m und n unabhängig aus Null, 1 und 2 ausgewählt sind; $R^{2a}$ Wasserstoff, Cyano, Methoxy, Ethoxy oder eine Gruppe $S(O)_qR^7$ bedeutet, wobei q Null, 1 oder 2 ist und $R^7$ Methyl oder Ethyl darstellt, oder $R^{2a}$ eine Gruppe $C{\equiv}CR^8$ ist, wobei $R^8$ Wasserstoff, $C_1$-$C_4$-Alkyl oder eine Silylgruppe enthaltend 3 $C_1$-$C_4$-Alkylgruppen oder 2 $C_1$-$C_4$-Alkylgruppen und eine Phenylgruppe darstellt, oder $R^{2a}$ Vinyl, Halogen, $C_1$-$C_3$-Alkyl gegebenenfalls substituiert durch Halogen, Methoxy, Ethoxy oder Cyano bedeutet oder $R^{2a}$ eine Gruppe $COR^9$ ist, worin $R^9$ Wasserstoff, $C_1$-$C_4$-Alkoxy, $C_1$-$C_4$-Alkyl oder eine Gruppe $NR^{10}R^{11}$ darstellt, wobei $R^{10}$ und $R^{11}$ unabhängig aus Wasserstoff, Methyl oder Ethyl ausgewählt sind; $R^{2b}$, das 2 bis 18 Kohlenstoffatome enthält, einen 5- oder 6-gliedrigen Ring enthaltend ein Sauerstoff-, Schwefel- und/oder Stickstoffatom oder eine Gruppe $R^{12}$ bedeutet, wobei $R^{12}$ eine $C_1$-$C_{16}$-Kohlenwasserstoffgruppe ist, jeweils gegebenenfalls substituiert durch eine Azido-, Cyano-, Nitro- oder 2 Hydroxygruppen oder durch 1 bis 5 Halogenatome, die gleich oder verschieden sind, oder durch 1 bis 4 Gruppen $R^{13}$, die gleich oder verschieden sind und jeweils 1 bis 4 Heteroatome, die gleich oder verschieden sind und aus Sauerstoff, Schwefel, Stickstoff und Silizium ausgewählt sind, 1 bis 16 Kohlenstoffatome und gegebenenfalls 1 bis 9 Fluor- oder Chloratome enthalten oder im Fall des 5- oder 6-gliedrigen Ringes gegebenenfalls durch eine Gruppe $R^{12}$ substituiert sind; $R^{4a}$, $R^{4b}$, $R^{6a}$ und $R^{6b}$ unabhängig aus Wasserstoff, $C_1$-$C_3$-Alkyl, $C_2$-$C_3$-Alkenyl oder Alkinyl jeweils gegebenenfalls substituiert durch Halogen, Cyano oder $C_1$-$C_4$-Alkoxy, Cyano, Halogen oder einer Gruppe $COR^{9'}$ ausgewählt sind, wobei $R^{9'}$ Wasserstoff, $C_1$-$C_4$-Alkyl oder eine Gruppe $NR^{10'}R^{11'}$ bedeutet, worin $R^{10'}$ und $R^{11'}$ unabhängig aus Wasserstoff, Methyl oder Ethyl ausgewählt sind; $R^{5a}$ eine nicht-aromatische Kohlenwasserstoffgruppe mit bis zu 7 Kohlenstoffatomen oder Phenyl jeweils gegebenenfalls substituiert durch Cyano, Halogen, $C_1$-$C_4$-Alkyl, $C_1$-$C_4$-Halogenalkyl, $C_3$-$C_4$-Cycloalkyl, $C_1$-$C_4$-Alkoxy oder eine Gruppe $S(O)_{q'}R^{7'}$ darstellt, wobei q' Null, 1 oder 2 ist und $R^{7'}$ Methyl oder Ethyl bedeutet, und $R^{5b}$ Wasserstoff oder $C_1$-$C_4$-Alkyl gegebenenfalls substituiert durch Alkoxy darstellt; mit der Maßgabe, daß, wenn $R^{2a}$ Wasserstoff, Methyl oder Ethyl bedeutet, $R^{2b}$ Acetylen ist oder 3 bis 18 Kohlenstoffatome enthält und eine Gruppe $R^{14}$ darstellt, wobei $R^{14}$ eine nicht-aromatische $C_1$-$C_{13}$-Kohlenwasserstoffgruppe gegebenenfalls substituiert durch eine $C_1$-$C_4$-Carbalkoxy- oder Cyanogruppe und/oder durch 1 oder 2 Hydroxygruppen und/oder durch 1 bis 5 Halogenatome, die gleich oder verschieden sind, und/oder durch 1 bis 3 Gruppen $R^{15}$, die gleich oder verschieden sind und jeweils 1 bis 4 Heteroatome, die gleich oder verschieden sind und aus Sauerstoff, Schwefel, Stickstoff und Silizium ausgewählt sind, 1 bis 10 Kohlenstoffatome und gegebenenfalls 1 bis 6 Fluor- oder Chloratome enthalten, bedeutet, oder $R^{2b}$ einen 6-gliedrigen aromatischen Ring substituiert durch Cyano und/oder 1 bis 3 Gruppen $R^{15}$ und/oder eine Gruppe $-C{\equiv}CH$, $C{\equiv}C-R^{14}$ oder $C{\equiv}C$-Halogen und/oder 1 bis 5 Halogenatome und/oder 1 bis 3 $C_1$-$C_4$-Halogenalkylgruppen bedeutet, wobei $R^{14}$ und $R^{15}$ wie vorstehend definiert sind; $R^{4a}$ und $R^{6a}$ gleich oder verschieden sind und aus Wasserstoff, Methyl, Trifluormethyl oder Cyano ausgewählt sind; $R^{4b}$ und $R^{6b}$ Wasserstoff bedeuten und $R^{5b}$ Wasserstoff oder Methyl darstellt, $R^{5a}$ nicht tert.Butyl ist.

2. Verbindung der Formel (I) nach Anspruch 1, worin $R^{2b}$ eine Phenylgruppe substituiert in Stellung 3, 4 oder 5 durch 1 bis 3 Substituenten jeweils ausgewählt aus Halogen, $C_1$-$C_4$-Halogenalkyl, Cyano, Azido,

eine Gruppe $(C{\equiv}C)_pR^{16}$ oder eine Gruppe $S(O)_wR^{17}$ bedeutet, wobei p 1 oder 2 ist, w Null, 1 oder 2 ist, $R^{16}$ Wasserstoff, Brom, Chlor oder Iod darstellt und $R^{17}$ Trifluormethyl, Methyl oder Ethyl bedeutet, und die Phenylgruppe weiters gegebenenfalls durch Fluor oder Chlor in Stellung 2 und/oder 6 substituiert ist.

**3.** Verbindung der Formel (I) nach Anspruch 1 oder 2, worin $R^{2b}$ Phenyl substituiert in Stellung 3, 4 oder 5 durch 1 bis 3 Substituenten jeweils ausgewählt aus Halogen, Cyano, $C_1$-$C_4$-Halogenalkyl oder eine Gruppe $C{\equiv}C$-$R^{21}$ bedeutet, wobei $R^{21}$ Wasserstoff, Methyl oder Ethyl jeweils gegebenenfalls substituiert durch Hydroxy, Methoxy, Ethoxy, Acetoxy darstellt oder $R^{21}$ $C_1$-$C_4$-Carbalkoxy oder eine Silylgruppe substituiert durch 3 $C_1$-$C_4$-Alkylgruppen bedeutet, und die Phenylgruppe weiters gegebenenfalls durch Fluor oder Chlor in Stellung 2 und/oder 6 substituiert ist.

**4.** Verbindung der Formel (I) nach Anspruch 1 oder 2, worin $R^{2b}$ eine Gruppe $-A(C{\equiv}C)Z$ bedeutet, wobei A eine Methylen- oder Polymethylenkette gegebenenfalls enthaltend eine Doppelbindung und/oder ein Sauerstoff- oder Schwefelatom ist und Z Wasserstoff, $C_1$-$C_5$-Alkyl, $C_1$-$C_3$-Alkoxymethyl oder eine Gruppe $SiR^{18}R^{19}R^{20}$ darstellt, wobei $R^{18}$ bis $R^{20}$ gleich oder verschieden sind und jeweils eine aliphatische $C_1$-$C_4$-Gruppe bedeuten oder $R^{18}$ und $R^{19}$ aliphatische $C_1$-$C_4$-Gruppen darstellen und $R^{20}$ eine Phenylgruppe ist.

**5.** Verbindung der Formel (I) nach Anspruch 1 oder 2, worin $R^{2b}$ eine Gruppe $-BZ^1$ ist, wobei B eine Gruppe $-CH_2O-$ oder $-CH_2S(O)_w-$, wobei w Null, 1 oder 2 ist, oder eine aliphatische $C_2$-$C_6$-Gruppe gegebenenfalls substituiert durch 1 bis 3 Halogenatome bedeutet und $Z^1$ Silyl substituiert durch 3 $C_1$-$C_4$-Alkylgruppen oder eine Gruppe

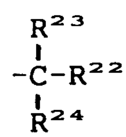

darstellt, worin $R^{22}$, $R^{23}$ und $R^{24}$ gleich oder verschieden sind und jeweils unabhängig aus Halogen, $C_1$-$C_4$-Alkyl gegebenenfalls substituiert durch Halogen, $C_1$-$C_4$-Alkoxy oder einer Gruppe $S(O)_qR^{25}$ ausgewählt sind, wobei q Null, 1 oder 2 ist und $R^{25}$ $C_1$-$C_4$-Alkyl bedeutet, oder $R^{22}$, $R^{23}$ und $R^{24}$ aus $C_1$-$C_4$-Alkoxy oder einer Gruppe $S(O)_wR^{26}$ ausgewählt sind, wobei w Null, 1 oder 2 ist und $R^{26}$ $C_1$-$C_4$-Alkyl gegebenenfalls substituiert durch Fluor bedeutet, oder $R^{22}$ und $R^{23}$ unter Bildung eines $C_3$-$C_6$-Cycloalkylringes verbunden sind oder eines von $R^{22}$, $R^{23}$ und $R^{24}$ Wasserstoff darstellen kann.

**6.** Verbindung der Formel (I) nach Anspruch 1, worin $R^{2b}$ eine Gruppe

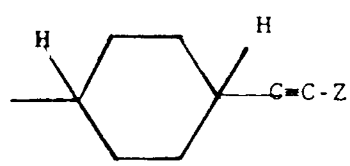

bedeutet, worin Z wie in Anspruch 4 definiert ist.

**7.** Verbindung der Formel (I) nach einem der Ansprüche 1 bis 6, worin $R^{2a}$ Wasserstoff, Methyl oder Ethyl bedeutet.

**8.** Verbindung der Formel (I) nach Anspruch 1, worin $R^{2b}$ Phenyl, substituiertes Phenyl oder einen Heterocyclus mit 4 bis 6 Kohlenstoffatomen bedeutet, ausgenommen daß, (1) wenn $R^{5a}$ geradkettiges Alkyl oder Tolyl ist und $R^{2a}$, $R^{4a}$, $R^{4b}$, $R^{6a}$, $R^{6b}$ und $R^{5b}$ alle Wasserstoff darstellen, $R^{2b}$ nicht p-Cyanophenyl bedeutet; (ii) wenn $R^{5a}$ und $R^{5b}$ beide Ethyl sind und $R^{4a}$, $R^{4b}$, $R^{6a}$, $R^{6b}$ und $R^{2a}$ alle Wasserstoff darstellen, $R^{2b}$ nicht p-Tolyl bedeutet; (iii) wenn $R^{5a}$ n-Pentyl ist und $R^{2a}$, $R^{4a}$, $R^{4b}$, $R^{6a}$, $R^{6b}$ und $R^{5b}$ alle Wasserstoff darstellen, $R^{2b}$ nicht p-Bromphenyl bedeutet, (iv) wenn $R^{5b}$ und $R^{5a}$ beide Methyl sind und $R^{4a}$, $R^{4b}$, $R^{6b}$ und $R^{2a}$ alle Wasserstoff darstellen, $R^{2b}$ nicht unsubstituiertes Phenyl

bedeutet, und (v) wenn $R^{2b}$ n-Propyl ist, $R^{2a}$ Methyl bedeutet und $R^{4a}$, $R^{4b}$, $R^{6a}$, $R^{6b}$ und $R^{5b}$ alle Wasserstoff darstellen, $R^{5a}$ nicht Ethyl oder Isopropyl bedeutet.

9. Verbindung der Formel (I) nach einem der Ansprüche 1 bis 8, worin $R^{4a}$, $R^{4b}$, $R^{6a}$ und $R^{6b}$ Wasserstoff bedeuten.

10. Verbindung nach Anspruch 1 der Formel (Ia):

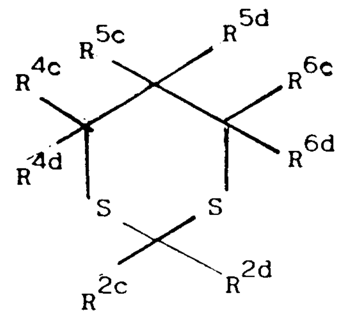

(Ia),

worin $R^{2c}$ aus Wasserstoff, Cyano, Methoxy oder einer Gruppe $S(O)_qR^7$ ausgewählt ist, wobei q Null, 1 oder 2 ist und $R^7$ Methyl oder Ethyl bedeutet, oder $R^{2c}$ Vinyl, Halogen, $C_1$-$C_3$-Alkyl gegebenenfalls substituiert durch Halogen, Methoxy, Ethoxy oder Cyano darstellt; $R^{2d}$ eine Gruppe $(C{\equiv}C)_rY(C{\equiv}C)_tZ^2$ bedeutet, wobei r Null oder 1 ist und t 1 oder 2 ist und die Summe von r und t nicht größer als 2 ist, Y eine Einfachbindung, eine Gruppe

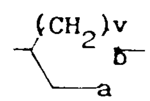

worin v 1, 2 oder 3 ist und das $(C{\equiv}C)_tZ^2$-Fragment an die Stellung a oder b des Ringes gebunden ist, darstellt oder Y eine Methylen- oder Polymethylenkette mit 1 bis 8 Kohlenstoffatomen bedeutet, in die 1 oder 2 Heteroatome und/oder Doppel- oder Dreifachbindungen eingefügt sein können, wobei die Kette gegebenenfalls durch 1 bis 4 Substituenten substituiert ist, die gleich oder verschieden sein können und jeweils unabhängig aus Hydroxy, Oxo, Halogen, $C_1$-$C_4$-Alkyl, $C_1$-$C_4$-Alkoxy, $C_1$-$C_4$-Acyloxy, Epoxy, einer $C_1$-$C_4$-Alkylidengruppe, einer $C_1$-$C_4$-Carbalkoxygruppe, $C_1$-$C_4$-Halogenalkyl oder Cyano ausgewählt sind, $Z^2$ aus Wasserstoff, $C_1$-$C_{10}$-Kohlenwasserstoff gegebenenfalls substituiert durch Halogen, $C_1$-$C_4$-Alkoxy, Hydroxy, Oxo, einer Gruppe $S(O)_qR^7$, wobei q Null, 1 oder 2 ist und $R^7$ Methyl oder Ethyl bedeutet, ausgewählt ist, oder $Z^2$ eine Cyanogruppe, $C_1$-$C_4$-Acyloxy oder Carbalkoxy, Halogen oder eine Gruppe $SiR^{27}R^{28}R^{29}$ darstellt, wobei $R^{27}$, $R^{28}$ und $R^{29}$ gleich oder verschieden sind und jeweils eine Kohlenwasserstoffgruppe mit bis zu 4 Kohlenstoffatomen bedeuten, oder $Z^2$ eine Gruppe $R^{30}OCO$ ist, worin $R^{30}$ $C_1$-$C_4$-Alkyl darstellt; $R^{4c}$, $R^{4d}$, $R^{6c}$ und $R^{6d}$ jeweils aus Wasserstoff, Methyl, Cyano und Trifluormethyl ausgewählt sind; $R^{5c}$ Alkyl, Cycloalkyl, Alkenyl, Cycloalkenyl oder Alkinyl jeweils enthaltend bis zu 7 Kohlenstoffatome oder Phenyl jeweils gegebenenfalls substituiert durch Halogen, Cyano, $C_1$-$C_4$-Alkyl, $C_1$-$C_4$-Halogenalkyl, $C_3$-$C_4$-Cycloalkyl, $C_1$-$C_4$-Alkoxy oder eine Gruppe $S(O)_qR^7$, wie vorstehend definiert, bedeutet; und $R^{5d}$ Wasserstoff oder $C_1$-$C_4$-Alkyl darstellt, mit der Maßgabe, daß $R^{5c}$ nicht tert.Butyl ist.

11. Verbindung der Formel (Ia) nach Anspruch 10, worin $R^{2d}$ eine Gruppe $-(C{\equiv}C)-Bu^t$ bedeutet.

12. Verbindung ausgewählt aus:
cis-2(a)-(4-Bromphenyl)-5(e)-butyl-1,3-dithian,
5(e)-tert.Butyl-2(e)-(4-methylphenyl)-1,3-dithian,
5(e)-tert.Butyl-2(e)-(4-nitrophenyl)-1,3-dithian,
2(e)-(4-Bromphenyl)-5(e)-neopentyl-1,3-dithian,
cis-2(e)-(4-Bromphenyl)-5(e)-tert.butyl-2(a)-methylthio-1,3-dithian,

2(e)-(4-Bromphenyl)-5(e)-tert.butyl-2(a)-methoxy-1,3-dithian,
trans-2(e)-(4-Bromphenyl)-5(e)-ethyl-1,3-dithian,
2(e)-(4-Bromphenyl)-2(a)-methyl-5(e)-(1,1-dimethylpropyl)-1,3-dithian,
2(e)-(4-Bromphenyl)-5(e)-tert.butyl-1,3-dithian-2(a)-carbonitril,
2(a)-(4-Bromphenyl)-5(e)-ethyl-2(e)-methyl-1,3-dithian,
trans-5(e)-tert.Butyl-2(e)-(4-tert.butylphenyl)-1,3-dithian,
2-(5(e)-tert.Butyl-1,3-dithian-2(e)-yl)-propan-2-on,
trans-5(e)-tert.Butyl-2(e)-(2,2-dibromethenyl)-1,3-dithian,
trans-2(e)-Bromethinyl-5(e)-tert.butyl-1,3-dithian,
4-(trans-5(e)-tert.Butyl-1,3-dithian-2(e)-yl)-2,2-dimethylbutan-3-on,
2(e)-(3,3-Dimethylbutyl)-5(e)-ethyl-1,3-dithian,
2(a)-(4-Bromphenyl)-2(e)-methyl-5(e)-(1,1-dimethylpropyl)-1,3-dithian.

**13.** Schädlingsbekämpfungsmittel umfassend eine Verbindung der Formel (I), wie in Anspruch 1 definiert, gemischt mit einem oder mehreren Trägern oder Verdünnungsmitteln.

**14.** Schädlingsbekämpfungsmittel nach Anspruch 13, das weiters einen Synergisten oder Verstärker enthält.

**15.** Schädlingsbekämpfungsmittel nach Anspruch 13 oder 14, das weiters einen oder mehrere pestizid wirksame Bestandteile, Lockstoffe, Abwehrmittel, Bakterizide, Fungizide und/oder Antiwurmmittel enthält.

**16.** Verbindung der Formel (I) zur Verwendung in der Human- oder Veterinärmedizin.

**17.** Verfahren zur Bekämpfung von Schädlingsbefall auf Pflanzen und/oder gelagerten Produkten, welches Verfahren das Aufbringen einer wirksamen Menge einer Verbindung der Formel (I), wie in Anspruch 1 definiert, auf die Pflanze und/oder das gelagerte Produkt umfaßt.

**18.** Verfahren zur Herstellung einer Verbindung der Formel (I) nach einem der Ansprüche 1 bis 11, welches Verfahren

(i) das Umsetzen einer Verbindung der Formel (II):

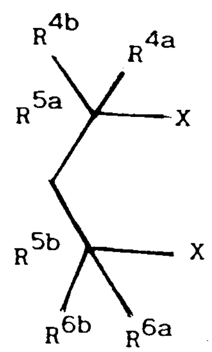

worin X SH bedeutet, mit einem geeigneten Aldehyd oder Keton der Formel

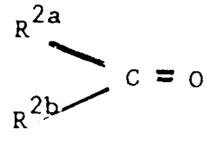

oder einem reaktiven Derivat hievon oder

(ii) zur Herstellung von Verbindungen, worin $R^{2a}$ Wasserstoff bedeutet, das Umsetzen eines Dithiaborinan-DimethylsulfidKomplexes einer Verbindung der Formel (II) mit einer Carbonsäure der Formel $R^{2b}CO_2H$,

(iii) zur Herstellung von Verbindungen, worin $R^{2a}$ Alkyl, Alkenyl oder Alkinyl bedeutet, das Umsetzen einer Verbindung der Formel (III)

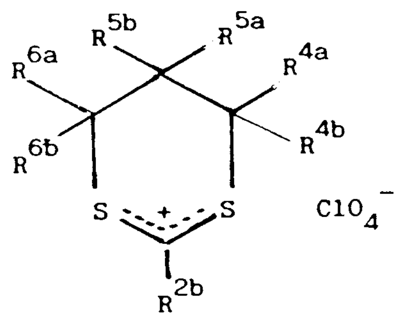

mit einem Alkohol $R^{2a}OH$ oder Grignard-Reagens $R^{2a}MgBr$, worin $R^{2b}$, $R^{4a}$, $R^{4b}$, $R^{5a}$, $R^{5b}$, $R^{6a}$ und $R^{6b}$ wie in einem der Ansprüche 1 bis 12 definiert sind, oder

(iv) zur Herstellung von Verbindungen, worin $R^{2a}$ Cyano bedeutet, das Umsetzen der entsprechenden Verbindung, worin $R^{2a}$ Methylthio darstellt, mit einer Verbindung $(Alk)_3SiCN$, worin Alk eine $C_1$-$C_4$-Alkylgruppe ist, oder

(v) zur Herstellung von Verbindungen, worin $R^{2a}$ Alkylthio bedeutet, das Umsetzen der entsprechenden Verbindung, worin $R^{2a}$ Wasserstoff darstellt, mit einem Dialkyldisulfid in Anwesenheit einer Alkyllithiumverbindung oder

(vi) zur Herstellung von Verbindungen, worin $R^{2b}$ Pyridylbedeutet, das Umsetzen der entsprechenden Verbindung, worin $R^{2b}$ Wasserstoff darstellt, mit Pyridin und nach einem der Schritte (i) bis (vi) gegebenenfalls das Oxidieren eines oder beider Ringschwefelatome oder

(vii) das Überführen einer Verbindung der Formel (I) in eine andere Verbindung der Formel (I) umfaßt.

**Patentansprüche für folgende Vertragsstaaten : ES, GR**

**1.** Verfahren zur Herstellung einer Verbindung der Formel (I):

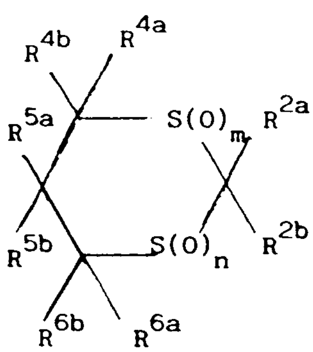

(I),

die 9 bis 30 Kohlenstoffatome enthält und worin m und n unabhängig aus Null, 1 und 2 ausgewählt sind; $R^{2a}$ Wasserstoff, Cyano, Methoxy, Ethoxy oder eine Gruppe $S(O)_qR^7$ bedeutet, wobei q Null, 1 oder 2 ist und $R^7$ Methyl oder Ethyl darstellt, oder $R^{2a}$ eine Gruppe $C{\equiv}CR^8$ ist, wobei $R^8$ Wasserstoff, $C_1$-$C_4$-Alkyl oder eine Silylgruppe enthaltend 3 $C_1$-$C_4$-Alkylgruppen oder 2 $C_1$-$C_4$-Alkylgruppen und eine Phenylgruppe darstellt, oder $R^{2a}$ Vinyl, Halogen, $C_1$-$C_3$-Alkyl gegebenenfalls substituiert durch Halogen, Methoxy, Ethoxy oder Cyano bedeutet oder $R^{2a}$ eine Gruppe $COR^9$ ist, worin $R^9$ Wasserstoff, $C_1$-$C_4$-Alkoxy, $C_1$-$C_4$-Alkyl oder eine Gruppe $NR^{10}R^{11}$ darstellt, wobei $R^{10}$ und $R^{11}$ unabhängig aus Wasserstoff, Methyl oder Ethyl ausgewählt sind; $R^{2b}$, das 2 bis 18 Kohlenstoffatome enthält, einen 5- oder 6-gliedrigen Ring enthaltend ein Sauerstoff-, Schwefel- und/oder Stickstoffatom oder eine Gruppe $R^{12}$ bedeutet, wobei $R^{12}$ eine $C_1$-$C_{16}$-Kohlenwasserstoffgruppe ist, jeweils gegebenenfalls substituiert

durch eine Azido-, Cyano-, Nitro- oder 2 Hydroxygruppen oder durch 1 bis 5 Halogenatome, die gleich oder verschieden sind, oder durch 1 bis 4 Gruppen $R^{13}$, die gleich oder verschieden sind und jeweils 1 bis 4 Heteroatome, die gleich oder verschieden sind und aus Sauerstoff, Schwefel, Stickstoff und Silizium ausgewählt sind, 1 bis 16 Kohlenstoffatome und gegebenenfalls 1 bis 9 Fluor- oder Chloratome enthalten oder im Fall des 5- oder 6-gliedrigen Ringes gegebenenfalls durch eine Gruppe $R^{12}$ substituiert sind; $R^{4a}$, $R^{4b}$, $R^{6a}$ und $R^{6b}$ unabhängig aus Wasserstoff, $C_1$-$C_3$-Alkyl, $C_2$-$C_3$-Alkenyl oder Alkinyl jeweils gegebenenfalls substituiert durch Halogen, Cyano oder $C_1$-$C_4$-Alkoxy, Cyano, Halogen oder einer Gruppe $COR^{9'}$ ausgewählt sind, wobei $R^{9'}$ Wasserstoff, $C_1$-$C_4$-Alkyl oder eine Gruppe $NR^{10'}R^{11'}$ bedeutet, worin $R^{10'}$ und $R^{11'}$ unabhängig aus Wasserstoff, Methyl oder Ethyl ausgewählt sind; $R^{5a}$ eine nicht-aromatische Kohlenwasserstoffgruppe mit bis zu 7 Kohlenstoffatomen oder Phenyl jeweils gegebenenfalls substituiert durch Cyano, Halogen, $C_1$-$C_4$-Alkyl, $C_1$-$C_4$-Halogenalkyl, $C_3$-$C_4$-Cycloalkyl, $C_1$-$C_4$-Alkoxy oder eine Gruppe $S(O)_{q'}R^{7'}$ darstellt, wobei q' Null, 1 oder 2 ist und $R^{7'}$ Methyl oder Ethyl bedeutet und $R^{5b}$ Wasserstoff oder $C_1$-$C_4$-Alkyl gegebenenfalls substituiert durch Alkoxy darstellt; mit der Maßgabe, daß, wenn $R^{2a}$ Wasserstoff, Methyl oder Ethyl bedeutet, $R^{2b}$ Acetylen ist oder 3 bis 18 Kohlenstoffatome enthält und eine Gruppe $R^{14}$ darstellt, wobei $R^{14}$ eine nicht-aromatische $C_1$-$C_{13}$-Kohlenwasserstoffgruppe gegebenenfalls substituiert durch eine $C_1$-$C_4$-Carbalkoxy- oder Cyanogruppe und/oder durch 1 oder 2 Hydroxygruppen und/oder durch 1 bis 5 Halogenatome, die gleich oder verschieden sind, und/oder durch 1 bis 3 Gruppen $R^{15}$, die gleich oder verschieden sind und jeweils 1 bis 4 Heteroatome, die gleich oder verschieden sind und aus Sauerstoff, Schwefel, Stickstoff und Silizium ausgewählt sind, 1 bis 10 Kohlenstoffatome und gegebenenfalls 1 bis 6 Fluor- oder Chloratome enthalten, bedeutet, oder $R^{2b}$ einen 6-gliedrigen aromatischen Ring substituiert durch Cyano und/oder 1 bis 3 Gruppen $R^{15}$ und/oder eine Gruppe -C≡CH, -C≡C-$R^{14}$ oder C≡C-Halogen und/oder 1 bis 5 Halogenatome und/oder 1 bis 3 $C_1$-$C_4$-Halogenalkylgruppen bedeutet, wobei $R^{14}$ und $R^{15}$ wie vorstehend definiert sind; $R^{4a}$ und $R^{6a}$ gleich oder verschieden sind und aus Wasserstoff, Methyl, Trifluormethyl oder Cyano ausgewählt sind; $R^{4b}$ und $R^{6b}$ Wasserstoff bedeuten und $R^{5b}$ Wasserstoff oder Methyl darstellt, $R^{5a}$ nicht tert.Butyl ist, welches Verfahren:

(i) das Umsetzen einer Verbindung der Formel (II):

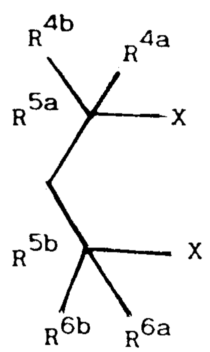

(II),

worin X SH bedeutet, mit einem geeigneten Aldehyd oder Keton der Formel

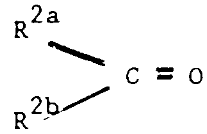

oder einem reaktiven Derivat hievon oder

(ii) wenn $R^{2a}$ Wasserstoff bedeutet, das Umsetzen eines Dithiaborinan-Dimethylsulfid-Komplexes einer Verbindung der Formel (II) mit einer Carbonsäure der Formel:

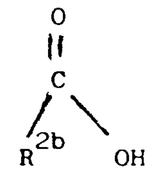

(iii) zur Herstellung von Verbindungen, worin $R^{2a}$ Alkyl,Alkenyl oder Alkinyl bedeutet, das Umsetzen einer Verbindung der Formel (III)

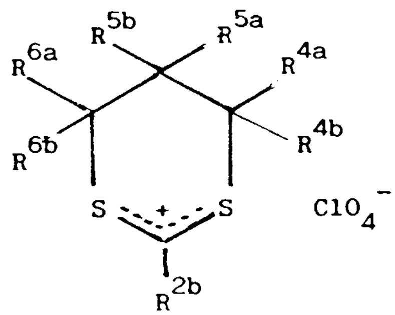

mit einem Alkohol $R^{2a}OH$ oder Grignard-Reagens $R^{2a}MgBr$, worin $R^{2b}$, $R^{4a}$, $R^{4b}$, $R^{5a}$, $R^{5b}$, $R^{6a}$ und $R^{6b}$ wie vorstehend definiert sind, oder

(iv) zur Herstellung von Verbindungen, worin $R^{2a}$ Cyano bedeutet, das Umsetzen der entsprechenden Verbindung, worin $R^{2a}$ Methylthio darstellt, mit einer Verbindung $(Alk)_3SiCN$, worin Alk eine $C_1$-$C_4$-Alkylgruppe ist, oder

(v) zur Herstellung von Verbindungen, worin $R^{2a}$ Alkylthio bedeutet, das Umsetzen der entsprechenden Verbindung, worin $R^{2a}$ Wasserstoff darstellt, mit einem Dialkyldisulfid in Anwesenheit einer Alkyllithiumverbindung oder

(vi) zur Herstellung von Verbindungen, worin $R^{2b}$ Pyridylbedeutet, das Umsetzen der entsprechenden Verbindung, worin $R^{2b}$ Wasserstoff darstellt, mit Pyridin und nach einem der Schritte (i) bis (vi) gegebenenfalls das Oxidieren eines oder beider Ringschwefelatome oder

(vii) das Überführen einer Verbindung der Formel (I) in eine andere Verbindung der Formel (I) umfaßt.

2. Verfahren nach Anspruch 1 zur Herstellung einer Verbindung der Formel (I), worin $R^{2b}$ eine Phenylgruppe substituiert in Stellung 3, 4 oder 5 durch 1 bis 3 Substituenten jeweils ausgewählt aus Halogen, $C_1$-$C_4$-Halogenalkyl, Cyano, Azido, eine Gruppe $(C\equiv C)_pR^{16}$ oder eine Gruppe $S(O)_wR^{17}$ bedeutet, wobei p 1 oder 2 ist, w Null, 1 oder 2 ist, $R^{16}$ Wasserstoff, Brom, Chlor oder Iod darstellt und $R^{17}$ Trifluormethyl, Methyl oder Ethyl bedeutet, und die Phenylgruppe weiters gegebenenfalls durch Fluor oder Chlor in Stellung 2 und/oder 6 substituiert ist.

3. Verfahren nach Anspruch 1 oder 2 zur Herstellung einer Verbindung der Formel (I), worin $R^{2b}$ Phenyl substituiert in Stellung 3, 4 oder 5 durch 1 bis 3 Substituenten jeweils ausgewählt aus Halogen, Cyano, $C_1$-$C_4$-Halogenalkyl oder eine Gruppe $C\equiv C$-$R^{21}$ bedeutet, wobei $R^{21}$ Wasserstoff, Methyl oder Ethyl jeweils gegebenenfalls substituiert durch Hydroxy, Methoxy, Ethoxy, Acetoxy darstellt oder $R^{21}$ $C_1$-$C_4$-Carbalkoxy oder eine Silylgruppe substituiert durch 3 $C_1$-$C_4$-Alkylgruppen bedeutet, und die Phenylgruppe weiters gegebenenfalls durch Fluor oder Chlor in Stellung 2 und/oder 6 substituiert ist.

4. Verfahren nach Anspruch 1 oder 2 zur Herstellung einer Verbindung der Formel (I), worin $R^{2b}$ eine Gruppe -A(C≡C)Z bedeutet, wobei A eine Methylen- oder Polymethylenkette gegebenenfalls enthaltend eine Doppelbindung und/oder ein Sauerstoff- oder Schwefelatom ist und Z Wasserstoff, $C_1$-$C_5$-Alkyl, $C_1$-$C_3$-Alkoxymethyl oder eine Gruppe $SiR^{18}R^{19}R^{20}$ darstellt, wobei $R^{18}$ bis $R^{20}$ gleich oder verschieden sind und jeweils eine aliphatische $C_1$-$C_4$-Gruppe bedeuten oder $R^{18}$ und $R^{19}$ aliphatische $C_1$-$C_4$-Gruppen darstellen und $R^{20}$ eine Phenylgruppe ist.

5. Verfahren nach Anspruch 1 oder 2 zur Herstellung einer Verbindung der Formel (I), worin $R^{2b}$ eine Gruppe $-BZ^1$ ist, wobei B eine Gruppe $-CH_2O-$ oder $-CH_2S(O)_w-$, wobei w Null, 1 oder 2 ist, oder eine aliphatische $C_2$-$C_6$-Gruppe gegebenenfalls substituiert durch 1 bis 3 Halogenatome bedeutet und $Z^1$ Silyl substituiert durch 3 $C_1$-$C_4$-Alkylgruppen oder eine Gruppe

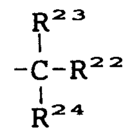

darstellt, worin $R^{22}$, $R^{23}$ und $R^{24}$ gleich oder verschieden sind und jeweils unabhängig aus Halogen, $C_1$-$C_4$-Alkyl gegebenenfalls substituiert durch Halogen, $C_1$-$C_4$-Alkoxy oder einer Gruppe $S(O)_qR^{25}$ ausgewählt sind, wobei q Null, 1 oder 2 ist und $R^{25}$ $C_1$-$C_4$-Alkyl bedeutet, oder $R^{22}$, $R^{23}$ und $R^{24}$ aus $C_1$-$C_4$-Alkoxy oder einer Gruppe $S(O)_wR^{26}$ ausgewählt sind, wobei w Null, 1 oder 2 ist und $R^{26}$ $C_1$-$C_4$-Alkyl gegebenenfalls substituiert durch Fluor bedeutet, oder $R^{22}$ und $R^{23}$ unter Bildung eines $C_3$-$C_6$-Cycloalkylringes verbunden sind oder eines von $R^{22}$, $R^{23}$ und $R^{24}$ Wasserstoff darstellen kann.

6. Verfahren nach Anspruch 1 zur Herstellung einer Verbindung der Formel (I), worin $R^{2b}$ eine Gruppe

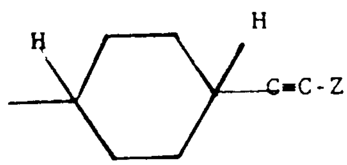

bedeutet, worin Z wie in Anspruch 4 definiert ist.

7. Verfahren nach Anspruch 1 zur Herstellung einer Verbindung der Formel (I), worin $R^{2a}$ Wasserstoff, Methyl oder Ethyl bedeutet.

8. Verfahren nach Anspruch 1 zur Herstellung einer Verbindung der Formel (I), worin $R^{2b}$ Phenyl, substituiertes Phenyl oder einen Heterocyclus mit 4 bis 6 Kohlenstoffatomen bedeutet, ausgenommen daß, (1) wenn $R^{5a}$ geradkettiges Alkyl oder Tolyl ist und $R^{2a}$, $R^{4a}$, $R^{4b}$, $R^{6a}$, $R^{6b}$ und $R^{5b}$ alle Wasserstoff darstellen, $R^{2b}$ nicht p-Cyanophenyl bedeutet; (ii) wenn $R^{5a}$ und $R^{5b}$ beide Ethyl sind und $R^{4a}$, $R^{4b}$, $R^{6a}$, $R^{6b}$ und $R^{2a}$ alle Wasserstoff darstellen, $R^{2b}$ nicht p-Tolyl bedeutet; (iii) wenn $R^{5a}$ n-Pentyl ist und $R^{2a}$, $R^{4a}$, $R^{4b}$, $R^{6a}$, $R^{6b}$ und $R^{5b}$ alle Wasserstoff darstellen, $R^{2b}$ nicht p-Bromphenyl bedeutet, (iv) wenn $R^{5b}$ und $R^{5a}$ beide Methyl sind und $R^{4a}$, $R^{4b}$, $R^{6b}$ und $R^{2a}$ alle Wasserstoff darstellen, $R^{2b}$ nicht unsubstituiertes Phenyl bedeutet, und (v) wenn $R^{2b}$ n-Propyl ist, $R^{2a}$ Methyl bedeutet und $R^{4a}$, $R^{4b}$, $R^{6a}$, $R^{6b}$ und $R^{5b}$ alle Wasserstoff darstellen, $R^{5a}$ nicht Ethyl oder Isopropyl bedeutet.

9. Verfahren nach Anspruch 1 zur Herstellung einer Verbindung der Formel (I), worin $R^{4a}$, $R^{4b}$, $R^{6a}$ und $R^{6b}$ Wasserstoff bedeuten.

10. Verfahren nach Anspruch 1 zur Herstellung einer Verbindung der Formel (Ia):

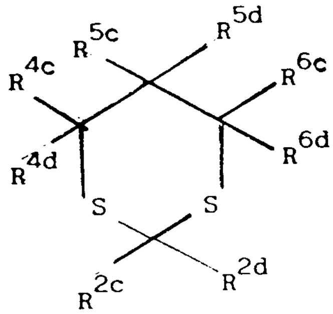

(Ia),

worin $R^{2c}$ aus Wasserstoff, Cyano, Methoxy oder einer Gruppe $S(O)_qR^7$ ausgewählt ist, wobei q Null, 1 oder 2 ist und $R^7$ Methyl oder Ethyl bedeutet, oder $R^{2c}$ Vinyl, Halogen, $C_1$-$C_3$-Alkyl gegebenenfalls substituiert durch Halogen, Methoxy, Ethoxy oder Cyano darstellt; $R^{2d}$ eine Gruppe $(C{\equiv}C)_rY(C{\equiv}C)_tZ^2$ bedeutet, wobei r Null oder 1 ist und t 1 oder 2 ist und die Summe von r und t nicht größer als 2 ist, Y eine Einfachbindung, eine Gruppe

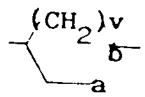

,

worin v 1, 2 oder 3 ist und das $(C{\equiv}C)_tZ^2$-Fragment an die Stellung a oder b des Ringes gebunden ist, darstellt oder Y eine Methylen- oder Polymethylenkette mit 1 bis 8 Kohlenstoffatomen bedeutet, in die 1 oder 2 Heteroatome und/oder Doppel- oder Dreifachbindungen eingefügt sein können, wobei die Kette gegebenenfalls durch 1 bis 4 Substituenten substituiert ist, die gleich oder verschieden sein können und jeweils unabhängig aus Hydroxy, Oxo, Halogen, $C_1$-$C_4$-Alkyl, $C_1$-$C_4$-Alkoxy, $C_1$-$C_4$-Acyloxy, Epoxy, einer $C_1$-$C_4$-Alkylidengruppe, einer $C_1$-$C_4$-Carbalkoxygruppe, $C_1$-$C_4$-Halogenalkyl oder Cyano ausgewählt sind, $Z^2$ aus Wasserstoff, $C_1$-$C_{10}$-Kohlenwasserstoff gegebenenfalls substituiert durch Halogen, $C_1$-$C_4$-Alkoxy, Hydroxy, Oxo, einer Gruppe $S(O)_qR^7$, wobei q Null, 1 oder 2 ist und $R^7$ Methyl oder Ethyl bedeutet, ausgewählt ist, oder $Z^2$ eine Cyanogruppe, $C_1$-$C_4$-Acyloxy oder Carbalkoxy, Halogen oder eine Gruppe $SiR^{27}R^{28}R^{29}$ darstellt, wobei $R^{27}$, $R^{28}$ und $R^{29}$ gleich oder verschieden sind und jeweils eine Kohlenwasserstoffgruppe mit bis zu 4 Kohlenstoffatomen bedeuten, oder $Z^2$ eine Gruppe $R^{30}OCO$ ist, worin $R^{30}$ $C_1$-$C_4$-Alkyl darstellt; $R^{4c}$, $R^{4d}$, $R^{6c}$ und $R^{6d}$ jeweils aus Wasserstoff, Methyl, Cyano und Trifluormethyl ausgewählt sind; $R^{5c}$ Alkyl, Cycloalkyl, Alkenyl, Cycloalkenyl oder Alkinyl jeweils enthaltend bis zu 7 Kohlenstoffatome oder Phenyl jeweils gegebenenfalls substituiert durch Halogen, Cyano, $C_1$-$C_4$-Alkyl, $C_1$-$C_4$-Halogenalkyl, $C_3$-$C_4$-Cycloalkyl, $C_1$-$C_4$-Alkoxy oder eine Gruppe $S(O)_qR^7$, wie vorstehend definiert, bedeutet; und $R^{5d}$ Wasserstoff oder $C_1$-$C_4$-Alkyl darstellt, mit der Maßgabe, daß $R^{5c}$ nicht tert.Butyl ist.

**11.** Verfahren nach Anspruch 1 zur Herstellung einer Verbindung der Formel (Ia), wie in Anspruch 10 definiert, worin $R^{2d}$ eine Gruppe $-(C{\equiv}C)-Bu^t$ bedeutet.

**12.** Verfahren nach Anspruch 1 zur Herstellung von:
cis-2(a)-(4-Bromphenyl)-5(e)-butyl-1,3-dithian,
5(e)-tert.Butyl-2(e)-(4-methylphenyl)-1,3-dithian,
5(e)-tert.Butyl-2(e)-(4-nitrophenyl)-1,3-dithian,
2(e)-(4-Bromphenyl)-5(e)-neopentyl-1,3-dithian,
cis-2(e)-(4-Bromphenyl)-5(e)-tert.butyl-2(a)-methylthio-1,3-dithian,
2(e)-(4-Bromphenyl)-5(e)-tert.butyl-2(a)-methoxy-1,3-dithian,
trans-2(e)-(4-Bromphenyl)-5(e)-ethyl-1,3-dithian,
2(e)-(4-Bromphenyl)-2(a)-methyl-5(e)-(1,1-dimethylpropyl)-1,3-dithian,
2(e)-(4-Bromphenyl)-5(e)-tert.butyl-1,3-dithian-2(a)-carbonitril,
2(a)-(4-Bromphenyl)-5(e)-ethyl-2(e)-methyl-1,3-dithian,
trans-5(e)-tert.Butyl-2(e)-(4-tert.butylphenyl)-1,3-dithian,
2-(5(e)-tert.Butyl-1,3-dithian-2(e)-yl)-propan-2-on,
trans-5(e)-tert.Butyl-2(e)-(2,2-dibromethenyl)-1,3-dithian,

trans-2(e)-Bromethinyl-5(e)-tert.butyl-1,3-dithian,
4-(trans-5(e)-tert.Butyl-1,3-dithian-2(e)-yl)-2,2-dimethylbutan-3-on,
2(e)-(3,3-Dimethylbutyl)-5(e)-ethyl-1,3-dithian,
2(a)-(4-Bromphenyl)-2(e)-methyl-5(e)-(1,1-dimethylpropyl)-1,3-dithian.

**13.** Schädlingsbekämpfungsmittel umfassend eine Verbindung der Formel (I), wie in einem der vorhergehenden Ansprüche definiert, gemischt mit einem oder mehreren Trägern oder Verdünnungsmitteln.

**14.** Schädlingsbekämpfungsmittel nach Anspruch 13, das weiters einen Synergisten oder Verstärker enthält.

**15.** Schädlingsbekämpfungsmittel nach Anspruch 13 oder 14, das weiters einen oder mehrere pestizid wirksame Bestandteile, Lockstoffe, Abwehrmittel, Bakterizide, Fungizide und/oder Antiwurmmittel enthält.

**16.** Verbindung der Formel (I) zur Verwendung in der Human- oder Veterinärmedizin.

**17.** Verfahren zur Bekämpfung von Gliederfüßer- oder Wurmschädlingen, welches Verfahren das Aufbringen einer wirksamen Menge einer Verbindung der Formel (I), hergestellt nach einem der Ansprüche 1 bis 12, auf den Gliederfüßer oder den Wurm umfaßt.

**18.** Verfahren zur Bekämpfung von Schädlingsbefall auf Tieren und/oder gelagerten Produkten und/oder einer Umgebung, welches Verfahren das Aufbringen einer Verbindung der Formel (I), wie in einem der vorhergehenden Ansprüche definiert, auf ein Tier und/oder auf das gelagerte Produkt und/oder eine Umgebung, die für Schädlingsbefall anfällig ist, umfaßt.

**19.** Verfahren zur Bekämpfung von Schädlingsbefall auf Pflanzen und/oder gelagerten Produkten und/oder einer Umgebung, welches Verfahren das Aufbringen einer wirksamen Menge einer Verbindung der Formel (I), wie in einem der vorhergehenden Ansprüche definiert, auf die Pflanze und/oder das gelagerte Produkt und/oder eine Umgebung, die für Schädlingsbefall anfällig ist, umfaßt.

**Revendications**
**Revendications pour les Etats contractants suivants : AT, BE, CH, DE, FR, GB, IT, LI, LU, NL, SE**

**1.** Composé de formule :

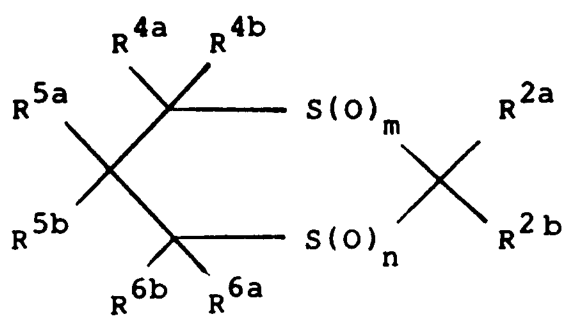

(I)

qui contient entre 9 et 30 atomes de carbone,
et où

m et n sont choisis indépendamment parmi 0, 1 et 2;

$R^{2a}$ est hydrogène, cyano, méthoxy, éthoxy, un radical $S(O)_qR^7$, où q est 0, 1 ou 2 et $R^7$ est méthyle ou éthyle, ou $R^{2a}$ est un radical $C \equiv CR^8$, où $R^8$ est hydrogène, $C_{1-4}$-alcoyle ou un radical silyle contenant trois radicaux $C_{1-4}$-alcoyle ou deux radicaux $C_{1-4}$-alcoyle et un radical phényle, ou $R^{2a}$ est vinyle, halo, $C_{1-3}$-alcoyle facultativement substitué par halo, méthoxy, éthoxy ou cyano, ou $R^{2a}$ est un radical $COR^9$, où $R^9$ est hydrogène, $C_{1-4}$-alcoxy, $C_{1-4}$-alcoyle ou un radical $NR^{10}R^{11}$, où $R^{10}$ ou $R^{11}$ sont choisis indépendamment parmi hydrogène, méthyle et éthyle;

$R^{2b}$, qui contient entre 2 et 18 atomes de carbone, est un cycle de 5 ou 6 chaînons contenant un

atome d'oxygène, de soufre et/ou d'azote ou un radical $R^{12}$, où $R^{12}$ est un radical $C_{1-16}$-hydrocarbyle, chacun facultativement substitué par un radical azido, cyano ou nitro ou deux radicaux hydroxyle, ou par 1 à 5 atomes halo qui sont identiques ou différents, ou par 1 à 4 radicaux $R^{13}$ qui sont identiques ou différents et contiennent chacun 1 à 4 hétéroatomes, qui sont identiques ou différents et sont choisis parmi oxygène, soufre, azote et silicium, 1 à 16 atomes de carbone et facultativement 1 à 9 atomes fluoro ou chloro, ou dans le cas du cycle de 5 ou 6 chaînons facultativement substitué par un radical $R^{12}$;

$R^{4a}$ et $R^{4b}$, $R^{6a}$ et $R^{6b}$ sont choisis indépendamment parmi hydrogène, $C_{1-3}$-alcoyle, $C_{2-3}$-alcényle ou alcynyle, chacun facultativement substitué par halo, cyano ou $C_{1-4}$-alcoxy; cyano, halo ou un radical $COR^{9'}$, où $R^{9'}$ est hydrogène, $C_{1-4}$-alcoxy, $C_{1-4}$-alcoyle ou un radical $NR^{10'}R^{11'}$, où $R^{10'}$ et $R^{11'}$ sont choisis indépendamment parmi hydrogène, méthyle et éthyle;

$R^{5a}$ est un radical hydrocarbyle non aromatique comptant jusqu'à 7 atomes de carbone ou phényle, chacun facultativement substitué par cyano, halo, $C_{1-4}$-alcoyle, $C_{1-4}$-haloalcoyle, $C_{3-4}$-cycloalcoyle, $C_{1-4}$-alcoxy ou un radical $S(O)_{q'}R^{7'}$, où q' est 0 1 ou 2 et $R^{7'}$ est méthyle ou éthyle et $R^{5b}$ est hydrogène ou $C_{1-4}$-alcoyle facultativement substitué par alcoxy; avec la restriction que lorsque $R^{2a}$ est hydrogène, méthyle ou éthyle; $R^{2b}$ est acétylène ou contient entre 3 et 18 atomes de carbone et est un radical $R^{14}$, où $R^{14}$ est un radical $C_{1-13}$-hydrocarbyle non aromatique facultativement substitué par un radical $C_{1-4}$-carbalcoxy ou cyano et/ou par 1 ou 2 radicaux hydroxyle et/ou par 1 à 5 atomes halo qui sont identiques ou différents et/ou par 1 à 3 radicaux $R^{15}$ qui sont identiques ou différents et contiennent chacun 1 à 4 hétéroatomes qui sont identiques ou différents et sont choisis parmi oxygène, soufre, azote et silicium, 1 à 10 atomes de carbone et facultativement 1 à 6 atomes fluoro ou chloro, ou $R^{2b}$ est un cycle aromatique à six chaînons substitué par cyano et/ou par 1 à 3 radicaux $R^{15}$ et/ou par un radical -C≡CH, -C≡C $R^{14}$ ou -C≡C-halo et/ou par 1 à 5 atomes halo et/ou par 1 à 3 radicaux $C_{1-4}$-haloalcoyle où $R^{14}$ et $R^{15}$ sont tels que définis ci-dessus; $R^{4a}$ et $R^{6a}$ sont identiques ou différents et sont choisis parmi hydrogène, méthyle, trifluorométhyle et cyano; $R^{4b}$ et $R^{6b}$ sont hydrogène et $R^{5b}$ est hydrogène ou méthyle, alors $R^{5a}$ n'est pas butyle tertiaire.

**2.** Composé de formule (I) suivant la revendication 1, dans lequel $R^{2b}$ est un radical phényle substitué aux positions 3, 4 et 5 par un à trois substituants choisis chacun parmi halo, $C_{1-4}$-haloalcoyle, cyano, azido, un radical $(C{\equiv}C)_pR^{16}$ ou un radical $(S)O_wR^{17}$, où p est 1 ou 2, w est 0, 1 ou 2, $R^{16}$ est hydrogène, brome, chlore ou iode et $R^{17}$ est trifluorométhyle, méthyle ou éthyle et le radical phényle est, en outre, facultativement substitué aux positions 2 et/ou 6 par fluoro ou chloro.

**3.** Composé de formule (I) suivant la revendication 1 ou 2, dans lequel $R^{2b}$ est phényle substitué aux positions 3, 4 et 5 par 1 à 3 substituants choisis chacun parmi halo, cyano, $C_{1-4}$-haloalcoyle ou un radical C≡C-$R^{21}$, où $R^{21}$ est hydrogène, méthyle ou éthyle, chacun facultativement substitué par hydroxyle, méthoxy, éthoxy, acétoxy; ou $R^{21}$ est $C_{1-4}$-carbalcoxy ou un radical silyle substitué par trois radicaux $C_{1-4}$-alcoyle et le radical phényle est, en outre, facultativement substitué aux positions 2 et/ou 6 par fluoro ou chloro.

**4.** Composé de formule (I) suivant la revendication 1 ou 2, dans lequel $R^{2b}$ est un radical -A(C≡C)Z, où A est une chaîne méthylène ou polyméthylène contenant facultativement une double liaison et/ou un atome d'oxygène ou de soufre et Z est hydrogène, $C_{1-5}$-alcoyle, $C_{1-3}$-alcoxyméthyle ou un radical $SiR^{18}R^{19}R^{20}$, où $R^{18}$ à $R^{20}$ sont identiques ou différents et sont chacun un radical $C_{1-4}$-aliphatique, ou $R^{18}$ et $R^{19}$ sont des radicaux $C_{1-4}$-aliphatiques et $R^{20}$ est un radical phényle.

**5.** Composé de formule (I) suivant la revendication 1 ou 2, dans lequel $R^{2b}$ est un radical -$BZ^1$, où B est un radical -$CH_2O$- ou -$CH_2S(O)_w$-, où w est 0, 1 ou 2, ou un radical $C_{2-6}$-aliphatique facultativement substitué par 1 à 3 atomes halo et $Z^1$ est silyle substitué par 1 à 3 radicaux $C_{1-4}$-alcoyle ou un radical -C-($R^{22}$)($R^{23}$)$R^{24}$, où $R^{22}$, $R^{23}$ et $R^{24}$ sont identiques ou différents et sont choisis chacun indépendamment parmi halo, $C_{1-4}$-alcoyle facultativement substitué par halo, $C_{1-4}$-alcoxy ou un radical $S(O)_qR^{25}$, où q est 0, 1 ou 2, $R^{25}$ est $C_{1-4}$-alcoyle, ou $R^{22}$, $R^{23}$ et $R^{24}$ sont choisis parmi $C_{1-4}$-alcoxy ou un radical $S(O)_wR^{26}$, où w est 0, 1 ou 2 et $R^{26}$ est $C_{1-4}$-alcoyle facultativement substitué par fluoro, ou $R^{22}$ et $R^{23}$ sont unis pour former un cycle $C_{3-6}$-cycloalcoyle, ou l'un d'entre $R^{22}$, $R^{23}$ et $R^{24}$ peut être hydrogène.

**6.** Composé de formule (I) suivant la revendication 1, dans lequel $R^{2b}$ est un radical :

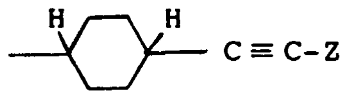

où Z est tel que défini dans la revendication 4.

**7.** Composé de formule (I) suivant l'une quelconque des revendications 1 à 6, dans lequel $R^{2a}$ est hydrogène, méthyle ou éthyle.

**8.** Composé de formule (I) suivant la revendication 1, dans lequel $R^{2b}$ est phényle, phényle substitué ou un hétérocycle comptant 4 à 6 atomes de carbone sauf que (i) lorsque $R^{5a}$ est alcoyle en chaîne droite ou tolyle et $R^{2a}$, $R^{4a}$, $R^{4b}$, $R^{6a}$, $R^{6b}$ et $R^{5b}$ sont tous hydrogène, $R^{2b}$ n'est pas para-cyanophényle; (ii) lorsque $R^{5a}$ et $R^{5b}$ sont tous deux éthyle et $R^{4a}$, $R^{4b}$, $R^{6a}$, $R^{6b}$ et $R^{2a}$ sont tous hydrogène, $R^{2b}$ n'est pas para-tolyle; (iii) lorsque $R^{5a}$ est n-pentyle et $R^{2a}$, $R^{4a}$, $R^{4b}$, $R^{6a}$, $R^{6b}$ et $R^{5b}$ sont tous hydrogène, $R^{2b}$ n'est pas para-bromophényle; (iv) lorsque $R^{5b}$ et $R^{5a}$ sont tous deux méthyle et $R^{4a}$, $R^{4b}$, $R^{6b}$ et $R^{2a}$ sont tous hydrogène, $R^{2b}$ n'est pas phényle non substitué et (v) lorsque $R^{2b}$ est n-propyle, $R^{2a}$ est méthyle et $R^{4a}$, $R^{4b}$, $R^{6a}$, $R^{6b}$ et $R^{5b}$ sont tous hydrogène, $R^{5a}$ n'est pas éthyle ou i-propyle.

**9.** Composé de formule (I) suivant l'une quelconque des revendications 1 à 8, dans lequel $R^{4a}$, $R^{4b}$, $R^{6a}$ et $R^{6b}$ sont hydrogène.

**10.** Composé suivant la revendication 1 de formule (Ia) :

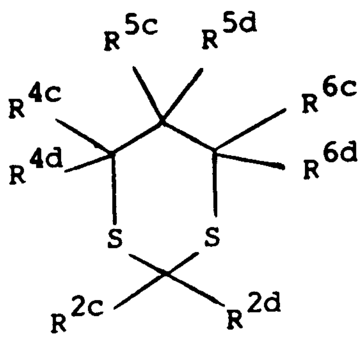

(Ia)

où

$R^{2c}$ est choisi parmi hydrogène, cyano, méthoxy, un radical $S(O)_qR^7$, où q est 0, 1 ou 2 et $R^7$ est méthyle ou éthyle, ou $R^{2c}$ est vinyle, halo, $C_{1-3}$-alcoyle facultativement substitué par halo, méthoxy, éthoxy ou cyano;

$R^{2d}$ est un radical $(C{\equiv}C)_rY(C{\equiv}C)_tZ^2$, où r est 0 ou 1 et t est 1 ou 2 et la somme de r et t n'est pas supérieure à 2, Y est une liaison simple, un radical

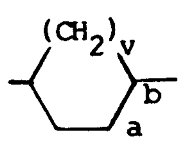

où v est 1, 2 ou 3 et le fragment $(C{\equiv}C)_tZ^2$ est uni à la position a ou b du cycle, ou Y est une chaîne méthylène ou polyméthylène contenant entre 1 et 8 atomes de carbone dans laquelle 1 ou 2 hétéroatomes et/ou doubles ou triples liaisons peuvent être intercalés, la chaine étant facultativement substituée par 1 à 4 substituants qui peuvent être identiques ou différents et sont chacun choisis indépendamment parmi hydroxyle, oxo, halo, $C_{1-4}$-alcoyle, $C_{1-4}$-alcoxy, $C_{1-4}$-alcyloxy, époxy, un radical $C_{1-4}$-alcoylidène, un radical $C_{1-6}$-carbalcoxy, $C_{1-4}$-haloalcoyle ou cyano, $Z^2$ est choisi parmi

hydrogène, $C_{1-10}$-hydrocarbyle facultativement substitué par halo, $C_{1-4}$-alcoxy, hydroxyle, oxo, un radical $S(O)_qR^7$ où q est 0, 1, 2 et $R^7$ est méthyle ou éthyle; ou $Z^2$ est un radical cyano, $C_{1-4}$-acyloxy ou carbalcoxy, halo ou un radical $SiR^{27}R^{28}R^{29}$, où $R^{27}$, $R^{28}$ et $R^{29}$ sont identiques ou différents et sont chacun un radical hydrocarbyle contenant jusqu'à 4 atomes de carbone, ou $Z^2$ est un radical $R^{30}OCO$, où $R^{30}$ est $C_{1-4}$-alcoyle;

$R^{4c}$, $R^{4d}$, $R^{6c}$ et $R^{6d}$ sont chacun choisis parmi hydrogène, méthyle, cyano et trifluorométhyle;

$R^{5c}$ est alcoyle, cycloalcoyle, alcényle, cycloalcényle ou alcynyle comptant chacun jusqu'à 7 atomes de carbone ou phényle, chacun facultativement substitué par halo, cyano, $C_{1-4}$-alcoyle, $C_{1-4}$-haloalcoyle, $C_{3-4}$-cycloalcoyle, $C_{1-4}$-alcoxy ou un radical $S(O)_qR^7$ tel que défini ci-dessus, et

$R^{5d}$ est hydrogène ou $C_{1-4}$-alcoyle, avec la restriction que $R^{5c}$ n'est pas butyle tertiaire.

**11.** Composé de formule (Ia) suivant la revendication 10, dans lequel $R^{2d}$ est un radical -(C≡C)-$Bu^t$.

**12.** Composé choisi parmi :

le cis-2(a)-(4-bromophényl)-5(e)-butyl-1,3-dithiane,
le (e)-(t-butyl-2(e)-(4-méthylphényl)-1,3-dithiane,
le 5(e)-(t-butyl-2(e)-(4-nitrophényl)-1,3-dithiane,
le 2(e)-(4-bromophényl)-5(e)-néopentyl-1,3-dithiane,
le cis-2(e)-(4-bromophényl)-5(e)-t-butyl-2(a)-méthylthio-1,3-dithiane,
le 2(e)-(4-bromophényl)-5(e)-t-butyl-2(a)-méthoxy-1,3-dithiane,
le trans-2(e)-(4-bromophényl)-5(e)-éthyl-1,3-dithiane,
le 2(e)-(4-bromophényl)-2(a)-méthyl-5(e)-(1,1-diméthylpropyl)-1,3-dithiane,
le 2(e)-(4-bromophényl)-5(e)-t-butyl-1,3-dithiane-2(a)-carbonitrile,
le 2(a)-(4-bromophényl)-5(e)-éthyl-2(e)-méthyl-1,3-dithiane,
le trans-5(e)-t-butyl-2(e)-(4-t-butylphényl)-1,3-dithiane,
la 2-(5(e)-t-butyl-1,3-dithian-2(e)-yl)propan-2-one,
le trans-5(e)-t-butyl-(2e)-(2,2-dibromoéthényl)-1,3-dithiane,
le trans-2(e)-bromoéthynyl-5(e)-t-butyl-1,3-dithiane,
la 4-trans-5(e)-t-butyl-1,3-dithiane-2(e)-yl)-2,2-diméthylbutan-3-one,
le 2(e)-(3,3-diméthylbutyl)-5(e)-éthyl-1,3-dithiane,
le 2(a)-(4-bromophényl)-2(e)-méthyl-5(e)-(1,1-diméthylpropyl)-1,3-dithiane.

**13.** Composition pesticide comprenant un composé de formule (I) tel que défini dans la revendication 1, en mélange avec un ou plusieurs excipients ou diluants.

**14.** Composition pesticide suivant la revendication 13, qui contient, en outre, un agent de synergie ou de potentialisation.

**15.** Composition pesticide suivant la revendication 13 ou 14, qui contient, en outre, un ou plusieurs constituants actifs pesticides, attractifs, répulsifs, bactéricides, fongicides et/ou anthelminthiques.

**16.** Composé de formule (I) à utiliser en médecine humaine ou vétérinaire.

**17.** Procédé de lutte contre les infestations par les organismes nuisibles sur des plantes et/ou des produits stockés, qui comprend l'administration d'une quantité efficace d'un composé de formule (I) tel que défini dans la revendication 1 à la plante et/ou au produit stocké.

**18.** Procédé de préparation d'un composé de formule( I) tel que défini dans l'une quelconque des revendications 1 à 11, qui comprend :

(i) la réaction d'un composé de formule (II) :

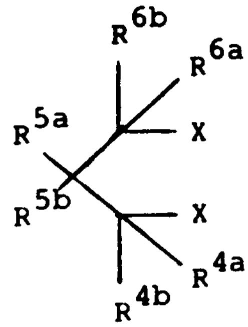

(II)

où X est SH, avec un aldéhyde ou cétone convenable de formule $R^{2a}(R^{2b})C=O$ ou un dérivé réactif de celui-ci, ou

(ii) pour la préparation de composés où $R^{2a}$ est hydrogène, la réaction d'un complexe dithiaborinane-sulfure de diméthyle d'un composé de formule (II) avec un acide carboxylique de formule $R^{2b}CO_2H$,

(iii) pour la préparation de composés où $R^{2a}$ est alcoyle, alcényle ou alcynyle, la réaction d'un composé de formule (III) :

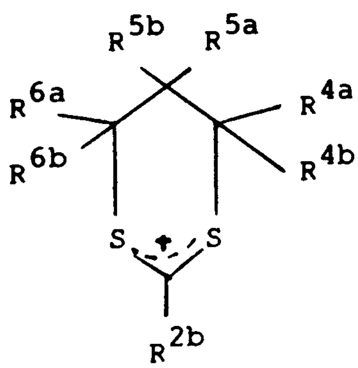

$ClO_4^-$

(III)

avec un alcool $R^{2a}OH$ ou réactif de Grignard $R^{2a}MgBr$, où $R^{2b}$, $R^{4a}$, $R^{4b}$, $R^{5a}$, $R^{5b}$, $R^{6a}$ et $R^{6b}$ sont définis dans l'une quelconque des revendications 1 à 12, ou

(iv) pour la préparation de composés où $R^{2a}$ est cyano, la réaction du composé correspondant où $R^{2a}$ est méthylthio avec un composé $(Alc)_3SiCN$, où Alc est un radical $C_{1-4}$-alcoyle, ou

(v) pour la préparation de composés où $R^{2a}$ est alcoylthio, la réaction du composé correspondant où $R^{2a}$ est hydrogène avec un disulfure de dialcoyle en présence d'un alcoyllithium, ou

(vi) pour la préparation de composés où $R^{2b}$ est pyridiyle, la réaction du composé correspondant où $R^{2b}$ est hydrogène avec la pyridine et après l'un quelconque des stades (i) à (vi), facultativement l'oxydation de l'un des atomes de soufre du cycle ou des deux, ou

(vii) la conversion d'un composé de formule (I) en un autre composé de formule (I).

**Revendications pour les Etats contractants suivants : ES, GR**

**1.** Procédé de préparation d'un composé de formule (I) :

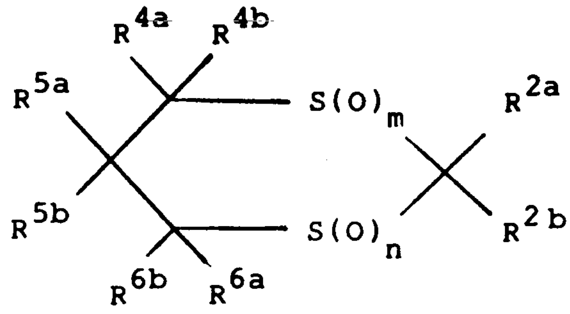

(I)

qui contient entre 9 et 30 atomes de carbone,
et où

m et n sont choisis indépendamment parmi 0, 1 et 2;

$R^{2a}$ est hydrogène, cyano, méthoxy, éthoxy, un radical $S(O)_qR^7$, où q est 0, 1 ou 2 et $R^7$ est méthyle ou éthyle, ou $R^{2a}$ est un radical $C{\equiv}CR^8$, où $R^8$ est hydrogène, $C_{1-4}$-alcoyle ou un radical silyle contenant trois radicaux $C_{1-4}$-alcoyle ou deux radicaux $C_{1-4}$-alcoyle et un radical phényle, ou $R^{2a}$ est vinyle, halo, $C_{1-3}$-alcoyle facultativement substitué par halo, méthoxy, éthoxy ou cyano, ou $R^{2a}$ est un radical $COR^9$, où $R^9$ est hydrogène, $C_{1-4}$-alcoxy, $C_{1-4}$-alcoyle ou un radical $NR^{10}R^{11}$, où $R^{10}$ ou $R^{11}$ sont choisis indépendamment parmi hydrogène, méthyle et éthyle;

$R^{2b}$, qui contient entre 2 et 18 atomes de carbone, est un cycle de 5 ou 6 chaînons contenant un atome d'oxygène, de soufre et/ou d'azote ou un radical $R^{12}$, où $R^{12}$ est un radical $C_{1-16}$-hydrocarbyle, chacun facultativement substitué par un radical azido, cyano ou nitro ou deux radicaux hydroxyle, ou par 1 à 5 atomes halo qui sont identiques ou différents, ou par 1 à 4 radicaux $R^{13}$ qui sont identiques ou différents et contiennent chacun 1 à 4 hétéroatomes, qui sont identiques ou différents et sont choisis parmi oxygène, soufre, azote et silicium, 1 à 16 atomes de carbone et facultativement 1 à 9 atomes fluoro ou chloro, ou dans le cas du cycle de 5 ou 6 chaînons facultativement substitué par un radical $R^{12}$;

$R^{4a}$ et $R^{4b}$, $R^{6a}$ et $R^{6b}$ sont choisis indépendamment parmi hydrogène, $C_{1-3}$-alcoyle, $C_{2-3}$-alcényle ou alcynyle, chacun facultativement substitué par halo, cyano ou $C_{1-4}$-alcoxy; cyano, halo ou un radical $COR^{9'}$, où $R^{9'}$ est hydrogène, $C_{1-4}$-alcoxy, $C_{1-4}$-alcoyle ou un radical $NR^{10'}R^{11'}$, où $R^{10'}$ et $R^{11'}$ sont choisis indépendamment parmi hydrogène, méthyle et éthyle;

$R^{5a}$ est un radical hydrocarbyle non aromatique comptant jusqu'à 7 atomes de carbone ou phényle, chacun facultativement substitué par cyano, halo, $C_{1-4}$-alcoyle, $C_{1-4}$-haloalcoyle, $C_{3-4}$-cycloalcoyle, $C_{1-4}$-alcoxy ou un radical $S(O)_{q'}R^{7'}$, où q' est 0 1 ou 2 et $R^{7'}$ est méthyle ou éthyle et $R^{5b}$ est hydrogène ou $C_{1-4}$-alcoyle facultativement substitué par alcoxy; avec la restriction que lorsque $R^{2a}$ est hydrogène, méthyle ou éthyle; $R^{2b}$ est acétylène ou contient entre 3 et 18 atomes de carbone et est un radical $R^{14}$, où $R^{14}$ est un radical $C_{1-13}$-hydrocarbyle non aromatique facultativement substitué par un radical $C_{1-4}$-carbalcoxy ou cyano et/ou par 1 ou 2 radicaux hydroxyle et/ou par 1 à 5 atomes halo qui sont identiques ou différents et/ou par 1 à 3 radicaux $R^{15}$ qui sont identiques ou différents et contiennent chacun 1 à 4 hétéroatomes, qui sont identiques ou différents et sont choisis parmi oxygène, soufre, azote et silicium, 1 à 10 atomes de carbone et facultativement 1 à 6 atomes fluoro ou chloro, ou $R^{2b}$ est un cycle aromatique à six chaînons substitué par cyano et/ou par 1 à 3 radicaux $R^{15}$ et/ou par un radical $-C{\equiv}CH$, $-C{\equiv}C-R^{14}$ ou $-C{\equiv}C$-halo et/ou par 1 à 5 atomes halo et/ou par 1 à 3 radicaux $C_{1-4}$-haloalcoyle où $R^{14}$ et $R^{15}$ sont tels que définis ci-dessus; $R^{4a}$ et $R^{6a}$ sont identiques ou différents et sont choisis parmi hydrogène, méthyle, trifluorométhyle et cyano; $R^{4b}$ et $R^{6b}$ sont hydrogène et $R^{5b}$ est hydrogène ou méthyle, alors $R^{5a}$ n'est pas butyle tertiaire,

lequel procédé comprend :

(i) la réaction d'un composé de formule (II) :

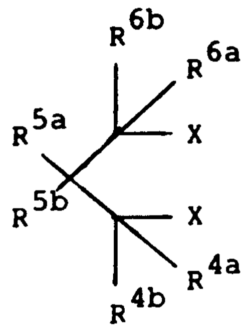

(II)

où X est SH, avec un aldéhyde ou cétone convenable de formule $R^{2a}(R^{2b})C=0$ ou un dérivé réactif de celui-ci, ou

(ii) lorsque $R^{2a}$ est hydrogène, la réaction d'un complexe dithiaborinane-sulfure de diméthyle d'un composé de formule (II) avec un acide carboxylique de formule $R^{2b}CO_2H$,

(iii) pour la préparation de composés où $R^{2a}$ est alcoyle, alcényle ou alcynyle, la réaction d'un composé de formule (III) :

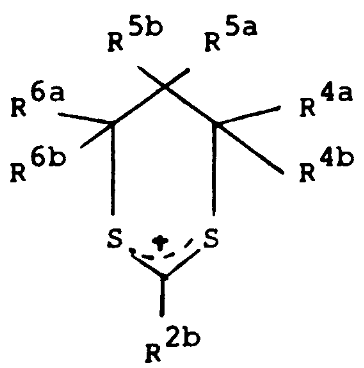

$ClO_4^-$

(III)

avec un alcool $R^{2a}OH$ ou réactif de Grignard $R^{2a}MgBr$, où $R^{2b}$, $R^{4a}$, $R^{4b}$, $R^{5a}$, $R^{5b}$, $R^{6a}$ et $R^{6b}$ sont définis ci-dessus, ou

(iv) pour la préparation de composés où $R^{2a}$ est cyano, la réaction du composé correspondant où $R^{2a}$ est méthylthio avec un composé $(Alc)_3SiCN$, où Alc est un radical $C_{1-4}$-alcoyle, ou

(v) pour la préparation de composés où $R^{2a}$ est alcoylthio, la réaction du composé correspondant où $R^{2a}$ est hydrogène avec un disulfure de dialcoyle en présence d'un alcoyllithium, ou

(vi) pour la préparation de composés où $R^{2b}$ est pyridiyle, la réaction du composé correspondant où $R^{2b}$ est hydrogène avec la pyridine et après l'un quelconque des stades (i) à (vi), facultativement l'oxydation de l'un des atomes de soufre du cycle ou des deux, ou

(vii) la conversion d'un composé de formule (I) en un autre composé de formule (I).

**2.** Procédé suivant la revendication 1 de préparation d'un composé de formule (I), où $R^{2b}$ est un radical phényle substitué aux positions 3, 4 et 5 par un à trois substituants choisis chacun parmi halo, $C_{1-4}$-haloalcoyle, cyano, azido, un radical $(C\equiv C)_pR^{16}$ ou un radical $(S)O_wR^{17}$, où p est 1 ou 2, w est 0, 1 ou 2, $R^{16}$ est hydrogène, brome, chlore ou iode et $R^{17}$ est trifluorométhyle, méthyle ou éthyle et le radical phényle est, en outre, facultativement substitué aux positions 2 et/ou 6 par fluoro ou chloro.

**3.** Procédé suivant la revendication 1 ou 2 de préparation d'un composé de formule (I), où $R^{2b}$ est phényle substitué aux positions 3, 4 et 5 par 1 à 3 substituants choisis chacun parmi halo, cyano, $C_{1-4}$-haloalcoyle ou un radical $C\equiv C-R^{21}$, où $R^{21}$ est hydrogène, méthyle ou éthyle, chacun facultativement substitué par hydroxyle, méthoxy, éthoxy, acétoxy; ou $R^{21}$ est $C_{1-4}$-carbalcoxy ou un radical silyle substitué par trois radicaux $C_{1-4}$-alcoyle et le radical phényle est, en outre, facultativement substitué aux positions 2 et/ou 6 par fluoro ou chloro.

**4.** Procédé suivant la revendication 1 ou 2 de préparation d'un composé de formule (I), où $R^{2b}$ est un radical -A(C≡C)Z, où A est une chaîne méthylène ou polyméthylène contenant facultativement une double liaison et/ou un atome d'oxygène ou de soufre et Z est hydrogène, $C_{1-5}$-alcoyle, $C_{1-3}$-alcoxyméthyle ou un radical $SiR^{18}R^{19}R^{20}$, où $R^{18}$ à $R^{20}$ sont identiques ou différents et sont chacun un radical $C_{1-4}$-aliphatique, ou $R^{18}$ et $R^{19}$ sont des radicaux $C_{1-4}$-aliphatiques et $R^{20}$ est un radical phényle.

**5.** Procédé suivant la revendication 1 ou 2 de préparation d'un composé de formule (I), où $R^{2b}$ est un radical $-BZ^1$, où B est un radical $-CH_2O-$ ou $-CH_2S(O)w-$, où w est 0, 1 ou 2, ou un radical $C_{2-6}$-aliphatique facultativement substitué par 1 à 3 atomes halo et $Z^1$ est silyle substitué par 1 à 3 radicaux $C_{1-4}$-alcoyle ou un radical $-C-(R^{22})(R^{23})R^{24}$, où $R^{22}$, $R^{23}$ et $R^{24}$ sont identiques ou différents et sont choisis chacun indépendamment parmi halo, $C_{1-4}$-alcoyle facultativement substitué par halo, $C_{1-4}$-alcoxy ou un radical $S(O)_qR^{25}$, où q est 0, 1 ou 2, $R^{25}$ est $C_{1-4}$-alcoyle, ou $R^{22}$, $R^{23}$ et $R^{24}$ sont choisis parmi $C_{1-4}$-alcoxy ou un radical $S(O)_wR^{26}$, où w est 0, 1 ou 2 et $R^{26}$ est $C_{1-4}$-alcoyle facultativement substitué par fluoro, ou $R^{22}$ et $R^{23}$ sont unis pour former un cycle $C_{3-6}$-cycloalcoyle, ou l'un d'entre $R^{22}$, $R^{23}$ et $R^{24}$ peut être hydrogène.

**6.** Procédé suivant la revendication 1 de préparation d'un composé de formule (I), où $R^{2b}$ est un radical :

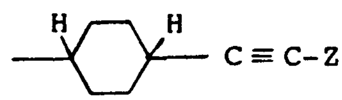

où Z est tel que défini dans la revendication 4.

**7.** Procédé suivant la revendication 1 de préparation d'un composé de formule (I), où $R^{2a}$ esthydrogène, méthyle ou éthyle.

**8.** Procédé suivant la revendication 1 de préparation d'un composé de formule (I), où $R^{2b}$ est phényle, phényle substitué ou un hétérocycle comptant 4 à 6 atomes de carbone sauf que (i) lorsque $R^{5a}$ est alcoyle en chaîne droite ou tolyle et $R^{2a}$, $R^{4a}$, $R^{4b}$, $R^{6a}$, $R^{6b}$ et $R^{5b}$ sont tous hydrogène, $R^{2b}$ n'est pas para-cyanophényle; (ii) lorsque $R^{5a}$ et $R^{5b}$ sont tous deux éthyle et $R^{4a}$, $R^{4b}$, $R^{6a}$, $R^{6b}$ et $R^{2a}$ sont tous hydrogène, $R^{2b}$ n'est pas para-tolyle; (iii) lorsque $R^{5a}$ est n-pentyle et $R^{2a}$, $R^{4a}$, $R^{4b}$, $R^{6a}$, $R^{6b}$ et $R^{5b}$ sont tous hydrogène, $R^{2b}$ n'est pas para-bromophényle; (iv) lorsque $R^{5b}$ et $R^{5a}$ sont tous deux méthyle et $R^{4a}$, $R^{4b}$, $R^{6b}$ et $R^{2a}$ sont tous hydrogène, $R^{2b}$ n'est pas phényle non substitué et (v) lorsque $R^{2b}$ est n-propyle, $R^{2a}$ est méthyle et $R^{4a}$, $R^{4b}$, $R^{6a}$, $R^{6b}$ et $R^{5b}$ sont tous hydrogène, $R^{5a}$ n'est pas éthyle ou i-propyle.

**9.** Procédé suivant la revendication 1 de préparation d'un composé de formule (I), où $R^{4a}$, $R^{4b}$, $R^{6a}$ et $R^{6b}$ sont hydrogène.

**10.** Procédé suivant la revendication 1 de préparation d'un composé de formule (Ia) :

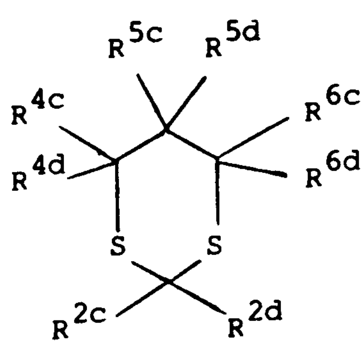

(Ia)

où

$R^{2c}$ est choisi parmi hydrogène, cyano, méthoxy, un radical $S(O)_qR^7$, où q est 0, 1 ou 2 et $R^7$ est méthyle ou éthyle, ou $R^{2c}$ est vinyle, halo, $C_{1-3}$-alcoyle facultativement substitué par halo, méthoxy, éthoxy ou cyano;

$R^{2d}$ est un radical $(C\equiv C)_rY(C\equiv C)_tZ^2$, où r est 0 ou 1 et t est 1 ou 2 et la somme de r et t n'est pas supérieure à 2, Y est une liaison simple, un radical

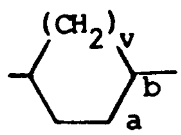

où v est 1, 2 ou 3 et le fragment $(C\equiv C)_tZ^2$ est uni à la position a ou b du cycle, ou Y est une chaîne méthylène ou polyméthylène contenant entre 1 et 8 atomes de carbone dans laquelle 1 ou 2 hétéroatomes et/ou doubles ou triples liaisons peuvent être intercalés, la chaîne étant facultativement substituée par 1 à 4 substituants qui peuvent être identiques ou différents et sont chacun choisis indépendamment parmi hydroxyle, oxo, halo, $C_{1-4}$-alcoyle, $C_{1-4}$-alcoxy, $C_{1-4}$-alcyloxy, époxy, un radical $C_{1-4}$-alcoylidène, un radical $C_{1-6}$-carbalcoxy, $C_{1-4}$-haloalcoyle ou cyano, $Z^2$ est choisi parmi hydrogène, $C_{1-10}$-hydrocarbyle facultativement substitué par halo, $C_{1-4}$-alcoxy, hydroxyle, oxo, un radical $S(O)_qR^7$ où q est 0, 1 ou 2 et $R^7$ est méthyle ou éthyle; ou $Z^2$ est un radical cyano, $C_{1-4}$-acyloxy ou carbalcoxy, halo ou un radical $SiR^{27}R^{28}R^{29}$, où $R^{27}$, $R^{28}$ et $R^{29}$ sont identiques ou différents et sont chacun un radical hydrocarbyle contenant jusqu'à 4 atomes de carbone, ou $Z^2$ est un radical $R^{30}OCO$, où $R^{30}$ est $C_{1-4}$-alcoyle;

$R^{4c}$, $R^{4d}$, $R^{6c}$ et $R^{6d}$ sont chacun choisis parmi hydrogène, méthyle, cyano et trifluorométhyle;

$R^{5c}$ est alcoyle, cycloalcoyle, alcényle, cycloalcényle ou alcynyle comptant chacun jusqu'à 7 atomes de carbone ou phényle, chacun facultativement substitué par halo, cyano, $C_{1-4}$-alcoyle, $C_{1-4}$-haloalcoyle, $C_{3-4}$-cycloalcoyle, $C_{1-4}$-alcoxy ou un radical $S(O)_qR^7$ tel que défini ci-dessus, et

$R^{5d}$ est hydrogène ou $C_{1-4}$-alcoyle, avec la restriction que $R^{5c}$ n'est pas butyle tertiaire.

**11.** Procédé suivant la revendication 1 de préparation d'un composé de formule (Ia) suivant la revendication 10, où $R^{2d}$ est un radical $-(C\equiv C)-Bu^t$.

**12.** Procédé suivant la revendication 1 de préparation :
du cis-2(a)-(4-bromophényl)-5(e)-butyl-1,3-dithiane,
du (e)-(t-butyl-2(e)-(4-méthylphényl)-1,3-dithiane,
du (e)-(t-butyl-2(e)-(4-nitrophényl)-1,3-dithiane,
du 2(e)-(4-bromophényl)-5(e)-néopentyl-1,3-dithiane,
du cis-2(e)-(4-bromophényl)-5(e)-t-butyl-2(a)-méthylthio-1,3-dithiane,
du 2(e)-(4-bromophényl)-5(e)-t-butyl-2(a)-méthoxy-1,3-dithiane,
du trans-2(e)-(4-bromophényl)-5(e)-éthyl-1,3-dithiane,
du 2(e)-(4-bromophényl)-2(a)-méthyl-5(e)-(1,1-diméthylpropyl)-1,3-dithiane,
du 2(e)-(4-bromophényl)-5(e)-t-butyl-1,3-dithiane-2(a)-carbonitrile,
du 2(a)-(4-bromophényl)-5(e)-éthyl-2(e)-méthyl-1,3-dithiane,
du trans-5(e)-t-butyl-2(e)-(4-t-butylphényl)-1,3-dithiane,
de la 2-(5(e)-t-butyl-1,3-dithian-2(e)-yl)propan-2-one,
du trans-5(e)-t-butyl-(2e)-(2,2-dibromoéthényl)-1,3-dithiane,
du trans-2(e)-bromoéthynyl-5(e)-t-butyl-1,3-dithiane,
de la 4-trans-5(e)-t-butyl-1,3-dithiane-2(e)-yl)-2,2-diméthylbutan-3-one,
du 2(e)-(3,3-diméthylbutyl)-5(e)-éthyl-1,3-dithiane,
du 2(a)-(4-bromophényl)-2(e)-méthyl-5(e)-(1,1-diméthylpropyl)-1,3-dithiane.

**13.** Composition pesticide comprenant un composé de formule (I) tel que défini dans l'une quelconque des revendications précédentes, en mélange avec un ou plusieurs excipients ou diluants.

**14.** Composition pesticide suivant la revendication 13, qui contient, en outre, un agent de synergie ou de potentialisation.

**15.** Composition pesticide suivant la revendication 13 ou 14, qui contient, en outre, un ou plusieurs constituants actifs pesticides, attractifs, répulsifs, bactéricides, fongicides et/ou anthelminthiques.

**16.** Composé de formule (I) à utiliser en médecine humaine ou vétérinaire.

**17.** Procédé de lutte contre les arthropodes ou helminthes nuisibles, qui comprend l'administration à l'arthropode ou à l'helminthe ou à leur environnement d'une quantité efficace d'un composé de formule (I) tel que préparé suivant l'une quelconque des revendications 1 à 12.

**18.** Procédé de lutte contre les infestations par des organismes nuisibles sur les animaux et/ou produits stockés et/ou sur un environnement, qui comprend l'administration d'une quantité efficace d'un composé de formule (I) tel que défini dans l'une quelconque des revendications précédentes à l'animal et/ou produit stocké et/ou à l'environnement susceptible de l'infestation par l'organisme nuisible.

**19.** Procédé de lutte contre les infestations par des organismes nuisibles sur les plantes et/ou produits stockés et/ou sur un environnement, qui comprend l'administration d'une quantité efficace d'un composé de formule (I) tel que défini dans l'une quelconque des revendications précédentes à la plante et/ou produit stocké et/ou à l'environnement susceptible de l'infestation par l'organisme nuisible.